# EUROPÄISCHE PATENTSCHRIFT

(11) **EP 1 569 936 B1**
(45) Veröffentlichungstag und Bekanntmachung des Hinweises auf die Patenterteilung: **18.03.2009**
(21) Anmeldenummer: 03789123.1
(22) Anmeldetag: 03.12.2003
(51) Int. Cl.: C07D 487/04, A61P 5/48

(54) **NEUE SUBSTITUIERTE IMIDAZO-PYRIDINONE UND IMIDAZO-PYRIDAZINONE, IHRE HERSTELLUNG UND IHRE VERWENDUNG ALS ARZNEIMITTEL**
NOVEL SUBSTITUTED IMIDAZO-PYRIDINONES AND IMIDAZO-PYRIDAZEIONES, THE PRODUCTION AND USE THEREOF AS MEDICAMENTS
NOUVELLES IMIDAZO-PYRIDINONES ET IMIDAZO-PYRIDAZINONES SUBSTITUEES, LEUR PRODUCTION ET LEUR UTILISATION EN TANT QUE MEDICAMENTS

(30) Priorität: 03.12.2002 DE 10256264; 07.03.2003 DE 10309927
(43) Veröffentlichungstag der Anmeldung: 07.09.2005
(73) Patentinhaber: Boehringer Ingelheim Pharma GmbH & Co. KG, 55216 Ingelheim am Rhein (DE)
(72) Erfinder: HAUEL, Norbert, 88433 Schemmerhofen (DE); HIMMELSBACH, Frank, 88441 Mittelbiberach (DE); LANGKOPF, Elke, 88447 Warthausen (DE); ECKHARDT, Matthias, 88400 Biberach (DE); MAIER, Roland, 88400 Biberach (DE); MARK, Michael, 88400 Biberach (DE); TADAYYON, Mohammad, Hertford SG14 1HQ (GB); KAUFFMANN-HEFNER, Iris, 88448 Attenweiler (DE)
(86) Internationale Anmeldenummer: PCT/EP2003/013648
(87) Internationale Veröffentlichungsnummer: WO 2004/050658

(56) Entgegenhaltungen:
- WO-A-02/14271
- WO-A-03/104229

## Beschreibung

Gegenstand der vorliegenden Erfindung sind neue substituierte Imidazopyridinone und Imidazo-pyridazinone der allgemeinen Formel 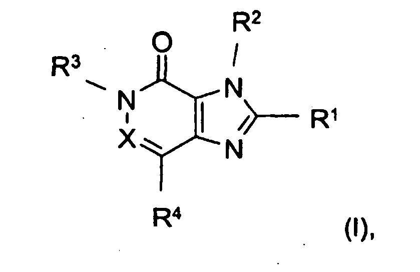 deren Tautomere, deren Enantiomere, deren Diastereomere, deren Gemische und deren Salze, insbesonders deren physiologisch verträgliche Salze mit anorganischen oder organischen Säuren, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

Gegenstand der vorliegenden Erfindung sind somit die obigen Verbindungen der allgemeinen Formel I, welche wertvolle pharmakologische Eigenschaften aufweisen, deren Tautomere, deren Enantiomere, deren Diastereomere, deren Gemische und deren Salze, die die pharmakologisch wirksamen Verbindungen enthaltenden Arzneimittel, deren Verwendung und Verfahren zu ihrer Herstellung.

Das Dokument WO 02/14271 beschreibt Prolin-Derivate als DPP-IV Hemmer. WO 03/104 229 beschriebt kondensierte hetero bizyklische Imidazolderivate als DPP-IV Hemmer.

In der obigen allgemeinen Formel I bedeuten
X ein Stickstoffatom oder eine Gruppe der Formel C-R⁵,
wobei R⁵ ein Wasserstoffatom oder eine Methylgruppe bedeutet,
R¹ eine 5-bis 7-gliedrige Cycloalkyleniminogruppe, die im Kohlenstoffgerüst durch eine Aminogruppe substituiert ist und durch eine C₁₋₃-Alkylgruppe substituiert sein kann,
oder eine durch eine C₅₋₇-Cycloalkylgruppe substituierte Aminogruppe,
wobei die C₅₋₇-Cycloalkylgruppe durch eine Aminogruppe substituiert ist oder ein Kohlenstoffatom in 3-Position der C₅₋₇-Cycloalkylgruppe durch eine -NH- Gruppe ersetzt ist,
R² eine Benzylgruppe, in der der Phenylrest durch ein oder zwei Fluor-, Chlor-oder Bromatome oder durch eine Cyanogruppe substituiert sein kann,
eine lineare oder verzweigte C₃₋₆-Alkenylgruppe,
eine C₃₋₅-Alkinylgruppe,
eine C₅₋₇-Cycloalkylmethylgruppe,
eine C₅₋₇-Cycloalkenylmethylgruppe,
oder eine Furylmethyl-, Thienylmethyl-, Pyrrolylmethyl-, Thiazolylmethyl-, imidazolylmethyl-, Pyridinylmethyl-, Pyrimidinylmethyl-, Pyridazinylmethyl- oder Pyrazinylmethylgruppe,
R³ eine lineare oder verzweigte C₁₋₆-Alkylgruppe,
eine gegebenenfalls im Arylteil durch ein Halogenatom, eine Cyano-, eine C₁₋₃-Alkyl- oder eine Methoxygruppe substituierte Phenyl-C₁₋₃-alkyl- oder Naphthyl-C₁₋₃-alkylgruppe,
eine 2-Phenyl-2-hydroxy-ethylgruppe,
eine Phenylcarbonylmethylgruppe,
in der die Phenylgruppe durch eine Hydroxy-, C₁₋₃-Alkyloxy-, Aminocarbonyl-C₁₋₃-alkoxy-, (C₁-₃-Alkylamino)-carbonyl-C₁₋₃-alkoxy-, [Di-(C₁₋₃-alkyl)-amino]-carbonyl-C₁₋₃-alkoxy-, Amino-, C₁₋₃-Alkyl-carbonylamino-, C₃₋₆-Cycloalkyl-carbonylamino-, C₁₋₃-Alkoxy-carbonylamino-, C₁₋₃-Alkylsulfonylamino-oder Aminocarbonylgruppe substituiert sein kann,
eine (3-Methyl-2-oxo-2,3-dihydro-benzoxazolyl)-carbonylmethylgruppe,
eine Thienylcarbonylmethylgruppe,
eine Heteroaryl-C₁₋₃-alkylgruppe,
wobei unter dem Ausdruck "Heteroarylgruppe" eine gegebenenfalls durch eie oder zwei C₁₋₃-Alkylgruppen oder durch eine Morpholin-4-yl-, Pyridyl-oder Phenylgruppe substituierte monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-oder Phenyl-C₁₋₃-alkylgruppe substituierte Iminogruppe, ein Sauerstoff- oder Schwefelatom oder
eine gegebenenfalls durch eine C₁₋₃-Alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe oder ein Sauerstoff- oder Schwefelatom und zusätzlich zwei oder drei Stickstoffatome enthält,
und wobei zusätzlich an die vorstehend erwähnten monocyclischen Heteroarylgruppen über zwei benachbarte Kohlenstoffatome ein Phenylring, der gegebenenfalls durch ein Halogenatom, durch eine oder zwei C₁₋₃-Alkylgruppen oder durch eine Trifluormethyl- oder Methoxygruppe substituiert sein kann, ankondensiert sein kann
und die Bindung über ein Atom des heterocyclischen Teils oder des ankondensierten Phenylrings erfolgen kann,
eine bicyclische Heteroarylmethylgruppe gemäß einer der Formeln 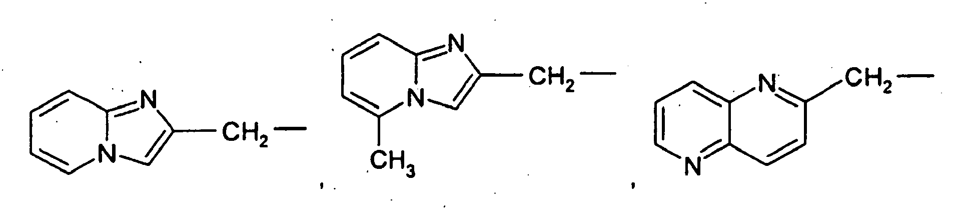 oder 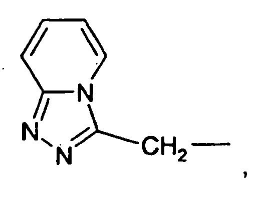 eine Gruppe der Formel 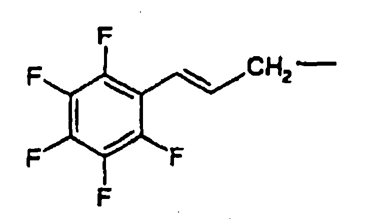 oder eine Gruppe der Formeln 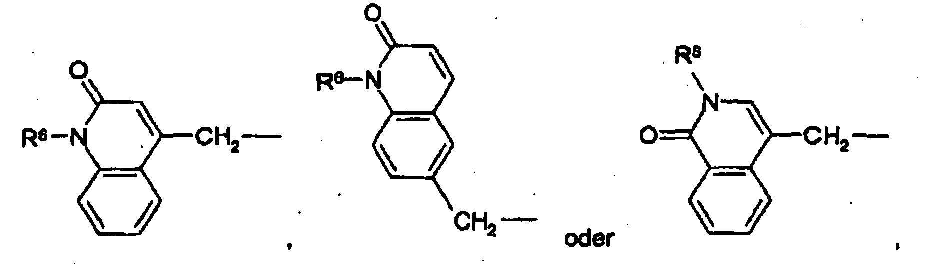 in der R⁶ jeweils ein Wasserstoffatom oder eine Methylgruppe bedeutet,
und R⁴ ein Wasserstoffatom oder eine C₁₋₃-Alkylgruppe,
wobei die in den Definitionen enthaltenen Alkyl- und Alkoxygruppen, die mehr als zwei Kohlenstoffatome aufweisen, soweit nichts anderes erwähnt wurde, geradkettig oder verzweigt sein können,
und wobei die Wasserstoffatome der in den Definitionen enthaltenen Methyl- oder Ethylgruppen ganz oder teilweise durch Fluoratome ersetzt sein können.
mit der Maßgabe, dass die folgenden beiden Verbindungen ausgeschlossen sind:
2-(2-Amino-cyclohexylamino)-3-(but-2-inyl)-5-methyl-3,5-dihydro-imidazo[4,5-d]pyridazin-4- und
2-(3-Amino-piperidin-1-yl)-3-(but-2-inyl)-5-methyl-3,5-dihydro-imidazo[4,5-d]pyridazin-4-,

Bevorzugt sind diejenigen Verbindungen der allgemeinen Formel I, in denen
X ein Stickstoffatom oder eine Methingruppe,
R¹ eine 3-Amino-piperidin-1-yl-, 3-Amino-3-methyl-piperidin-1-yl-, 3-Amino-pyrrolidin-1-yl-, 1,4-Diazepan-1-yl-, (2-Amino-cyclohexyl)-amino- oder Piperidin-3-yl-aminogruppe,
R² eine Benzylgruppe, in der der Phenylrest durch ein oder zwei Fluoratome, durch ein Chlor- oder Bromatom oder durch eine Cyanogruppe substituiert sein kann,
eine lineare oder verzweigte C₃₋₈-Alkenylgruppe,
eine Propin-3-yl- oder But-2-in-4-ylgruppe,
eine Cyclopropylmethylgruppe,
eine C₅₋₇-Cycloalkenylmethylgruppe,
oder eine Furylmethyl-oder Thienylmethylgruppe,
R³ eine lineare oder verzweigte C₁₋₆-Akylgruppe,
eine gegebenenfalls im Arylteil durch ein Fluor-, Chlor- oder Bromatom oder durch eine Cyano-. C₁₋₃-Alkyl- oder Methoxygruppe substituierte Phenyl-C₁₋₂-alkyl- oder Naphthyl-C₁₋₂-alkylgruppe,
eine 2-Phenyl-2-hydroxy-ethylgruppe,
eine Phenylcarbonylmethylgruppe,
in der die Phenylgruppe durch eine Hydroxy-, C₁₋₂-Alkyloxy-, Aminocarbonyl-C₁₋₃-alkoxy-, (C₁₋₃-Alkylamino)-carbonyl-C₁₋₃-alkoxy-, [Di-(C₁₋₃-alkyl)-amino]-carbonyl-C₁₋₃-alkoxy-, Amino-, C₁₋₃-Alkyl-carbonylamino-, C₃₋₈-Cycloalkyl-carbonylamino-, C₁₋₃-Alkoxy-carbonylamino-, C₁₋₃-Alkylsulfonylamino-oder Aminocarbonylgruppe substituiert sein kann,
eine (3-Methyl-2-oxo-2,3-dihydro-benzoxazolyl)-carbonylmethylgruppe,
eine Thienylcarbonylmethylgruppe,
eine Heteroaryl-methylgruppe,
wobei unter dem Ausdruck "Heteroarylgruppe" eine gegebenenfalls durch eine oder zwei Methylgruppen oder durch eine Pyridyl- oder Phenylgruppe substituierte Pyridinyl-, Pyrimidinyl-, Pyridazinyl-, Pyrazinyl-, Thiazolyl-, Oxazolyl-, Isothiazolyl-, Isoxazolyl-, Pyrazolyl-, Imidazolyl-, Oxadiazolyl-, Thiadiazolyl- oder Thienylgruppe zu verstehen ist,
und wobei zusätzlich an die vorstehend erwähnten monocyclischen Heteroarylgruppen über zwei benachbarte Kohlenstoffatome ein Phenylring, der gegebenenfalls durch ein Chloratom, durch eine oder zwei Methylgruppen oder durch eine Trifluormethyl- oder Methoxygruppe substituiert sein kann, ankondensiert sein kann
und die Bindung über ein Atom des heterocyclischen Teils oder des ankondensierten Phenylrings erfolgen kann,
eine Imidazo[1,2-a]pyridin-2-yl-methyl-gruppe der Formeln 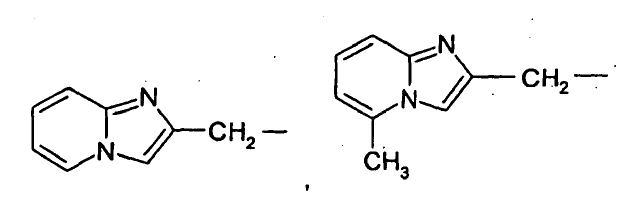 oder 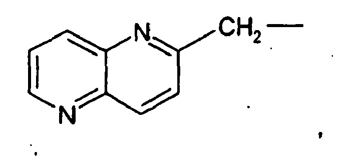 eine 1,2,4-Triazolo[4,3-a]pyridin-3-yl-gruppe der Formel 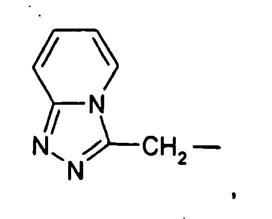 oder eine Gruppe der Formeln 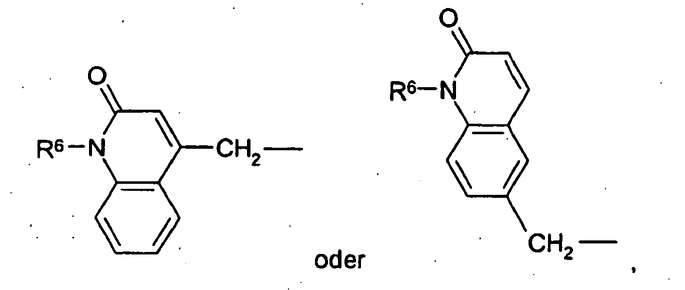 in der R⁶ jeweils ein Wasserstoffatom oder eine Methylgruppe bedeutet,
und R⁴ ein Wasserstoffatom oder eine Methylgruppe bedeuten,
wobei die in den Definitionen enthaltenen Alkyl- und Alkoxygruppen, die mehr als zwei Kohlenstoffatome aufweisen, soweit nichts anderes erwähnt wurde, geradkettig oder verzweigt sein können,
und wobei die Wasserstoffatome der in den Definitionen enthaltenen Methyl- oder Ethylgruppen ganz oder teilweise durch Fluoratome ersetzt sein können,
mit der Maßgabe, dass die folgenden beiden Verbindungen ausgeschlossen sind:
2-(2-Amino-cyclohexylamino)-3-(but-2-inyl)-5-methyl-3,5-dihydro-imidazo[4,5-d]pyridazin-4- und
2-(3-Amino-piperidin-1-yl)-3-(but-2-inyl)-5-methyl-3,5-dihydro-imidazo[4,5-d]pyridazin-4-,
deren Tautomere, deren Enantiomere, deren Diastereomere, deren Gemische und deren Salze,
insbesondere jedoch diejenigen Verbindungen der allgemeinen Formel I, in denen
X, R², R³ und R⁴ wie oben erwähnt definiert sind und
R¹ eine 3-Amino-piperidin-1-yl-gruppe bedeutet,
mit der Maßgabe, dass die folgende Verbindung ausgeschlossen ist:
2-(3-Amino-piperidin-1-yl)-3-(but-2-inyl)-5-methyl-3,5-dihydro-imidazo[4,5-d]pyridazin-4-,
deren Tautomere, deren Enantiomere, deren Diastereomere, deren Gemische und deren Salze.

Eine zweite Untergruppe der bevorzugten Verbindungen betrifft diejenigen Verbindungen der allgemeinen Formel I, in denen
X, R¹, R³ und R⁴ wie oben erwähnt definiert sind und
R² eine 3-Methylallyl-, eine 3,3-Dimethylallyl- oder eine But-2-in-4-ylgruppe bedeutet,
mit der Maßgabe, dass die folgenden beiden Verbindungen ausgeschlossen sind:
2-(2-Amino-cyclohexylamino)-3-(but-2-inyl)-5-methyl-3,5-dihydro-imidazo[4,5-d]pyridazin-4- und
2-(3-Amino-piperidin-1-yl)-3-(but-2-inyl)-5-methyl-3,5-dihydro-imidazo[4,5-d]pyridazin-4-,
deren Tautomere, deren Enantiomere, deren Diastereomere, deren Gemische und deren Salze.

Besonders bevorzugt sind folgende Verbindungen der allgemeinen Formel I:
(1) 2-(3-Amino-piperidin-1-yl)-3-(but-2-inyl)-5-(naphthalin-1-ylmethyl)-3,5-dihydro-imidazo[4,5-d]pyridazin-4-on 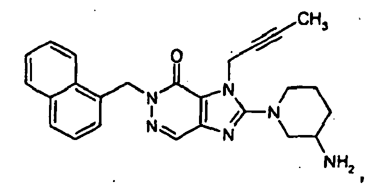
(2) 2-(3-Amino-piperidin-1-yl)-3-but2-inyl-5-(3-methyl-isochinolin-1-ylmethyl)-3,5-dihydro-imidazo[4,5-d]pyridazin-4-on, 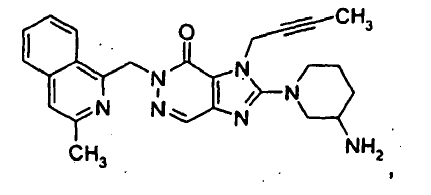
(3) 2-(3-Amino-piperidin-1-yl)-3-(but-2-inyl)-5-(chinazolin-2-ylmethyl)-3,5-dihydro-imidazo[4,5-d]pyridazin-4-on 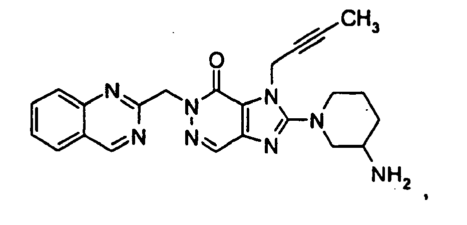
(4) 2-((*R*)-3-Amino-piperidin-1-yl)-3-(but-2-inyl)-5-(4-methyl-chinazolin-2-ylmethyl)-3,5-dihydro-imidazo[4,5-d]pyridazin-4-on 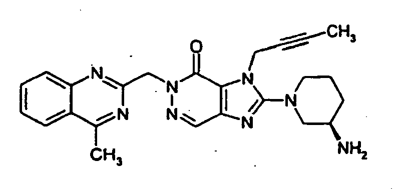
(5) 2-((*R*)-3-Amino-piperidin-1-yl)-3-(but-2-inyl)-5-(4-cyano-naphthalin-1-ylmethyl)-3,5-dihydro-imidazo[4,5-d]pyridazin-4-on 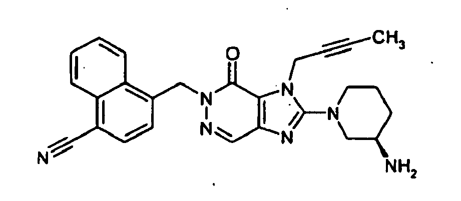
(6) 2-((*R*)-3-Amino-piperidin-1-yl)-3-(but-2-inyl)-5-(4-bromnaphth-1-ylmethyl)-3,5-dihydro-imidazo[4,5-d]pyridazin-4-on 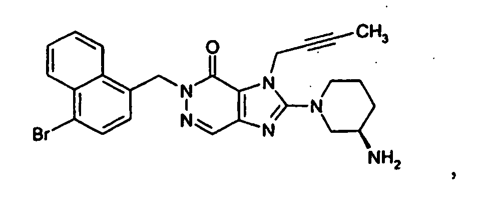
(7) 2-((*R*)-3-Amino-piperidin-1-yl)-3-(but-2-inyl)-5-(benzo[1,2,5]thiadiazol-5-ylmethyl)-3,5-dihydro-imidazo[4,5-d]pyridazin-4-on 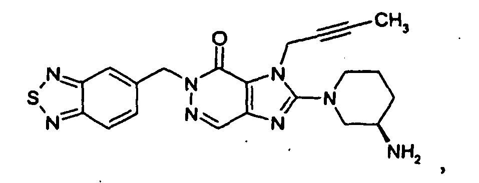
(8) 2-((*R*)3-Amino-piperidin-1-yl)-3-(2-chlorbenzyl)-5-(3-methyl-isochinolin-1-ylmethyl)-3,5-dihydro-imidazo[4,5-d]pyridazin-4-on 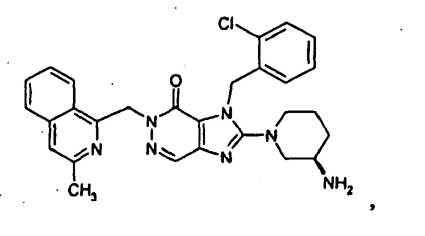
(9) 2-(3-Amino-piperidin-1-yl)-3-(but-2-inyl)-5-(chinoxalin-6-ylmethyl)-3,5-dihydro-imidazo[4,5-d]pyridazin-4-on 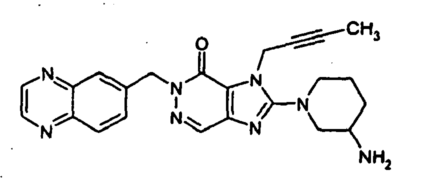
(10) 2-((*R*)-3-Amino-piperidin-1-yl)-3-(but-2-inyl)-5-(2,3-dimethyl-chinoxalin-6-ylmethyl)-3,5-dihydro-imidazo[4,5-d]pyridazin-4-on 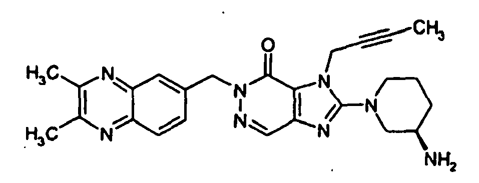
(11) 2-((*R*)-3-Amino-piperidin-1-yl)-3-(but-2-inyl)-5-(5-methyl-imidazo[1,2-a]pyridin-2-ylmethyl)-3,5-dihydro-imidazo[4,5-d]pyridazin-4-on 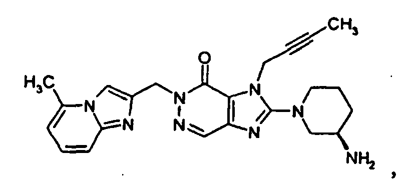
(12) 2-((*R*)-3-Amino-piperidin-1-yl)-3-(but-2-inyl)-5-(1-methyl-1*H*-chinolin-2-on-6-ylmethyl)-3,5-dihydro-imidazo[4,5-d]pyridazin-4-on 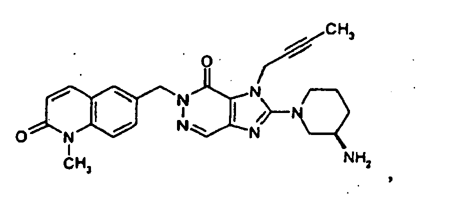
(13) 2-((*R*)-3-Amino-piperidin-1-yl)-3-(but-2-inyl)-5-(4-methyl-phthalazin-1-ylmethyl)-3,5-dihydro-imidazo[4,5-d]pyridazin-4-on 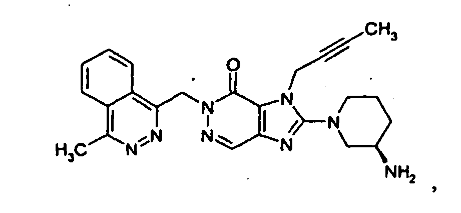
(14) 2-((*R*)-3-Amino-piperidin-1-yl)-3-(but-2-inyl)-5-([1,5]naphthyridin-2-ylmethyl)-3,5-dihydro-imidazo[4,5-d]pyridazin-4-on 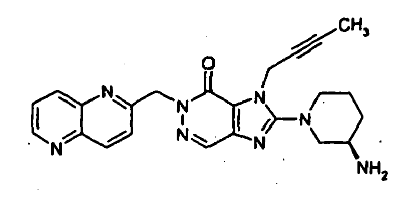 und
(15) 2-((*R*)-3-Amino-piperidin-1-yl)-3-(but-2-inyl)-5-(2,3,8-trimethyl-chinoxalin-6-ylmethyl)-3,5-dihydro-imidazo[4,5-d]pyridazin-4-on 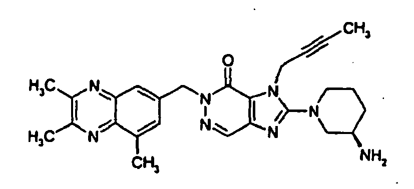
sowie deren Enantiomere und deren Salze.

Erfindungsgemäß erhält man die Verbindungen der allgemeinen Formel I nach an sich bekannten Verfahren, beispielsweise nach folgenden Verfahren:
a) Umsetzung einer Verbindung der allgemeinen Formel 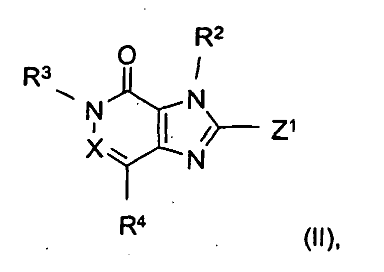 in der X, R², R³ und R⁴ wie eingangs erwähnt definiert sind und Z¹ eine nukleofuge Austrittsgruppe wie beispielsweise ein Chlor- oder Bromatom oder eine C₁₋₃-Alkyl-sulfanyl-, C₁₋₃-Alkylsulfinyl- oder C₁₋₃-Alkylsulfonylgruppe bedeutet,
   mit einem Amin der allgemeinen Formel

   H-R¹ (III),

   in der R¹ wie eingangs definiert ist.
   Die Umsetzung wird zweckmäßigerweise in einem Lösungsmittel wie Isopropanol, Butanol, Tetrahydrofuran, Dioxan, Dimethylformamid, Dimethylsulfoxid, Ethylenglycolmonomethylether, Ethylenglycoldiethylether oder Sulfolan gegebenenfalls in Gegenwart einer anorganischen oder tertiären organischen Base, z.B. Natriumcarbonat, Kaliumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß des Amins der allgemeinen Formel R^{4'}-H durchgeführt werden.
b) Entschützung einer Verbindung der allgemeinen Formel 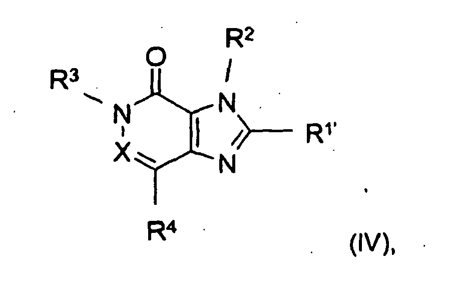 in der R², R³ und R⁴ wie eingangs erwähnt definiert sind und
   R^{1'} eine der eingangs für R¹ erwähnten Gruppen bedeutet, in der die Imino-, Amino- bzw. Alkylaminogruppe durch eine Schutzgruppe substituiert ist.

Die Freisetzung einer Aminogruppe aus einer geschützten Vorstufe ist eine Standardreaktion in der synthetischen organischen Chemie. Als Schutzgruppen kommen eine Vielzahl von Gruppen in Frage. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocienski, Protecting Groups, Georg Thieme Vertrag, 1994.

Als Beispiele für Schutzgruppen seien genannt:
die tert.-Butyloxycarbonylgruppe, die sich durch Behandeln mit einer Säure wie beispielsweise Trifluoressigsäure oder Salzsäure oder durch Behandlung mit Bromtrimethylsilan oder Iodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C abspalten lässt,
die 2.2.2-Trichlorethoxycarbonylgruppe, die sich abspalten lässt durch Behandeln mit Metallen wie beispielsweise Zink oder Cadmium in einem Lösungsmittel wie Essigsäure oder einem Gemisch aus Tetrahydrofuran und einer schwachen wässrigen Säure bei Temperaturen zwischen 0°C und der Siedetemperatur des verwendeten Lösungsmittels und
die Carbobenzyloxycarbonylgruppe, die sich beispielsweise abspalten lässt durch Hydrogenolyse in Gegenwart eines Edelmetallkatalysators wie beispielsweise Palladium-Kohle und einem Lösungsmittel wie beispielsweise Alkohole, Essigester, Dioxan, Tetrahydrofuran oder Gemische dieser Lösungsmittel bei Temperaturen zwischen 0°C und dem Siedepunkt des Lösungsmittels, durch Behandeln mit Bortribromid in Methylenchlorid bei Temperaturen zwischen -20°C und Raumtemperatur, oder durch Behandeln mit Aluminiumchlorid/Anisol bei Temperaturen zwischen 0°C und Raumtemperatur.

Gewünschtenfalls wird anschließend ein während den Umsetzungen zum Schutze von reaktiven Gruppen verwendeter Schutzrest abgespalten und/oder
eine so erhaltene Verbindung der allgemeinen Formel I in ihre Stereoisomere aufgetrennt und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure, überführt.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert-Butyl-, Trityl-, Benzyl- oder Tetrahydropyranylgruppe,
als Schutzreste für eine Carboxygruppe die Trimethylsilyl-, Methyl-, Ethyl-, tert-Butyl-, Benzyl- oder Tetrahydropyranylgruppe,
als Schutzreste für eine Phosphonogruppe eine Alkylgruppe wie die Methyl-, Ethyl-, Isopropyl- oder n-Butylgruppe, die Phenyl- oder Benzylgruppe und
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Spaltung nur eines Alkylrestes von einer O,O'-Dialkyl-phosphonogruppe erfolgt beispielsweise mit Natriumiodid in einem Lösungsmittel wie Aceton, Ethylmethylketon, Acetonitril oder Dimethylformamid bei Temperaturen zwischen 40 und 150°C, vorzugsweise jedoch bei Temperaturen zwischen 60 und 100°C.

Die Abspaltung beider Alkylreste von einer O,O'-Dialkyl-phosphonogruppe erfolgt beispielsweise mit Jodtrimethylsilan, Bromtrimethylsilan oder Chlortrimethylsilan/Natriumiodid in einem Lösungsmittel wie Methylchlorid, Chloroform oder Acetonitril bei Temperaturen zwischen 0°C und der Siedetemperatur des Reaktionsgemisches, vorzugsweise jedoch bei Temperaturen zwischen 20 und 60°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D-und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin,

Cyclohexylamin, Ethanolamin, Diethanolamin und Triethanolamin in Betracht.

Die als Ausgangsstoffe verwendeten Verbindungen der allgemeinen Formeln II bis IV sind entweder literaturbekannt oder man erhält diese nach an sich literaturbekannten Verfahren, beispielsweise durch die in Schema 1 bis 5 dargestellten Synthesewege. Bevorzugt ist die in Schema 4 wiedergegebene Synthese.

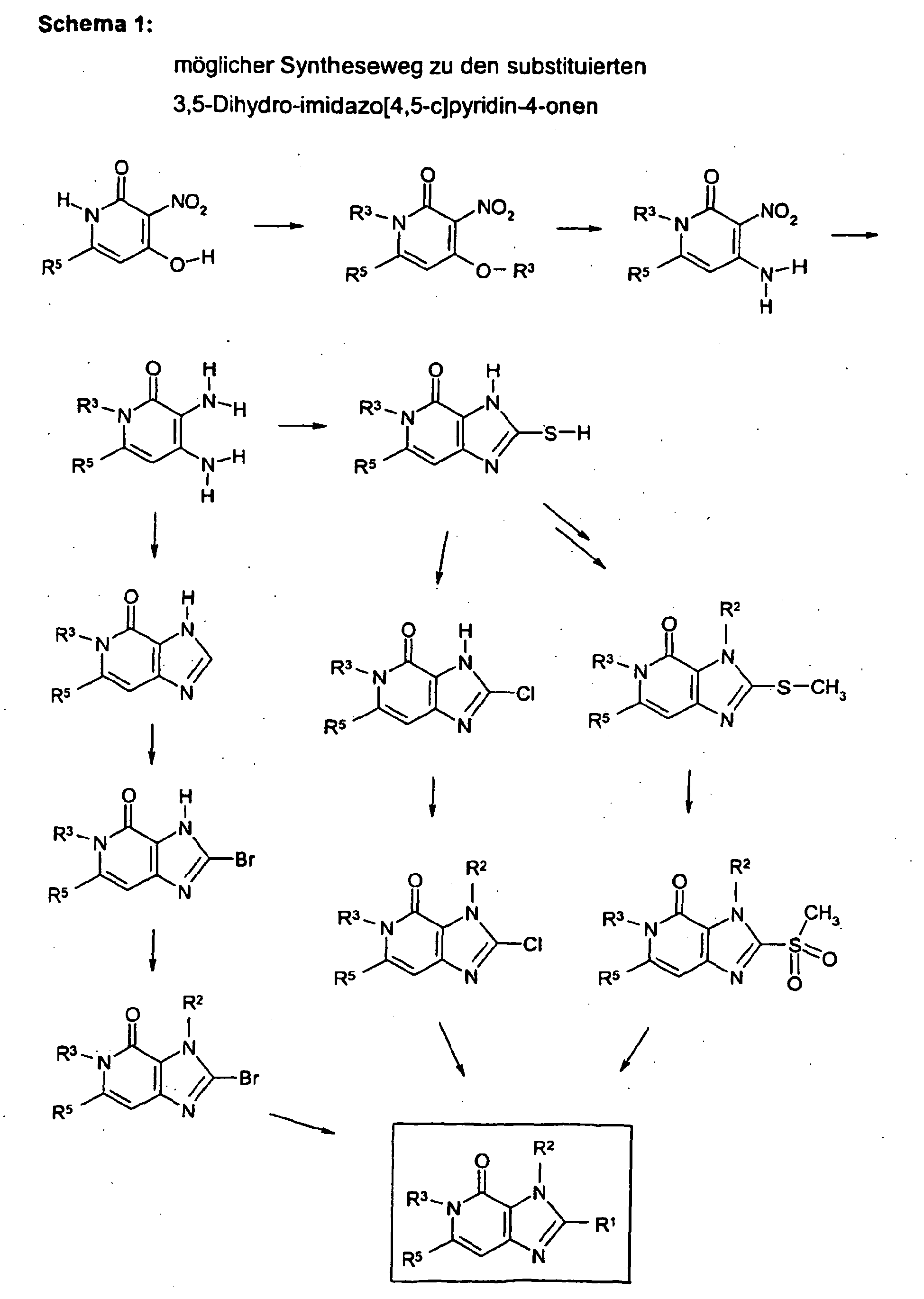

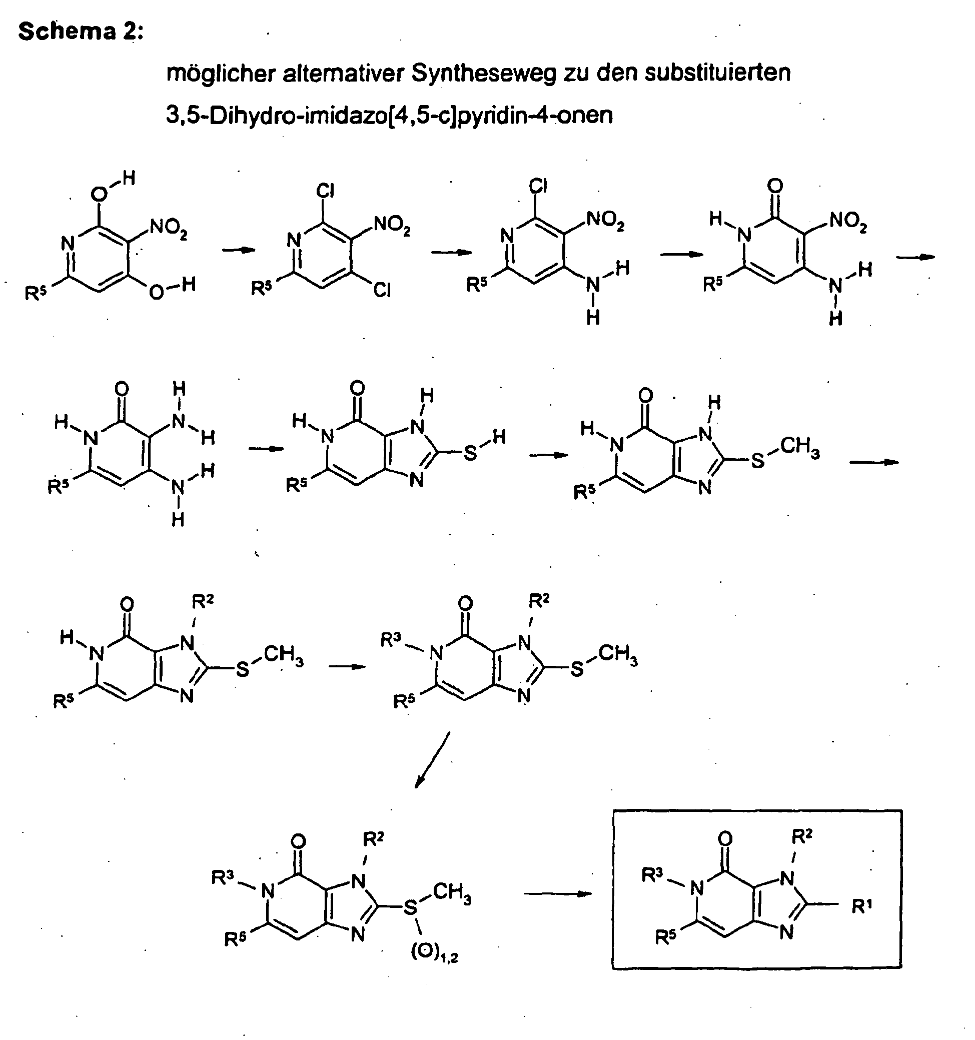

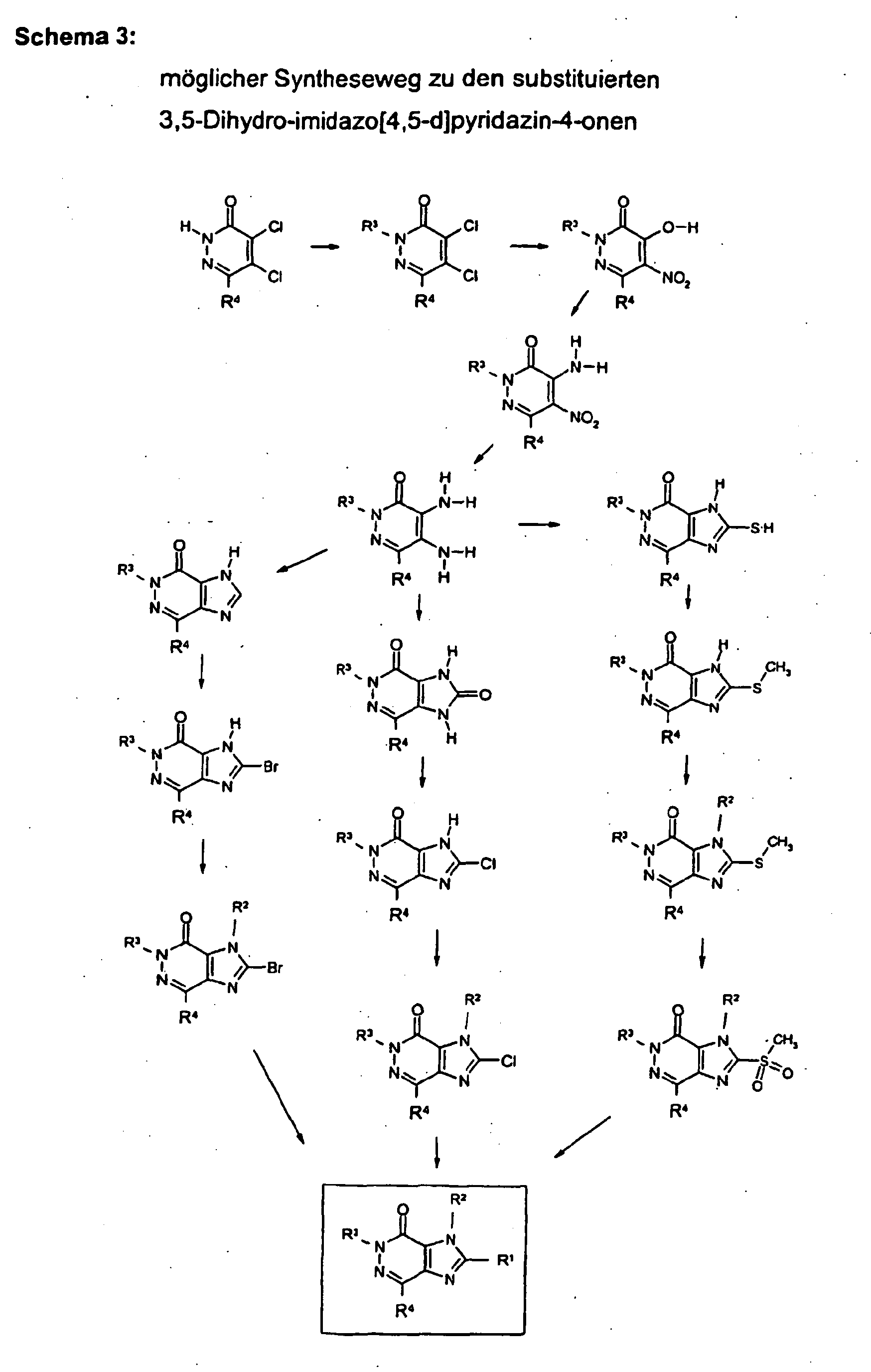

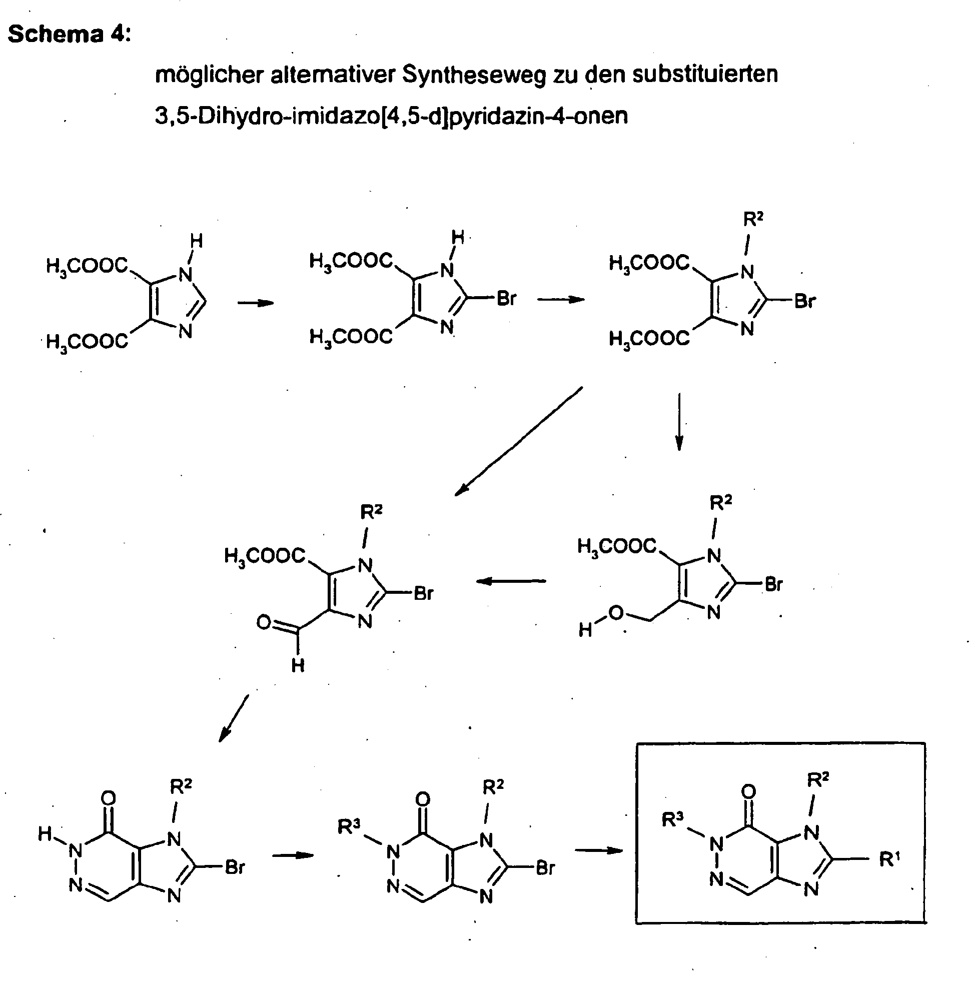

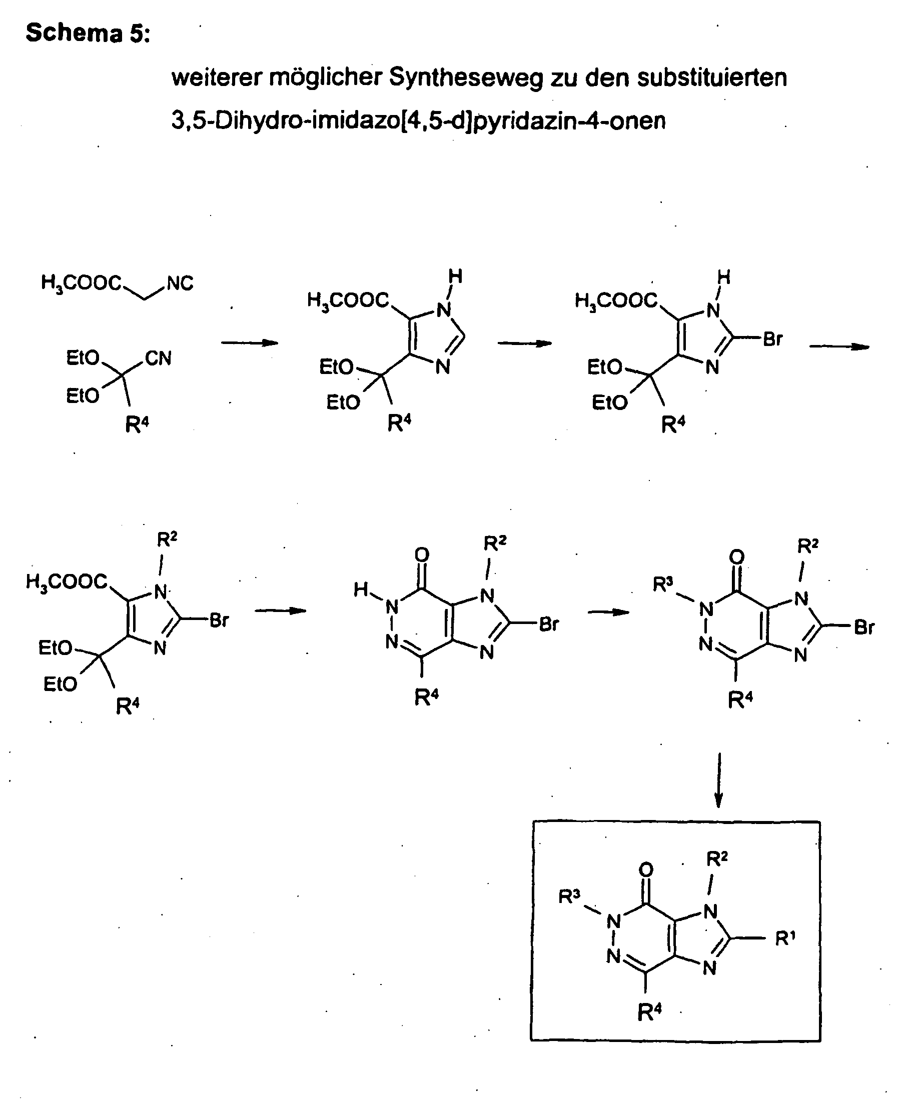

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10mM Tris HCl, 0.15 M NaCl, 0.04 t.i.u. Aprotinin, 0.5%

Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:

50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP-IV-Hemmung IC₅₀ [nM] |
|---|---|
| 2 | 13 |
| 7 | 5.4 |
| 182 | 1.2 |
| 191 | 14 |
| 195 | 17 |
| 202 | 9.8 |
| 205 | 5.4 |
| 217 | 10 |

Die erfindungsgemäß hergestellten Verbindungen sind gut verträglich, da beispielsweise nach oraler Gabe von 10 mg/kg der Verbindung des Beispiels 2 an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ I und Typ II, diabetische Komplikationen, metabolische Azidose oder Ketose, Insulinresistenz, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B.

GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Ebenso sind sie zur Prävention und Therapie von chronischen entzündlichen Darmerkrankungen geeignet. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Des weiteren sind die Substanzen geeignet, Mangelzustände von Wachstumshormon, die mit Minderwuchs einhergehen, zu beeinflussen.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570), alpha-Glucosidasehemmer (z.B. Acarbose, Voglibose), alpha2-Antagonisten, Insulin und Insulinanaloga, GLP-1 und GLP-1 Analoga (z.B. Exendin-4) oder Amylin. Daneben Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, Cholesterolresorptionsinhibitoren wie zum Beispiel Ezetimibe, gallensäurebindende Substanzen wie zum Beispiel Colestyramin, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin oder β3-Agonisten wie SB-418790 oder AD-9677. Daneben ist eine Kombination mit Medikamenten zur Beeinflussung des Bluthochdrucks wie z.B. All Antagonisten oder ACE Inhibitoren, Diuretika, β-Blocker und andere oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

Für die Synthese der Verbindungen, die eine chirale 3-Amino-piperidin-1-ylgruppe enthalten, wurde das entsprechende chirale Reagenz, nämlich (*R*)-3-(tert.-Butyloxycarbonylamino)-piperidin als Ausgangsmaterial verwendet. (*R*)-3-(tert.-Butyloxycarbonylamino)-piperidin wurde ausgehend von (*R*)-1-Benzyl-3-(tert.-butyloxycarbonylamino)-piperidin, welches analog dem literaturbekannten (*S*)-Enantiomer hergestellt wurde (s. Moon, Sung-Hwan; Lee, Sujin; Synth.Commun.; 28; 21; 1998; 3919-3926), wie folgt hergestellt:
Ca. 2 g (*R*)-1-Benzyl-3-(tert.-butyloxycarbonylamino)-piperidin in 20 ml Methanol werden 24 Stunden bei Raumtemperatur und einem Wasserstoffdruck von 3 bar in Gegenwart von 200 mg Palladium auf Aktivkohle (10% Pd) hydriert. Danach wird vom Katalysator abgesaugt und das Filtrat zur Trockene eingeengt. Schmelzpunkt: 119°C
Massenspektrum (ESI⁺): m/z = 201 [M+H]⁺

### Beispiel 1

### 2-(3-Amino-piperidin-1-yl)-3-benzyl-5-(naphthalin-1-ylmethyl)-3,5-dihydro-imidazo[4,5-d]pyridazin-4-on

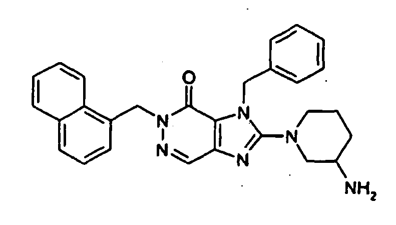

### 1 a) 4,5-Dichlor-2-naphthalin-1-ylmethyl-2H-pyridazin-3-on

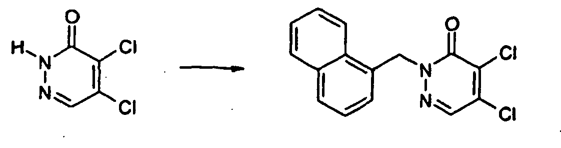

Zu einer Lösung von 10.0 g (60.61 mMol) 4,5-Dichlor-3-hydroxy-pyridazin in 50 ml Dimethylsulfoxid wurden 9.0 g (65 mMol) Kaliumcarbonat gegeben, dann 9.42 g (63 mMol) 1-(Chlormethyl)-naphthalin zugesetzt und 17 Stunden bei 50°C gerührt. Die dunkle Lösung wurde nach dem Abkühlen mit 300 ml dest. Wasser versetzt, dann 300 ml Dichlormethan eingerührt, über Celite abgesaugt, die wäßrige Phase abgetrennt und noch dreimal mit je 50 ml Dichlormethan extrahiert. Die vereinigten organischen Phasen wurden mit Wasser gewaschen, über Natriumsulfat getrocknet und eingeengt. Das so erhaltene Rohprodukt wurde in 250 ml Dichlormethan gelöst, die Lösung über Kieselgel filtriert und dann eingeengt. Der Rückstand wurde mit Petrolether verrieben, abgesaugt und getrocknet.
Ausbeute: 67.6% der Theorie.
C₁₅H₁₀Cl₂N₂O (305.17)
Rf-Wert: 0.71 (Kieselgel, Dichlormethan)
Massenspektrum: (M+H)⁺ = 305/7 (CI)

### 1 b) 4-Hydroxy-2-naphthalin-1-ylmethyl-5-nitro-2H-pyridazin-3-on

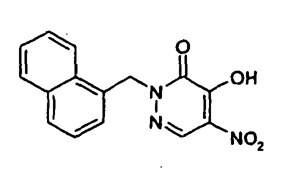

Zu einer Lösung von 12 g (39.3 mMol) 4,5-Dichlor-2-naphthalin-1-ylmethyl-2H-pyridazin-3-on in 120 ml Dimethylformamid wurde eine Lösung von 11.04 g (160 mMol) Natriumnitrit in 40 ml Wasser gegeben und das Gemisch 24 Stunden bei 85°C gerührt. Dann wurde im Vakuum eingeengt und der Rückstand mit einer Mischung aus 30 ml halbkonzentrierter Salzsäure und 30 ml Ethanol verrührt, wobei das Produkt kristallisierte. Es wurde abgesaugt, mit Ethanol gewaschen und getrocknet.
Ausbeute: 81.7% der Theorie
C₁₅H₁₁N₃O₄ (297.27)
Rf-Wert: 0.32 (Kieselgel, Dichlormethan/Ethanol 19 : 1)

### 1 c) 4-Amino-2-naphthalin-1-ylmethyl-5-nitro-2H-pyridazin-3-on

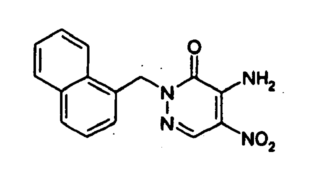

9.4 g (31.6 mMol) 4-Hydroxy-2-naphthatin-1-ylmethyl-5-nitro-2H-pyridazin-3-on wurden mit 150 ml gesättigter methanolischer Ammoniaklösung versetzt und in der Rothbombe 24 Stunden auf 130°C erhitzt. Das Gemisch wurde am Rotationsverdampfer auf ca. 40 ml Volumen eingengt, das ausgefallene Produkt abgesaugt und aus Tetrahydrofuran umkristallisiert.
Ausbeute: 53.4% der Theorie
C₁₅H₁₂N₄O₃ (296.29)
Rf-Wert: 0.68 (Kieselgel, Dichlormethan/Ethanol 50 : 1)
Massenspektrum: (M + H)⁺ = 297
(M-H)⁻ = 295

### 1 d) 4,5-Diamino-2-naphthalin-1-ylmethyl-2H-pyridazin-3-on

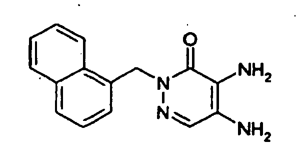

5 g (16.88 mMol) 4-Amino-2-naphthalin-1-ylmethyl-5-nitro-2H-pyridazin-3-on, gelöst in 150 ml Tetrahydrofuran, wurden unter Zusatz von 250 mg Platinoxid in einer Parr-Apparatur bei Raumtemperatur und 2 atm H₂ reduziert. Der Katalysator wurde abfiltriert, das Filtrat eingeengt und das so erhaltene Rohprodukt ohne weitere Reinigung weiterverarbeitet.
Ausbeute: 99% der Theorie
C₁₅H₁₄N₄O (266.3)
Rf-Wert: 0.14 (Kieselgel, Dichlormethan/Ethanol 19:1)

### 1 e) 2-Mercapto-5-(naphthalin-1-ylmethyl)-3,5-dihydro-imidazo[4,5-d]pyridazin-4-on

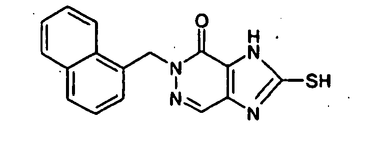

Zu einer Lösung von 4.4 g (16.5 mmol) 4,5-Diamino-2-naphthalin-1-ylmethyl-2H-pyridazin-3-on in 100 ml Tetrahydrofuran wurden 4.99 g (28.0 mmol) N,N'-Thiocarbonyldiimidazol gegeben und über Nacht bei Raumtemperatur gerührt. Dann wurde im Vakuum eingedampft, der Rückstand mit ca. 30 ml Wasser versetzt, mit Salzsäure schwach angesäuert, das ausgefallene Produkt abgesaugt, mit Wasser gewaschen und getrocknet.
Ausbeute: 98% der Theorie
C₁₆H₁₂N₄OS (308.36)
Rf-Wert: 0.22 (Kieselgel, Dichlormethan/Ethanol 19 : 1)
Massenspektrum: (M - H)⁻ = 307

### 1 f) 2-Methylsulfanyl-5-(naphthalin-1-ylmethyl)-3,5-dihydro-imidazo[4,5-d]pyridazin-4-on

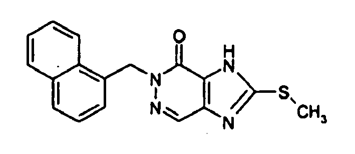

Zu einer Suspension von 5.3 g (17.19 mMol) 2-Mercapto-5-(naphthalin-1-ylmethyl)-3,5-dihydro-imidazo[4,5-d]pyridazin-4-on in 100 ml Dichlormethan und 100 ml Methanol wurden 2.38 g (17.2 mMol) Kaliumcarbonat und 1.07 ml (17.20 mMol) Jodmethan gegeben und über Nacht bei Raumtemperatur gerührt. Anschließend wurde das Lösungsmittel abgedampft, der Rückstand mit ca. 30 ml Wasser versetzt, mit 2N Salzsäure angesäuert, das so erhaltene Produkt abgesaugt, mit Wasser nachgewaschen und getrocknet.

Ausbeute: 54.1 % der Theorie
C₁₇H₁₄N₄OS (322.39)
Rf-Wert: 0.70 (Kieselgel, Dichlormethan/Ethanol 50:1)
Massenspektrum: (M + H)⁺ = 323
¹H-NMR-Spektrum (d₆-DMSO): δ = 2.70 (s, 3H); 5.81 (s, 2H); 7.20 (dd, 1H); 7.43 (t, 1 H); 7.57 (m, 2H); 7.86 (dd, 1 H); 7.95 (dd, 1H); 8.29 (dd, 1 H); 8.38 (s, 1 H), 13.85 (breites s, 1 H) ppm.

### 1 g) 3-Benzyl-2-methylsulfanyl-5-(naphthalin-1-ylmethyl)-3,5-dihydro-imidazo[4,5-d]pyridazin-4-on

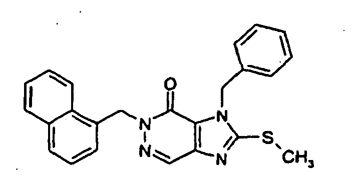

Eine Lösung von 1.0 g (3.10mMol) 2-Methylsulfanyl-5-(naphthalin-1-ylmethyl)-3,5-dihydro-imidazo[4,5-d]pyridazin-4-on in 15 ml Dimethylformamid wurde mit 547 mg (3.20 mMol) Benzylbromid und dann mit 442 mg (3.20 mMol) Kaliumcarbonat versetzt und über Nacht bei Raumtemperatur gerührt. Dann wurde mit ca. 40 ml Wasser verdünnt und dreimal mit je 15 ml Essigester extrahiert. Die organischen Extrakte wurden mit Wasser gewaschen, über Natriumsulfat getrocknet und eingeengt. Das so erhaltene Rohprodukt wurde durch Säulenchromatographie (Kieselgel; Elutionsmittel: Petrolether mit 10 - 20% Essigester) gereinigt.
Ausbeute: 54.7% der Theorie
C₂₄H₂₀N₄OS (412.52)
Rf-Wert: 0.77 (Kieselgel, Petrolether/Essigester 1 : 1)
Massenspektrum: (M + H)⁺ = 413

### 1 h) 3-Benzyl-2-methylsulfonyl-5-(naphthalin-1-ylmethyl)-3,5-dihydro-imidazo[4,5-d]pyridazin-4-on

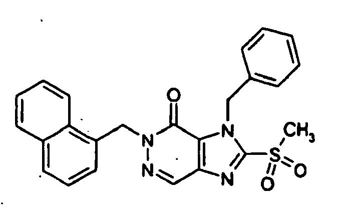

Eine Lösung von 700 mg (1.70 mmol) 3-Benzyl-2-methylsulfanyl-5-(naphthalin-1-ylmethyl)-3,5-dihydro-imidazo[4,5-d]pyridazin-4-on in 30 ml konzentrierter Essigsäure wurde unter Rühren bei Raumtemperatur tropfenweise mit einer Lösung von 395 mg (2.50 mMol) Kaliumpermanganat versetzt und weitere zwei Stunden gerührt. Da die Oxidation noch nicht vollständig war, wurden weitere 150 mg Kaliumpermanganat, gelöst in 5 ml Wasser, zugegeben und nochmals zwei Stunden gerührt. Die Reaktionslösung wurde danach mit 0.5 g Natriumhydrogensulfit versetzt, dann mit ca. 40 ml Wasser verdünnt und dreimal mit je 20 ml Dichlormethan extrahiert. Die organischen Extrakte wurden mit 5%iger Natriumhydrogensulfit-Lösung, dann mit Wasser gewaschen und über Natriumsulfat getrocknet. Das nach dem Einengen gewonnene Rohprodukt wurde durch Säulenchromatographie (Kieselgel; Elutionsmittel: Dichlormethan mit 1% Ethanol) gereinigt.
Ausbeute: 55.7% der Theorie
C₂₄H₂₀N₄O₃S (444.52)
Rf-Wert: 0.41 (Kieselgel, Petrolether/Essigester 7 : 3)
Massenspektrum: (M + H)⁺ = 445

### 1 i) [1-(1-Benzyl-6-naphthalin-1-ylmethyl-7-oxo-6,7-dihydro-1H-imidazo[4,5-d]pyridazin-2-yl)-piperidin-3-yl]-carbaminsäure-tert.-butylester

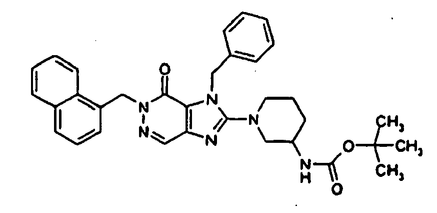

200 mg (0.45 mMol) 3-Benzyl-2-methylsulfonyl-5-(naphthalin-1-ylmethyl)-3,5-dihydro-imidazo[4,5-d]pyridazin-4-on und 600 mg (3.0 mMol) Piperidin-3-yl-carbaminsäure-tert.-butylester wurden zusammen unter Stickstoff 16 Stunden bei 150°C gerührt. Das Reaktionsgemisch wurde dann in 30 ml Dichlormethan gelöst, die Lösung mit 1 N Natronlauge gewaschen und über Natriumsulfat getrocknet. Das nach dem Einengen erhaltene Rohprodukt wurde durch Säulenchromatographie (Kieselgel; Elutionsmittel: Dichlormethan mit 1 - 2% Ethanol) gereinigt. Ausbeute: 26.6% der Theorie
C₃₃H₃₆N₆O₃ (564.69)
Rf-Wert: 0.59 (Kieselgel, Dichlormethan/Ethanol 19:1)
Massenspektrum: (M + H)⁺ = 565

### 1 j) 2-(3-Amino-piperidin-1-yl)-3-benzyl-5-(naphthalin-1-ylmethl)-3,5-dihydro-imidazo[4,5-d]pyridazin-4-on-Hydrochlorid

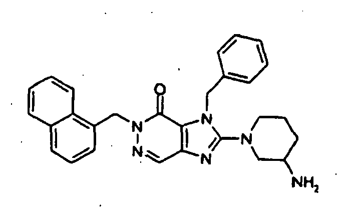

Eine Lösung von 60 mg (0.106 mMol) [1-(1-Benzyl-6-naphthalin-1-ylmethyl-7-oxo-6,7-dihydro-1H-imidazo[4,5-d]pyridazin-2-yl)-piperidin-3-yl]-carbaminsäure-tert.-butylester in 5 ml Dichlormethan wurde mit 0.5 ml Trifluoressigsäure versetzt und zwei Stunden bei Raumtemperatur gerührt. Das Gemisch wurde zur Trockne eingedampft, der Rückstand in 5 ml Dichlormethan gelöst und die Lösung mit 1 N Natronlauge und Wasser gewaschen und dann über Natriumsulfat getrocknet. Es wurde erneut eingedampft, der Rückstand in einer Mischung aus je 3 ml Diethylether und Aceton gelöst und durch Zutropfen von etherischer Salzsäure das Hydrochlorid des Produktes ausgefällt. Dieses wurde abgesaugt und getrocknet. Ausbeute: 37.7% der Theorie
C₂₈H₂₈N₆O x HCl (501.04)
Rf-Wert: 0.22 (Kieselgel, Dichlormethan/Ethanol 19 : 1)
Massenspektrum: (M + H)⁺ = 465

### Beispiel 2

### 2-(3-Amino-piperidin-1-yl)-3-(but-2-inyl)-5-(naphthalin-1-ylmethyl)-3,5-dihydro-imidazo[4,5-d]pyridazin-4-on

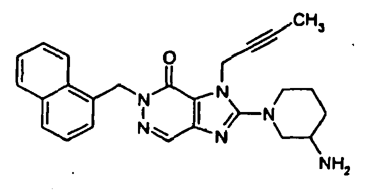

### 2 a) 3-(But-2-inyl)-2-methylsulfanyl-5-(naphthalin-1-ylmethyl)-3,5-dihydro-imidazo[4,5-d]pyridazin-4-on

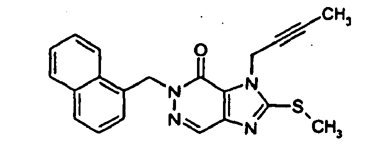

Eine Lösung von 900 mg (2.79 mMol) 2-Methylsulfanyl-5-(naphthalin-1-ylmethyl)-3,5-dihydro-imidazo[4,5-d]pyridazin-4-on (Beispiel 1f) in 15 ml Dimethylformamid wurde mit 415 mg (3.0 mMol) Kaliumcarbonat und 399 mg (3.0 mMol) 1-Brom-2-butin versetzt und acht Stunden bei Raumtemperatur gerührt. Dann wurde das Gemisch mit ca. 30 ml Wasser verdünnt und mit Natriumchlorid gesättigt, wobei das Reaktionsprodukt auskristallisierte. Es wurde abgesaugt und durch Säulenchromatographie (Kieselgel, Elutionsmittel: Petrolether mit 10 - 50% Essigester) gereinigt.
Ausbeute: 71.7% der Theorie
C₂₁H₁₈N₄OS (374.47)
Rf-Wert: 0.69 (Kieselgel, Petrolether/Essigester 1:1)
Massenspektrum: (M + H)⁺ = 375

### 2 b) 3-(But-2-inyl)-2-methylsulfonyl-5-(naphthalin-1-ylmethyl)-3,5-dihydro-imidazo[4,5-d]pyridazin-4-on

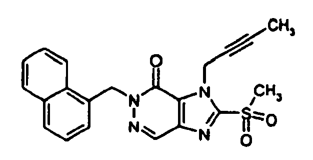

Eine Lösung von 600 mg (1.60 mmol) 3-(But-2-inyl)-2-methylsulfanyl-5-(naphthalin-1-ylmethyl)-3,5-dihydro-imidazo[4,5-d]pyridazin-4-on in 30 ml Eisessig wurde tropfenweise unter Rühren bei Raumtemperatur mit einer Lösung von 500 mg Kaliumpermanganat in 20 ml Wasser versetzt. Nach drei Stunden bei Raumtemperatur wurde eine Natriumhydrogensulfitlösung zugetropft, bis das Reaktionsgemisch nahezu wieder entfärbt war. Dann wurde mit ca. 50 ml Wasser verdünnt und mit Natriumchlorid gesättigt. Das dabei ausgefallene Rohprodukt wurde abgesaugt und durch Säulenchromatographie (Aluminiumoxid; Elutionsmittel: Dichlormethan) gereinigt.
Ausbeute: 43.0% der Theorie
C₂₁H₁₄N₄O₃S (406.47)
Rf-Wert: 0.70 (Kieselgel, Dichlormethan/Ethanol 19 : 1)
Massenspektrum: (M + H)⁺ = 407
¹H-NMR-Spektrum (d₆-DMSO): δ = 1.80 (s, 3H); 3.60 (s, 3H); 5.61 (s, 2H); 5.85 (s, 2H); 7.30 (dd, 1 H); 7.45 (t, 1 H); 7.58 (m, 2H); 7.90 (dd, 1 H); 7.96 (dd, 1H); 8.30 (dd, 1 H); 8.64 (s, 1H) ppm.

### 2 c) [1-(1-(But-2-inyl)-6-naphthalin-1-ylmethyl-7-oxo-6,7-dihydro-1H-imidazo[4,5-d]pyridazin-2-yl)-piperidin-3-yl]-carbaminsäure-tert.-butylester

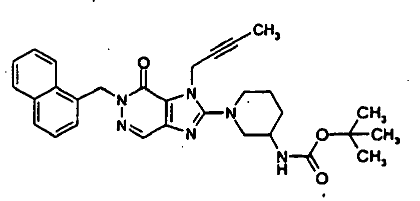

Eine Mischung aus 260 mg (0.64 mMol) 3-(But-2-inyl)-2-methylsulfonyl-5-(naphthalin-1-ylmethyl)-3,5-dihydro-imidazo[4,5-d]pyridazin-4-on und 800 mg (3.99 mMol) Piperidin-3-yl-carbaminsäure-tert.-butylester wurde unter Stickstoff zwei Stunden bei 150°C gerührt. Nach dem Abkühlen wurde in ca. 15 ml Dichlormethan gelöst, mit verdünnter Ammoniaklösung gewaschen und über Natriumsulfat getrocknet: Das nach dem Einengen erhaltene Rohprodukt wurde durch Säulenchromatographie (Kieselgel; Elutionsmittel: Dichlormethan mit 1- 5% Ethanol) gereinigt
Ausbeute: 35.6% der Theorie
C₃₀H₃₄N₆O₃ (526.64)
Rf-Wert: 0.53 (Kieselgel, Dichlormethan/Ethanol 19 : 1)
Massenspektrum: (M + H)⁺ = 527

### 2 d) 2-(3-Amino-Piperidin-1-yl)-3-(but-2-inyl)-5-(naphthalin-1-ylmethyl)-3,5-dihydro-imidazo[4,5-d]pyridazin-4-on - Hydrochlorid

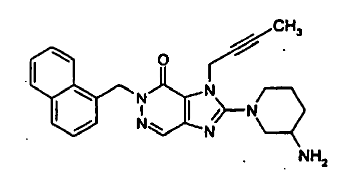

Eine Lösung von 120 mg (0.23 mMol) [1-(1-(But-2-inyl)-6-naphthalin-1-ylmethyl-7-oxo-6,7-dihydro-1H-imidazo[4,5-d]pyridazin-2-yl)-piperidin-3-yl]-carbaminsäure-tert.-butylester und 1.0 ml Trifluoressigsäure in 10 ml Dichlormethan wurde bei Raumtemperatur drei Stunden gerührt und anschließend zur Trockne eingedampft: Der Rückstand wurde in 15 ml Dichlormethan gelöst, die Lösung mit 1 N Natronlauge gewaschen, über Natriumsulfat getrocknet und eingengt. Das so erhaltene Rohprodukt wurde durch Säulenchromatographie (Kieselgel; Elutionsmittel: Dichlormethan mit 2 - 5% Ethanol) gereinigt. Das Produkt wurde in 8 ml Essigester gelöst, und durch Zutropfen von etherischer Salzsäure wurde das Hydrochlorid gefällt, abgesaugt und getrocknet.
Ausbeute: 66.3% der Theorie
C₂₅H₂₆NO x HCl (462.99)
Rf-Wert: 0.22 (Kieselgel, Dichlormethan/Ethanol 9 : 1)
Massenspektrum: (M + H)⁺ = 427
¹H-NMR-Spektrum (d₆-DMSO): δ= 1.69 (m, 2H); 1.80 (s, 3H); 1.93 (m, 1H): 2.07 (m, 1H); 3.20 (m, 2H); 3.40 (m, 1H); 3.52 (m, 1H): 3.73 (m, 1H): 5.19 (m, 2H); 5.80 (s, 2H); 7.22 (d, 1H); 7.45 (t, 1H): 7.57 (m, 2H); 7.88 (dd, 1H); 7.96 (d, 1H); 8.29 (d, 1H); 8.31 (s, 1H); 8.40 (breites s, 3H) ppm.

### Beispiel 3

### 3,5-Dibenzyl-2-(piperazin-1-yl)-3,5-dihydro-imidazo[4,5-c]pyridin-4-on

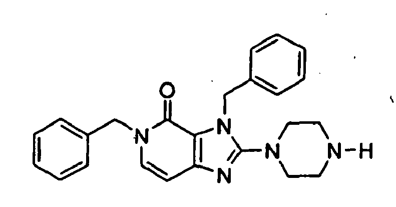

### 3 a) 2,4-Dichlor-3-nitropyridin

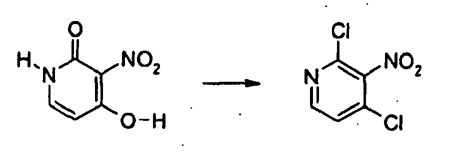

Eine Lösung von 30.0 g (0.192 Mol) 2,4-Dihydroxy-3-nitropyridin in 300 ml Phosphoroxychlorid wurde 50 Stunden zum Rückfluß erhitzt, dann ca. 200 ml Phosphoroxychlorid abdestilliert und der Rückstand mit Eiswasser (ca. 300 ml) zersetzt. Die so erhaltene dunkle Lösung wurde zweimal mit je 150 ml Essigester extrahiert, die organischen Phasen mit gesättigter Natriumchlorid-Lösung gewaschen, getrocknet und eingeengt. Das so erhaltene Rohprodukt wurde durch Säulenchromatographie gereinigt (Kieselgel, Elutionsmittel: Dichlormethan).
Ausbeute: 75% der Theorie.
Rf-Wert: 0.88 (Kieselgel, Dichlormethan/Ethanol = 9:1)
Massenspektrum: M⁺ = 192/4/6

### 3 b) 4-Amino-2-chlor-3-nitropyridin

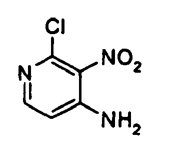

Eine Lösung von 28.0 g (0.193 Mol) 2,4-Dichlor-3-nitropyridin in 300 ml mit Ammoniak gesättigtem Ethanol wurde bei Raumtemperatur vier Tage lang gerührt, dann zur Trockne eingedampft und das so erhaltene Rohprodukt durch Säulenchromatographie gereinigt (Kieselgel, Elutionsmittel: Dichlormethan mit 0 - 5 % Ethanol). Ausbeute: 71% der Theorie.
C₅H₄ClN₃O₂ (173.56)
Massenspektrum: (M + H)⁺ 174/6

### 3 c) 4-Amino-2-hydroxy-3-nitropyridin

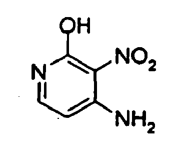

Eine Lösung von 18.0 g (104 mMol) 4-Amino-2-chlor- 3-nitropyridin in 120 ml Dimethylsulfoxid und 30 ml Wasser wurde vier Stunden bei 130°C gerührt. Die Lösung wurde dann abgekühlt und über Nacht unter Eiskühlung stehengelassen. Das dabei auskristallisierte Produkt wurde abgesaugt, mit wenig Wasser gewaschen und bei 50°C getrocknet.
Ausbeute: 69% der Theorie.
C₅H₅N₃O₃ (155.11)

| | |
|---|---|
| Massenspektrum: | (M + H)⁺ = 156 |
| | (M-H)- = 154 |

### 3 d) 3,4-Diamino-2-hydroxypyridin

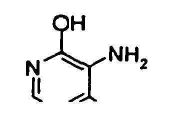

11.0 g (71 mMol) 4-Amino-2-hydroxy-3-nitropyridin wurden in 150 ml Dimethylformamid gelöst und bei Raumtemperatur durch katalytische Hydrierung (Pd/C 10%) reduziert.
Ausbeute: 83% der Theorie.
C₅H₇N₃O (125.13)
Massenspektrum: (M + H)⁺ = 126

### 3 e) 2-Mercapto-3,5-dihydro-imidazo[4,5-c]pyridin-4-on

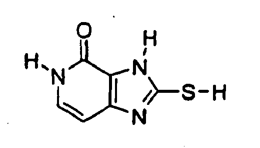

Eine Suspension von 5.0 g (39.96 mmol) 3,4-Diamino-2-hydroxypyridin und 12.82 g (80.0 mmol) Kalium-ethylxanthogenat in 100 ml Ethanol wurde drei Stunden unter Rückfluß erhitzt. Das Gemisch wurde dann auf Raumtemperatur abgekühlt und mit ca. 20 ml Diethylether versetzt. Das ausgefallene Produkt wurde abfiltriert, mit ca. 10 ml Diethylether gewaschen, getrocknet, in ca. 30 ml Wasser gelöst und diese Lösung mit konzentrierter Salzsäure angesäuert. Das dabei ausgefallene Produkt wurde abgesaugt, mit 15 ml Wasser gewaschen und bei 50°C getrocknet. Ausbeute: 82% der Theorie.
C₆H₅N₃OS (167.19)

| | |
|---|---|
| Massenspektrum: | (M + H)⁺ = 168 |
| | (M - H)⁻ = 166 |

### 3 f) 2-Methylmercapto-3,5-dihydro-imidazo[4,5-c]pyridin-4-on

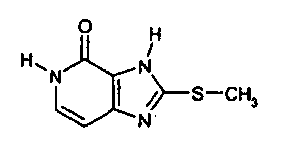

Zu einer Suspension von 5.30 g (31.7 mMol) 2-Mercapto-3,5-dihydro-imidazo[4,5-c]pyridin-4-on in 100 ml Dichlormethan und 50 ml Methanol wurden 4.38 g (31.7 mMol) Kaliumcarbonat und 1.97 ml (31.7 mMol) Methyljodid gegeben und das Gemisch über Nacht bei Raumtemperatur gerührt. Dann wurden weitere 15 ml Methanol zugegeben und ungelöste Bestandteile abfiltriert. Das Filtrat wurde eingedampft und das so erhaltene Rohprodukt durch Säulenchromatographie gereinigt (Kieselgel, Elutionsmittel: Dichlormethan mit 5-25% Ethanol).
Ausbeute: 96% der Theorie.
C₇H₇N₃OS (181.22)
Rf-Wert: 0.53 (Kieselgel, Dichlormethan/Ethanol 9 : 1)
Massenspektrum: (M + H)⁺ = 182
¹H-NMR-Spektrum (d₆-DMSO): δ = 2.62 (s, 3H); 6.40 (breites s, 1 H); 7.03 (d, 1 H); 11.12 (breites s, 1 H); 12.95 (breites d, 1 H) ppm.

### 3 g) 3,5-Dibenzyl-2-methylmercapto-3,5-dihydro-imidazo[4,5-c]pyridin-4-on

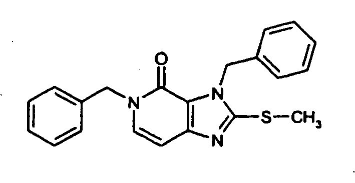

Zu einer Lösung von 362 mg (2.0 mMol) 2-Methylmercapto-3,5-dihydro-imidazo-[4,5-c]pyridin-4-on in 5.0 ml Dimethylformamid wurden 553 mg (4.0 mMol) Kaliumcarbonat und 0.48 ml (4.0 mMol) Benzylbromid gegeben und diese Mischung drei Stunden bei Raumtemperatur gerührt. Anschließend wurde mit 10 ml Wasser verdünnt und dreimal mit je 10 ml Essigester extrahiert. Die organischen Extrakte wurden getrocknet und eingeengt, das so erhaltene Rohprodukt durch Säulenchromatographie gereinigt (Kieselgel, Elutionsmittel: Dichlormethan mit 0-3% Ethanol).
Ausbeute: 26% der Theorie.
C₂₁H₁₉N₃OS (361.47)
Rf-Wert: 0.62 (Kieselgel, Dichlormethan/Ethanol 19 : 1)
Massenspektrum: (M + H)⁺ = 362
¹H-NMR-Spektrum (d₆-DMSO): δ = 2.67 (s, 3H); 5.21 (s, 2H); 5.62 (s, 2H); 6.63 (d, 1 H); 7.20 - 7.37 (m, 10 H); 7.56 (d, 1H) ppm.

### 3 h) 3,5-Dibenzyl-2-methansulfonyl-3,5-dihydro-imidazo[4,5-c]pyridin-4-on

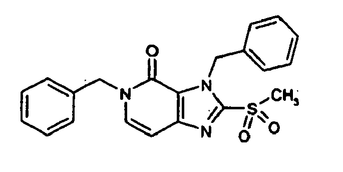

Zu einer Lösung von 181 mg (0.50 mMol) 3,5-Dibenzyl-2-methylmercapto-3,5-dihydro-imidazo[4,5-c]pyridin-4-on in 10 ml Dichlormethan wurde bei Raumtemperatur unter Rühren eine Lösung von 190 mg (1.10 mMol) 3-Chlor-peroxybenzoesäure in 5 ml Dichlormethan tropfenweise zugegeben. Nach beendeter Zugabe wurde noch weitere 30 Minuten gerührt, dann das Reaktiongemisch mit ca. 25 ml 5%iger Natriumhydrogencarbonatlösung ausgeschüttelt, die organische Phase abgetrennt, über Natriumsulfat getrocknet und eingedampft. Das so erhaltene Rohprodukt, in dem ca. 20% der Methansulfinyl-Verbindung enthalten war, wurde ohne weitere Reinigung weiterverarbeitet.
Ausbeute: ca. 75% der Theorie
C₂₁H₁₉N₃O₃S (393.47)
Rf-Wert: 0.66 (Kieselgel, Dichlormethan/Ethanol 19:1)
Massenspektrum: (M + H)⁺ = 394

### 3 i) 3,5-Dibenzyl-2-(piperazin-1-yl)-3,5-dihydro-imidazo[4,5-c]pyridin-4-on

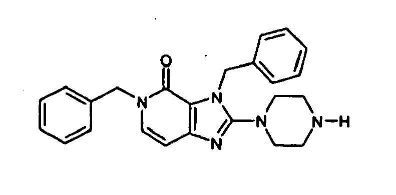

860 mg (10 mMol) Piperazin wurden unter Kühlung tropfenweise mit 660 mg (11 mMol) Eisessig versetzt, dann 180 mg 3,5-Dibenzyl-2-methansulfonyl-3,5-dihydro-imidazo[4,5-c]pyridin-4-on (Rohprodukt aus Beispiel 3 h) hinzugefügt und das Gemisch 24 Stunden bei 150°C geführt. Nach dem Abkühlen wurden ca. 10 ml Wasser zugegeben, mit konzentrierter Ammoniaklösung alkalisch gestellt und das Gemisch dreimal mit je 5 ml Dichlormethan extrahiert. Die Extrakte wurden über Natriumsulfat getrocknet und eingedampft. Das so erhaltene Rohprodukt wurde durch Säulenchromatographie (Kieselgel; Elutionsmittel: Petrolether mit 20 - 60% Essigester) gereinigt.
Ausbeute: 5.5% der Theorie
C₂₄H₂₅N₅O (399.50)
Rf-Wert: 0.28 (Kieselgel, Petrolether/Essigester 7 : 3)
Massenspektrum: (M + H)⁺ = 400

### Beispiel 4

### 2-(3-Amino-piperidin-1-yl)-5-benzyl-3-(but-2-inyl)-3,5-dihydro-imidazo[4,5-c]pyridin-4-on

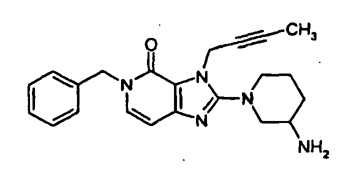

### 4 a) 1-Benzyl-4-benzyloxy-3-nitro-pyridin-2-on

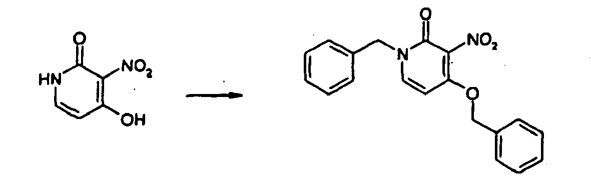

Eine Lösung von 4.68 g (30 mMol) 2,4-Dihydroxy-3-nitro-pyridin in 100 ml Dimethylformamid wurde bei Raumtemperatur portionsweise mit 2.88 g (60 mMol) einer 50%igen Suspension von Natriumhydrid in Paraffinöl versetzt und 15 Minuten gerührt. Dann wurden 8.91 ml (75 mMol) Benzylbromid hinzugefügt und 24 Stunden bei 80°C gerührt. Die Lösung wurde dann vorsichtig in ca. 250 ml Wasser eingerührt und dreimal mit je 70 ml Essigester extrahiert. Die Extrakte wurden mit gesättigter Natriumchlorid-Lösung gewaschen, getrocknet und im Vakuum eingeengt. Der Rückstand wurde mit Diethylether verrieben, abgesaugt und getrocknet. Das so erhaltene Produkt wurde ohne Reinigung weiterverarbeitet.
Ausbeute: 59% der Theorie.
C₁₉H₁₆N₂O₄ (336.35)
Massenspektrum: (M+H)⁺ = 337

### 4 b) 4-Amino-1-benzyl-3-nitro-pyridin-2-on

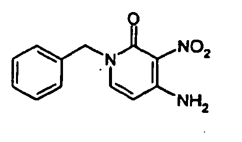

Zu einer Lösung von 5.5 g (16.3 mMol) 1-Benzyl-4-benzyloxy-3-nitro-pyridin-2-on in 50 ml Dichlormethan wurden 20 ml einer gesättigten ethanolischen Ammoniaklösung gegeben und im geschlossenen Kolben 3 Tage bei Raumtemperatur gerührt. Die Lösung wurde zur Trockne eingedampft, der Rückstand mit Diethylether verrieben, abgesaugt und getrocknet.
Ausbeute: 97% der Theorie.
C₁₂H₁₁N₃O₃ (245.24)
Rf-Wert: 0.31 (Kieselgel; Dichlormethan / Ethanol 19 : 1)

### 4 c) 1-Benzyl-3,4-diamino-Pyridin-2-on

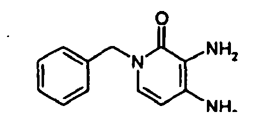

3.9 g (15.9 mMol) 4-Amino-1-benzyl-3-nitro-pyridin-2-on, gelöst in 250 ml Tetrahydrofuran, wurden über Platinoxid bei 50 psi H₂-Druck und Raumtemperatur hydriert. Nach Abfiltrieren des Katalysators wurde das Filtrat eingedampft und der Rückstand roh weiterverarbeitet.
Ausbeute: 99% der Theorie.
C₁₂H₁₃N₃O (215.26)

### 4 d) 5-Benzyl-2-mercapto-3,5-dihydro-imidazo[4,5-c]pyridin-4-on

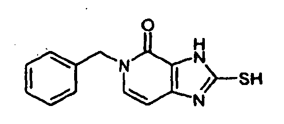

Eine Lösung von 3.40 g (15.8 mMol) 1-Benzyl-3,4-diamino-pyridin-2-on und 4.99 g (28.0 mMol) N,N'-Thiocarbonyl-diimidazol in 200 ml Tetrahydrofuran wurde über Nacht bei Raumtemperatur gerührt, dann eingedampft, der Rückstand mit ca. 50 ml Wasser versetzt, mit 2N Salzsäure auf pH 7 eingestellt und diese Lösung ca. 30 Minuten bei Raumtemperatur gerührt. Das dabei ausgefallene Produkt wurde abgesaugt, mit ca. 50 ml Wasser gewaschen und bei 50°C getrocknet.
Ausbeute: 98% der Theorie.
C₁₃H₁₁N₃OS (257.32)
Rf-Wert:: 0.45 (Kieselgel; Dichlormethan / Ethanol 9 : 1)

### 4 e) 5-Benzyl-2-methylsulfanyl-3,5-dihydro-imidazo[4,5-c]pyridin-4-on

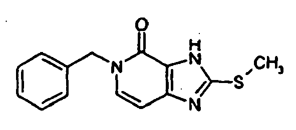

4.0 g (15.55 mMol) 5-Benzyl-2-mercapto-3,5-dihydro-imidazo[4,5-c]pyridin-4-on wurden in einer Mischung aus 100 ml Dichlormethan und 50 ml Methanol suspendiert, dann 2.15 g (15.55 mMol) Kaliumcarbonat und 0.97 ml (15.55 mMol) Jodmethan zugegeben und 1 Stunde bei Raumtemperatur gerührt. Anschließend wurde filtriert, das Filtrat eingeengt und der Rückstand roh weiter umgesetzt. Ausbeute: 94% der Theorie.
C₁₄H₁₃N₃OS (271.34)
Rf-Wert:: 0.49 (Kieselgel; Dichlormethan / Ethanol 9 : 1)
Massenspektrum: (M+H)⁺ = 272

### 4 f) 5-Benzyl-3-(but-2-inyl)-2-methylsulfanyl-3.5-dihydro-imidazo[4,5-c]pyridin-4-on

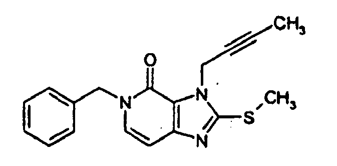

Zu einer Lösung von 4.0 g (14.7 mMol) 5-Benzyl-2-methylsulfanyl-3,5-dihydro-imidazo[4,5-c]pyridin-4-on und 1.31 ml (15.0 mMol) 1-Brom-2-butin in 100 ml Dimethylformamid wurden 2.07 g (15.0 mMol) Kaliumcarbonat gegeben und eine Stunde bei Raumtemperatur gerührt. Es wurden dann ca. 200 ml gesättigte Natriumchtorid-Lösung hinzugegeben und dreimal mit je 50 ml Essigester extrahiert. Die Extrakte wurden mit gesättigter Natriumchlorid-Lösung gewaschen, über Natriumsulfat getrocknet und eigedampft. Das so erhaltene Rohprodukt wurde durch Säulenchromatographie gereinigt (Kieselgel; Elutionsmittel: Dichlormethan mit 0 - 2% Ethanol).
Ausbeute: 48% der Theorie.
C₁₈H₁₇N₃OS (323.42)
Rf-Wert:: 0.71 (Kieselgel; Dichlormethan / Ethanol 9 : 1)
¹H-NMR-Spektrum (d₆-DMSO): δ = 1.78 (s, 3H); 2.70 (s, 3H); 5.18 (s, 2H); 5.23 (s, 2H); 6.61 (d, 1H); 7.23 - 7.38 (m, 5H); 7.53 (d, 1H) ppm.
Massenspektrum: (M+H)⁺ = 324

### 4 g) 5-Benzyl-3-(but-2-inyl)-2-methylsulfonyl-3,5-dihydro-imidazo[4,5-c]pyridin-4-on

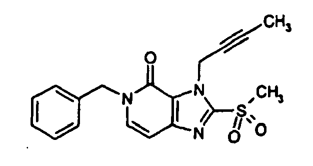

Zu einer Lösung von 400 mg (1.24 mMol) 5-Benzyl-3-(but-2-inyl)-2-methylsulfanyl-3,5-dihydro-imidazo[4,5-c]pyridin-4-on in 10 ml konzentrierter Essigsäure wurde langsam unter Rühren eine Lösung von 316 mg (2.0 mMol) Kaliumpermanganat in 10 ml Wasser getropft. Nach drei Stunden bei Raumtemperatur wurden 400 mg Natriumbisulfit (Na₂S₂O₅) hinzugefügt und mit ca. 30 ml Wasser verdünnt. Es wurde fünfmal mit je 30 ml Dichlormethan extrahiert, die Extrakte über Natriumsulfat getrocknet und eingeengt. Das so erhaltene Produkt wurde ohne Reinigung weiter umgesetzt.
Ausbeute: 50% der Theorie.
C₁₈H₁₇N₃O₃S (355.42)
Rf-Wert:: 0.40 (Kieselgel; Dichlormethan / Ethanol 19 : 1)
Massenspektrum: (M+H)⁺ = 356

### 4 h) [1-(5-Benzyl-3-(but-2-inyl)-4-oxo-4,5-dihydro-3H-imidazo[4,5-c]pyridin-2-yl)-piperidin-3-yl]-carbaminsäure-tert-butylester

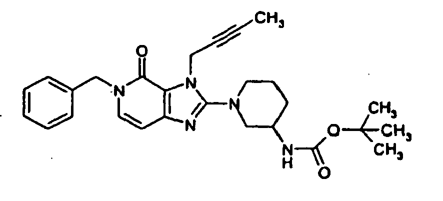

Ein Gemisch aus 220 mg (0.62 mMol) 5-Benzyl-3-(but-2-inyl)-2-methylsulfonyl-3,5-dihydro-imidazo[4,5-c]pyridin-4-on und 781.1 mg (3.90 mMol) Piperidin-3-yl-carbaminsäure-tert-butylester wurde unter Stickstoff 5 Stunden auf 170°C erhitzt. Nach dem Abkühlen auf Raumtemperatur wurde das so erhaltene Rohprodukt durch Säulenchromatographie gereinigt (Kieselgel; Elutionsmittel: Dichlormethan mit 0 - 3% Ethanol).
Ausbeute: 23% der Theorie
C₂₇H₃₃N₅O₃ (475.60)
Rf-Wert:: 0.32 (Kieselgel; Dichlormethan / Ethanol 19 : 1)

| | |
|---|---|
| Massenspektrum: | M⁺ = 475 |
| | (M + H)⁺ = 476 |

### 4 i) 2-(3-Amino-piperidin-1-yl)-5-benzyl-3-(but-2-inyl)-3,5-dihydro-imidazo[4,5-c]pyridin-4-on

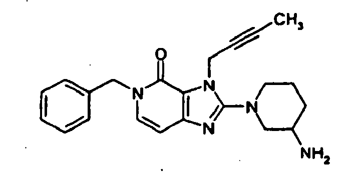

Eine Lösung von 70.0 mg (0.147 mMol) [1-(5-Benzyl-3-(but-2-inyl)-4-oxo-4,5-dihydro=3H-imidazo[4,5-c]pyridin-2-yl)-piperidin-3-yl]-carbaminsäure-tert-butylester und 0.5 ml Trifluoressigsäure in 3.0 ml Dichlormethan wurde eine Stunde bei Raumtemperatur gerührt. Das Gemisch wurde dann im Vakuum eingedampft und der Rückstand in ca. 5 ml Dichlormethan gelöst. Diese Lösung wurde mit ca. 5 ml 1 N Natronlauge gewaschen, dann über Natriumsulfat getrocknet und eingedampft. Der Rückstand wurde in ca. 3 ml Essigester gelöst, und durch Zutropfen von etherischer Salzsäure wurde das Produkt als Hydrochlorid gefällt, abgesaugt und getrocknet.
Ausbeute: 41% der Theorie.
C₂₂H₂₅N₅O x HCl (411.94)
Rf-Wert: 0.22 (Kieselgel; Dichlormethan / Ethanol 9 : 1)

| | |
|---|---|
| Massenspektrum: | M⁺ = 375 |
| | (M + H)⁺ = 376 |

### Beispiel 6

### 2-((R)-3-Amino-piperidin-1-yl)-3-(but-2-inyl)-5-{[1,2,4]triazolo[4,3-a]pyridin-3-ylmethyl}-3,5-dihydro-imidazo[4,5-d]pyridazin-4-on-Hydrochlorid

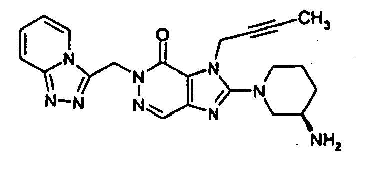

Hergestellt analog Beispiel 1j durch Abspaltung der Boc-Schutzgruppe mit Trifluoressigsäure in Dichlormethan.
Ausbeute: 72% der Theorie.
C₂₁H₂₃N₉O (417.48)
Massenspektrum: (M + H)⁺ = 418
¹H-NMR-Spektrum (d₆-DMSO): δ = 1.70 (m, 2H); 1.80 (s, 3H); 1.93 (m, 1 H); 2.02 (m, 1 H); 3.20 (m, 2H); 3.38 (m, 1 H); 3.50 (m, 1H); 3.74 (m, 1 H); 5.17 (m, 2H); 5.98 (s, 2H); 7.45 (t, 1H); 7.90 (dd, 1H); 8.02 (d, 1H); 8.33 (s, 1H); 8.50 (breites s, 3H); 8.98 (d, 1H) ppm.

### Beispiel 7

### 2-(3-Amino-piperidin-1-yl)-3-(but-2-inyl)-5-(3-methyl-isochinolin-1-ylmethyl)-3,5-dihydro-imidazo[4,5-d]pyridazin-4-on

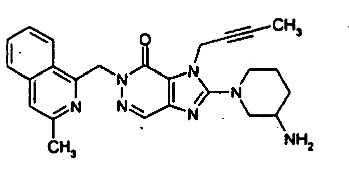

Hergestellt analog Beispiel 1j aus {1-[1-(But-2-inyl)-6-(3-methyl-isochinolin-1-ylmethyl)-7-oxo-6,7-dihydro-1*H*-imidazo[4,5-d]pyridazin-2-yl]-piperidin-3-yl}-carbaminsäure-tert-butylester und Trifluoressigsäure in Dichlormethan.
Ausbeute: 57% der Theorie.
C₂₅H₂₇N₇O x HCl (477.99)
Rf-Wert: 0.13 (Kieselgel; Dichlormethan / Ethanol 9:1)
Massenspektrum: (M + H)⁺= 442

### Beispiel 35

### 2-(3-Amino-piperidin-1-yl)-3-(but-2-inyl)-5-(naphthalin-1-ylmethyl)-3,5-dihydro-imidazo[4,5-c]pyridin-4-on

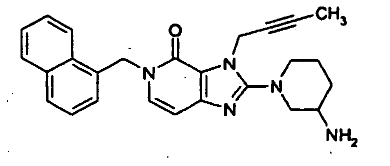

Hergestellt analog Beispiel 1j durch Abspaltung der Boc-Schutzgruppe mit Tifluoressigsäure in Dichlormethan.
Ausbeute: 92% der Theorie.
C₂₆H₂₇N₅O (425.54)
Massenspektrum: (M + H)⁺ = 426
Rf-Wert: 0.54 (Kieselgel; Dichlormethan / Methanol / konz. Ammoniak 8 : 2 : 0.1)

### Beispiel 86

### 2-(3-Amino-piperidin-1-yl)-3-(3-methyl-but-2-enyl)-5-(3-methyl-isochinolin-1-ylmethyl)-3,5-dihydro-imidazo[4,5-c]pyridin-4-on

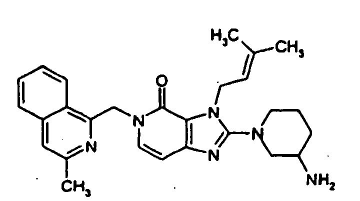

Hergestellt analog Beispiel 1j durch Abspaltung der Boc-Schutzgruppe mit Tifluoressigsäure in Dichlormethan.
Ausbeute: 84% der Theorie.
C₂₇H₃₂N₆O (456.59)
Massenspektrum: (M + H)⁺ = 457
¹H-NMR-Spektrum (d₆-DMSO): δ = 1.11 -1.25 (m, 1 H); 1.57 -1.90 (m, 9H); 2.50 - 2.65 (m, 1 H); 2.75 - 2.88 (m, 2H); 3.25 - 3.45 (m, 2H); 4.81 (d, 2H); 5.33 (t. 1 H); 5.78 (s, 2H); 6,48 (d, 1 H); 7.40 (d, 1H); 7.58 - 7.65 (m, 2H); 7.72 (t, 1 H); 7.89 (d, 1H); 8.40 (d, 1H) ppm.

### Beispiel 136

### 2-((R)-3-Amino-piperidin-1-yl)-3-(but-2-inyl)-5-(4-methyl-chinazolin-2-ylmethyl)-3,5-dihydro-imidazo[4,5-d]pyridazin-4-on

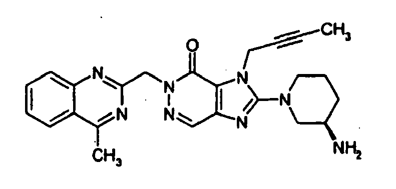

### 136 a) 2-Brom-1-(but-2-inyl)-1H-imidazol-4,5-dicarbonsäure-dimethylester

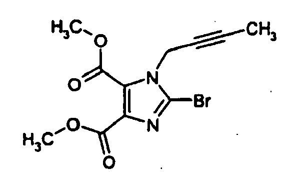

Eine Lösung von 15.0 g (57.0 mMol) 2-Brom-imidazol-4,5-dicarbonsäure-dimethylester, 5.15 ml (57.0 mMol) 1-Brom-2-butin und 50 ml N,N-Diisopropylethylamin in 280 ml Tetrahydrofuran wurde eine Stunde zum Rückfluß erhitzt. Das Gemisch wurde eingedampft, der Rückstand mit ca. 100 ml Wasser versetzt und dreimal mit je 70 ml Essigester extrahiert. Die Extrakte wurden mit 50 ml Wasser gewaschen, getrocknet und eingeengt. Das so erhaltene Rohprodukt wurde durch Säulenchromatographie gereinigt (Kieselgel; Elutionsmittel: Dichlormethan mit 0 - 2% Ethanol).
Ausbeute: 75% der Theorie.

C₁₁H₁₁BrN₂O₄ (315.13)
Rf-Wert:: 0.82 (Kieselgel; Dichlormethan / Ethanol 9 : 1)
Massenspektrum: (M + H)⁺ = 315/317

### 136 b) 2-Brom-3-(but-2-inyl)-5-formyl-3H-imidazol-4-carbonsäure-methylester

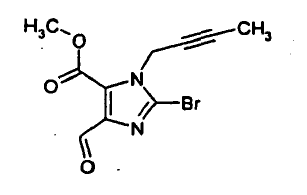

Zu einer Lösung von 13.5 g (42.84 mMol) 2-Brom-1-(but-2-inyl-1*H*-imidazol-4,5-dicarbonsäure-dimethylester in 220 ml Tetrahydrofuran wurden unter Argon-Atmosphäre bei -70°C 43 ml (43 mMol) einer 1-molaren Lösung von Diisobutylaluminiumhydrid in Tetrahydrofuran innerhalb 20 Minuten zugetropft. Es wurde weitere 4 Stunden bei -70°C gerührt, dann 20 ml einer Mischung aus 1M Salzsäure und Tetrahydrofuran zugetropft. Nach dem Erwärmen auf Raumtemperatur wurden ca. 200 ml Wasser hinzugegeben und dreimal mit je 70 ml Essigester extrahiert. Die Extrakte wurden getrocknet und eingeengt, und das so erhaltene Rohprodukt durch Säulenchromatographie gereinigt (Kieselgel; Elutionsmittel: Petrolether mit 20 - 50% Essigester).
Ausbeute: 52% der Theorie.
C₁₀H₉BrN₂O₃ (285.10)
Massenspektrum: (M+H)⁺ = 285/287
¹H-NMR-Spektrum (d₆-DMSO): δ = 1.80 (s, 3H); 3.93 (s, 3H); 5.11 (s, 2H); 10.12 (s, 1H) ppm.

### 136 c) 2-Brom-3-(but-2-inyl)-3,5-dihydro-imidazo[4,5-d]pyridazin-4-on

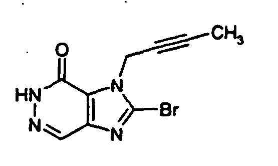

Zu einer Lösung von 1.80 g (6.31 mMol) 2-Brom-3-(but-2-inyl)-5-formyl-3*H-*imidazol-4-carbonsäure-methylester in 25 ml Ethanol wurden bei Raumtemperatur 0.31 ml Hydrazinhydrat (99%, 6.31 mMol), gelöst in 1 ml Ethanol, zugetropft. Fünf Minuten später wurden 1.5 ml konzentrierte Essigsäure zugefügt und 30 Minuten zum Rückfluß erhitzt. Nach dem Abkühlen wurde der ausgefallene Feststoff abgesaugt, mit 10 ml Ethanol und 20 ml Diethylether gewaschen und getrocknet. Ausbeute: 74% der Theorie.
C₉H₇BrN₄O (267.09)
Massenspektrum: (M+H)⁺ = 267/269
¹H-NMR-Spektrum (d₆-DMSO): δ = 1.80 (s, 3H); 5.28 (s, 2H); 8.38 (s, 1H); 12.99 (s, 1 H) ppm.

### 136 d) 2-Brom-3-(but-2-inyl)-5-(4-methyl-chinazolin-2-ylmethyl)-3,5-dihydro-imidazo[4,5-d]pyridazin-4-on

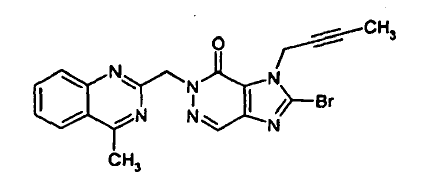

Zu einer Lösung von 300 mg (1.12 mMol) 2-Brom-3-(but-2-inyl)-3,5-dihydro-imidazo[4,5-d]pyridazin-4-on und 260.1 mg (1.35 mMol) 2-Chlormethyl-4-methylchinazolin in 4.0 ml Dimethylformamid wurden 489 mg (1.5 mMol) Cäsiumcarbonat gegeben und dieses Gemisch 2 Stunden unter Argon-Atmosphäre bei 80°C gerührt. Dann wurde mit 10 ml Wasser verdünnt und 30 Minuten bei Raumtemperatur gerührt. Das dabei ausgefallene Rohprodukt wurde abgesaugt und durch Säulenchromatographie gereinigt (Kieselgel; Elutionsmittel: Petrolether mit 20 - 50% Essigester).
Ausbeute: 51% der Theorie.
C₁₉H₁₅BrN₆O (423.28)

### 136 e) {1-[1-(But-2-inyl)-6-(4-methyl-chinazolin-2-ylmethyl)-7-oxo-6,7-dihydro-1H-imidazo[4,5-d]pyridazin-2-yl]-piperidin-3-yl}-carbaminsäure-tert-butylester

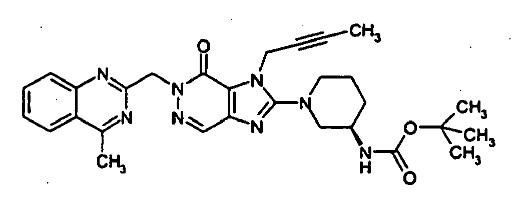

Eine Lösung von 240 mg (0.567 mMol) 2-Brom-3-(but-2-inyl)-5-(4_-methylchinazolin-2-ylmethyl)-3,5-dihydro-imidazo[4,5-d]pyridazin-4-on und 140.2 mg (0.70 mmol) (*R*)-Piperidin-3-yl-carbaminsäure-tert-butylester in 4 ml Dimethylsulfoxid wurde mit 95 mg (0.9 mMol) Natriumcarbonat versetzt und 3 Stunden bei 80°C gerührt. Dann wurden nochmals 50 mg Piperidin-3-yl-carbaminsäure-tert-butylester hinzugefügt und weitere 2 Stunden bei 80°C gerührt. Nach dem Abkühlen auf Raumtemperatur wurde mit 10 ml Wasser versetzt und 30 Minuten gerührt. Das dabei ausgefallene Produkt wurde abgesaugt, mit 5 ml Wasser gewaschen und getrocknet.
Ausbeute: 81 % der Theorie.
C₂₉H₃₄N₈O₃ (542.65)

### 136 f) 2-((R)-3-Amino-piperidin-1-yl)-3-(but-2-inyl)-5-(4-methyl-chinazolin-2-ylmethyl)-3,5-dihydro-imidazo[4,5-d]pyridazin-4-on

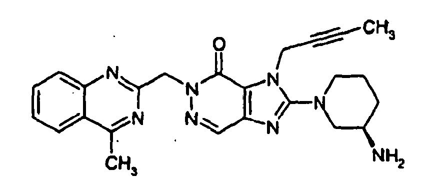

Hergestellt analog 1j aus 240 mg (0.442 mMol) {1-[1-(But-2-inyl)-6-(4-methylchinazolin-2-ylmethyl)-7-oxo-6,7-dihydro-1*H*-imidazo[4,5-d]pyridazin-2-yl]-piperidin-3-yl}-carbaminsäure-tert-butylester mit Trifluoressigsäure in Dichlormethan.
Ausbeute: 56% der Theorie.
C₂₄H₂₆N₈O (442.53)
Rf-Wert:: 0.42 (Kieseigel, Dichlormethan / Ethanol 9 : 1 unter Zusatz von 1 Tropfen konz. Ammoniak-Lösung)
Massenspektrum: (M+H)⁺ = 443
¹H-NMR-Spektrum (d₆-DMSO): δ = 1.23 - 1.37 (m, 1H); 1.60 - 1.95 (m, 6H); 2.75 - 3.10 (m, 6H); 3.58 - 3.72 (m, 2H); 5.10 (s, 2H); 5.61 (s, 2H); 7.70 (t, 1H); 7.82 (d, 1 H); 7.93 (t, 1 H); 8.28 (d, 1 H); 8.30 (s, 1H); ppm (austauschbare Protonen nicht sichtbar).

### Beispiel 138

### 2-((R)-3-Amino-piperidin-1-yl)-3-(but-2-inyl)-5-(benzo[d]isothiazol-3-ylmetyl)-3,5-dihydro-imidazo[4,5-d]pyridazin-4-on

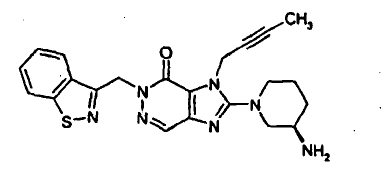

Hergestellt analog Beispiel 1j durch Abspaltung der Boc-Schutzgruppe mit Trifluoressigsäure in Dichlormethan.
Ausbeute: 83% der Theorie.
C₂₂H₂₃N₇OS (433.54)
Massenspektrum: (M + H)⁺ = 434
Rf-Wert: 0.15 (Kieselgel; Dichlormethan / Methanol 9 : 1)

### Beispiel 139

### 2-((R)-3-Amino-piperidin-1-yl)-3-(but-2-inyl)-5-(benzo[d]isoxazol-3-ylmethyl)-3,5-dihydro-imidazo[4,5-d]pyridazin-4-on

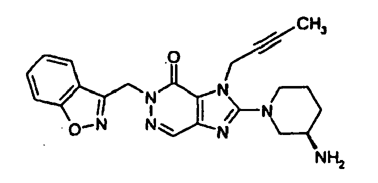

Hergestellt analog Beispiel 1j durch Abspaltung der Boc-Schutzgruppe mit Trifluoressigsäure in Dichlormethan.
Ausbeute: 67% der Theorie.
C₂₂H₂₃N₇O₂ (417.47)
Massenspektrum: (M + H)⁺ = 418
Rf-Wert: 0.40 (Kieselgel; Dichlormethan / Methanol / konz. Ammoniak 9 : 1 : 0.1)

### Beispiel 140

### 2-((R)-3-Amino-piperidin-1-yl)-3-(but-2-inyl)-5-(1-methyl-1H-indazol-3-ylmethyl)-3,5-dihydro-imidazo[4,5-d]pyridazin-4-on

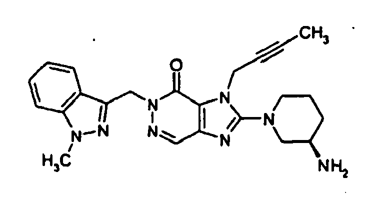

Hergestellt analog Beispiel 1j durch Abspaltung der Boc-Schutzgruppe mit Trifluoressigsäure in Dichlormethan.
Ausbeute: 90% der Theorie.
C₂₃H₂₆N₈O (430.52)
Massenspektrum: (M H)⁺ = 431
Rf-Wert: 0.15 (Kieselgel; Dichlormethan / Methanol 9:1)

### Beispiel 178

### 2-(3-Amino-piperidin-1-yl)-3-(but-2-inyl)-5-(2-oxo-2-phenyl-ethyl)-3,5-dihydro-imidazo[4,5-d]pyridazin-4-on

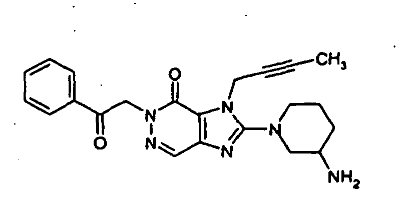

Hergestellt analog Beispiel 1j durch Abspalten der Boc-Schutzgruppe mit Trifluoressigsäure in Dichlormethan.
Ausbeute: 79% der Theorie.
C₂₃H₂₉N₆O₂ (420.51)
Massenspektrum: (M+H)⁺ = 421
¹H-NMR-Spektrum (d₆-DMSO): δ = 1.16 - 1.27 (m, 1H); 1.60 -1.95 (m, 9H); 2.65 (dd, 1H); 2.88 (m, 2H); 3.35 - 3.54 (m, 2H); 4.87 (d, 2H); 5.33 (t, 1 H); 5.70 (s, 2H); 7.60 (t, 2H); 7.72 (t, 1H); 8.07 (d, 2H); 8.30 (s, 1 H) ppm.

Analog den vorstehend genannten Beispielen und anderen literaturbekannten Verfahren können die folgende Verbindungen hergestellt werden:

| Bsp. | Struktur | Bsp. | Struktur |
|---|---|---|---|
| (1) | 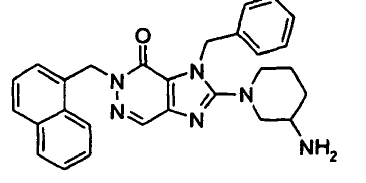 | (2) | 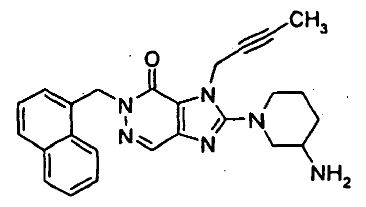 |
| (3) | 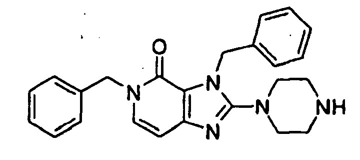 | (4) | 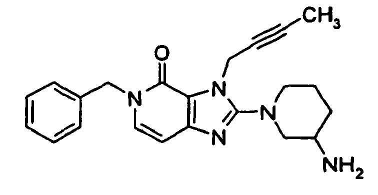 |
| (5) | 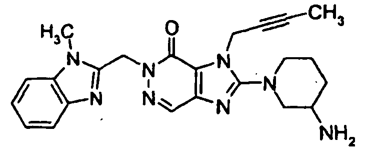 | (6) | 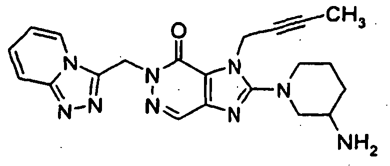 |
| (7) | 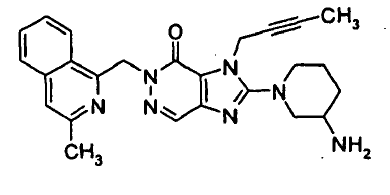 | (8) | 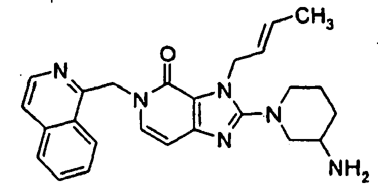 |
| (9) | 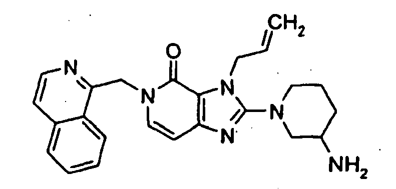 | (10) | 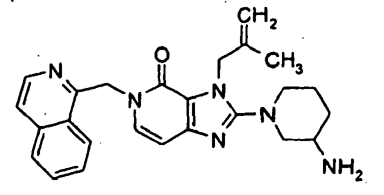 |
| (11) | 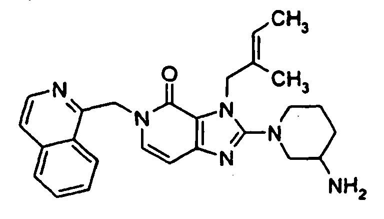 | (12) | 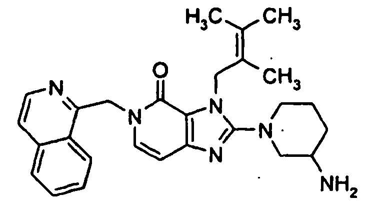 |
| (13) | 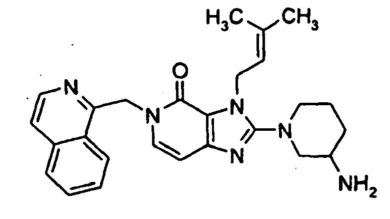 | (14) | 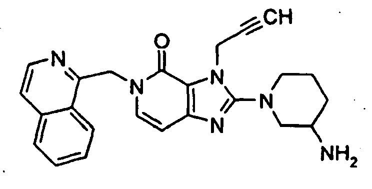 |
| (15) | 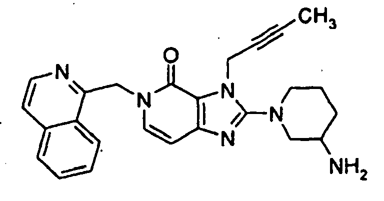 | (16) | 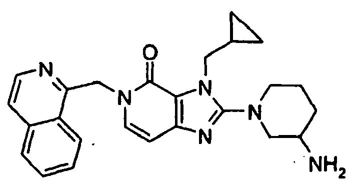 |
| (17) | 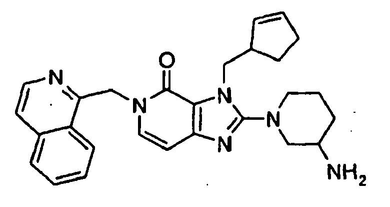 | (18) | 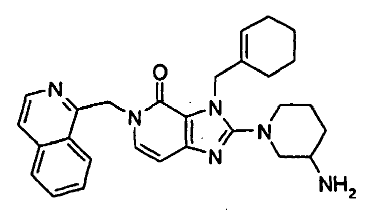 |
| (19) | 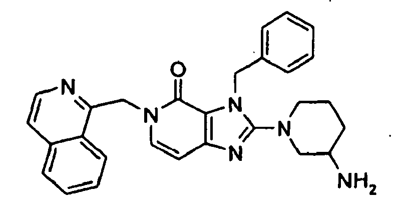 | (20) | 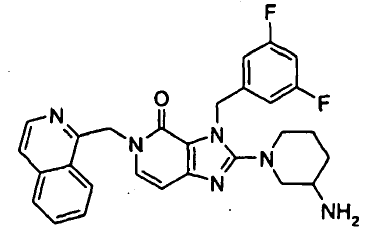 |
| (21) | 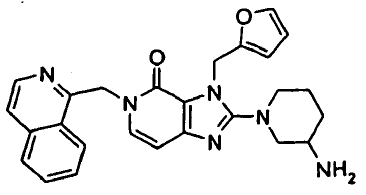 | (22) | 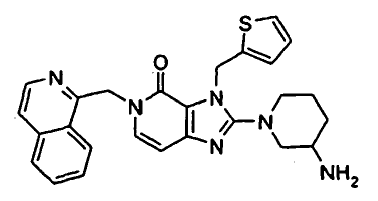 |
| (23) | 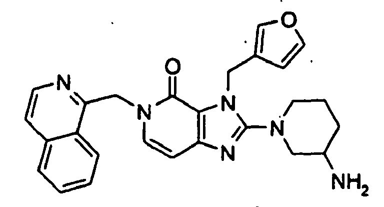 | (24) | 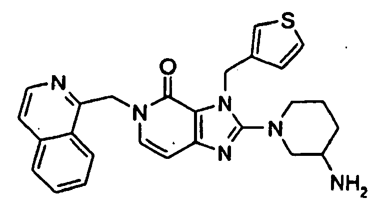 |
| (25) | 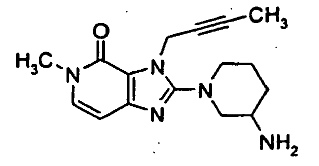 | (26) | 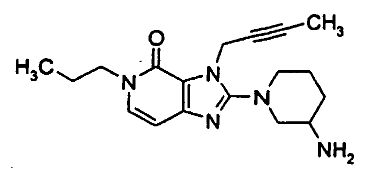 |
| (27) | 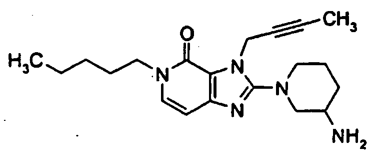 | (28) | 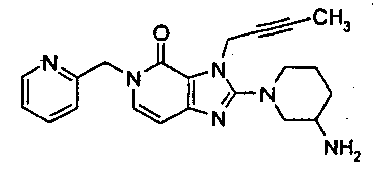 |
| (29) | 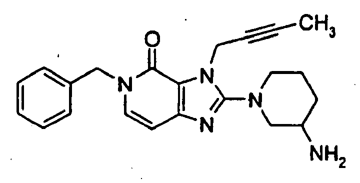 | (30) | 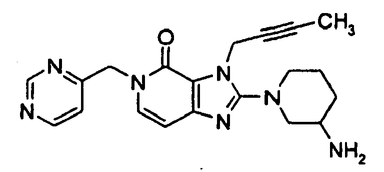 |
| (31) | 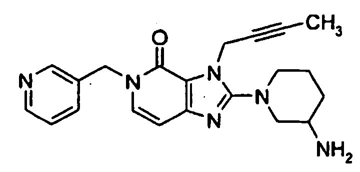 | (32) | 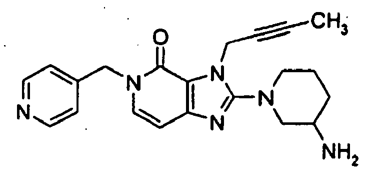 |
| (33) | 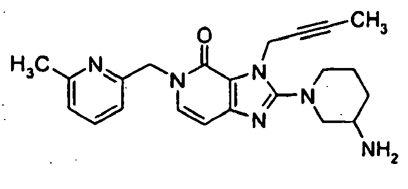 | (34) | 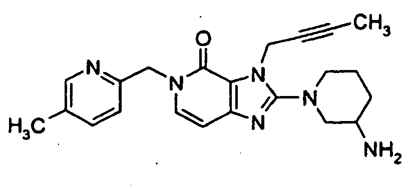 |
| (35) | 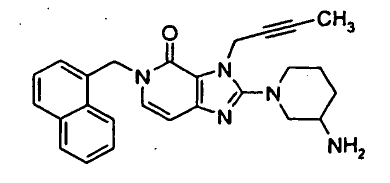 | (36) | 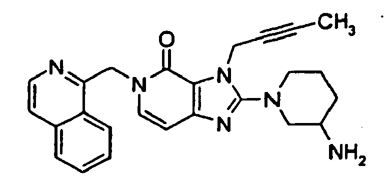 |
| (37) | 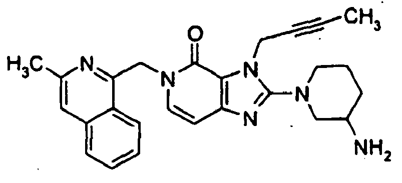 | (38) | 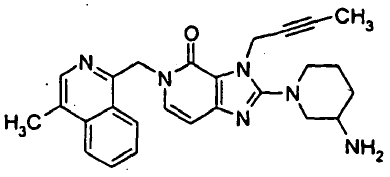 |
| (39) | 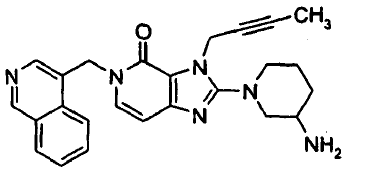 | (40) | 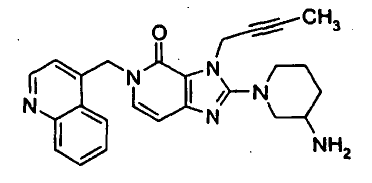 |
| (41) | 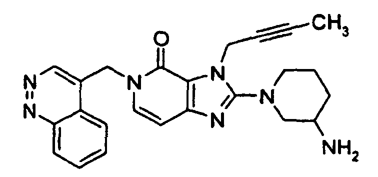 | (42) | 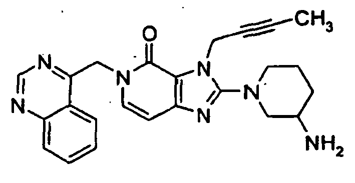 |
| (43) | 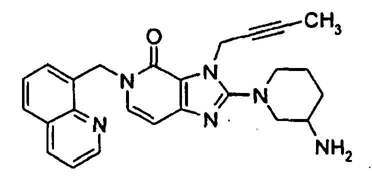 | (44) | 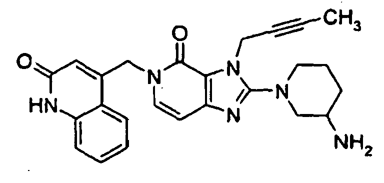 |
| (45) | 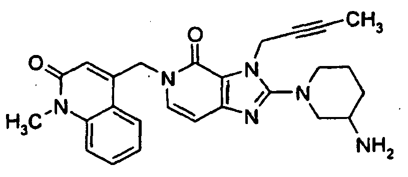 | (46) | 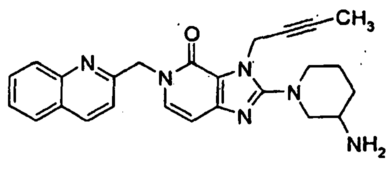 |
| (47) | 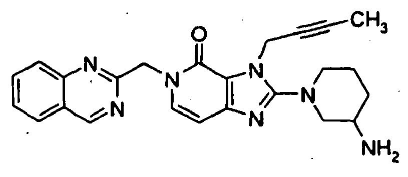 | (48) | 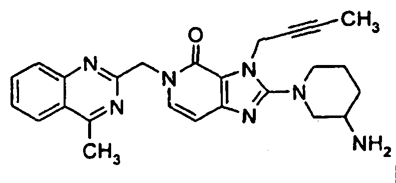 |
| (49) | 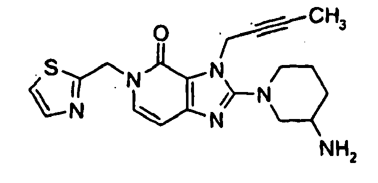 | (50) | 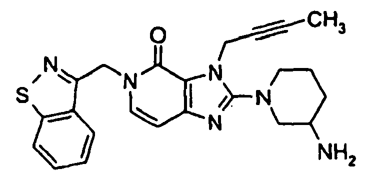 |
| (51) | 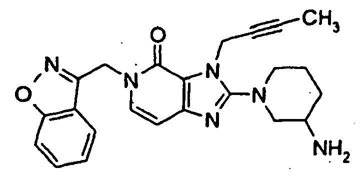 | (52) | 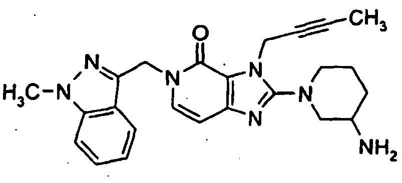 |
| (53) | 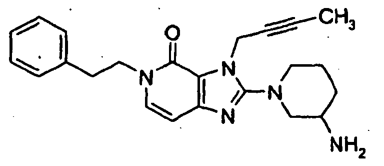 | (54) | 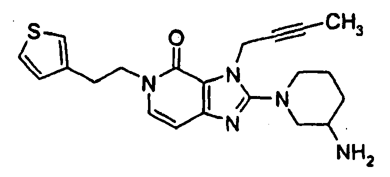 |
| (55) | 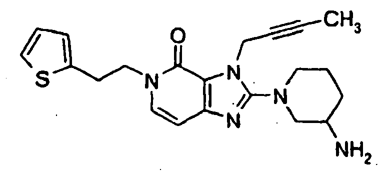 | (56) | 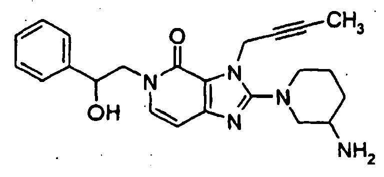 |
| (57) | 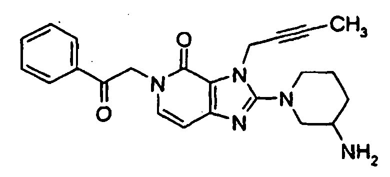 | (58) | 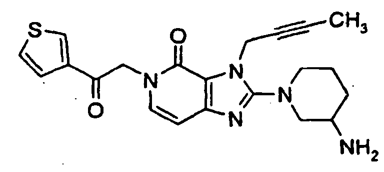 |
| (59) | 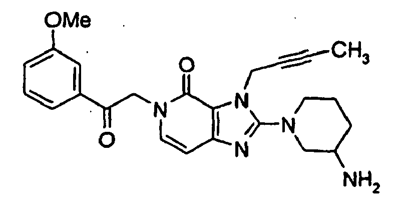 | (60) | 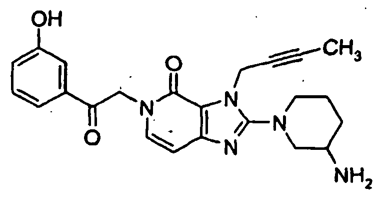 |
| (61) | 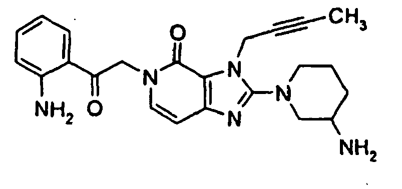 | (62) | 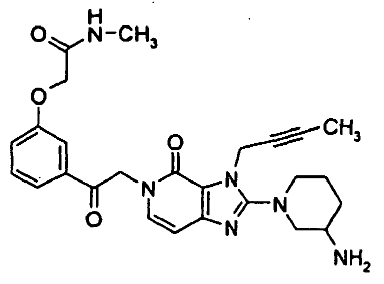 |
| (63) | 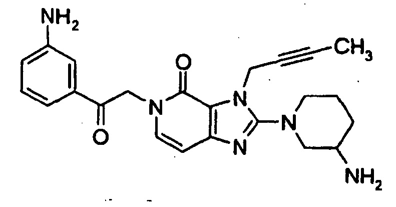 | (64) | 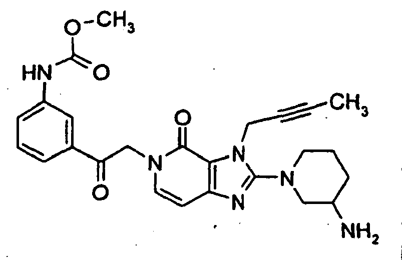 |
| (65) | 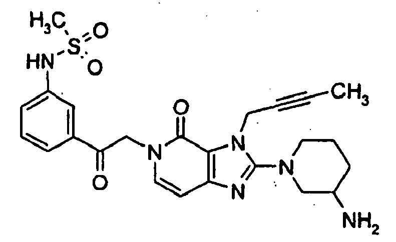 | (66) | 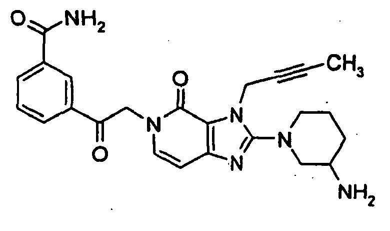 |
| (67) | 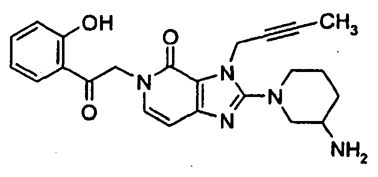 | (68) | 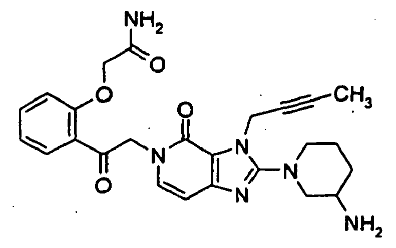 |
| (69) | 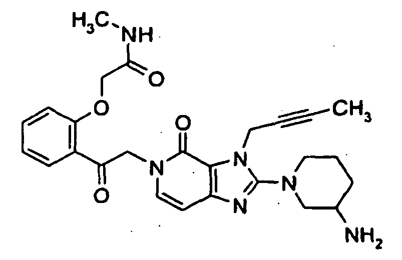 | (70) | 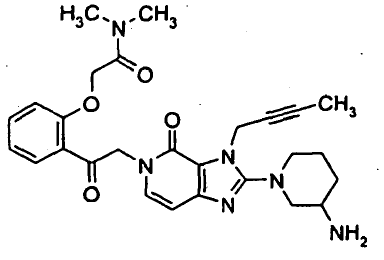 |
| (71) | 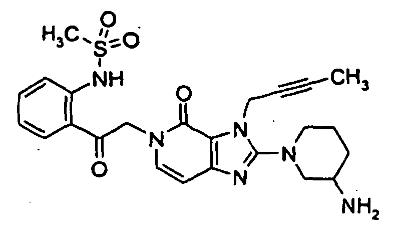 | (72) | 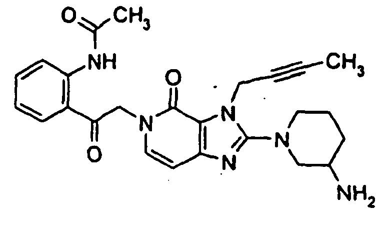 |
| (73) | 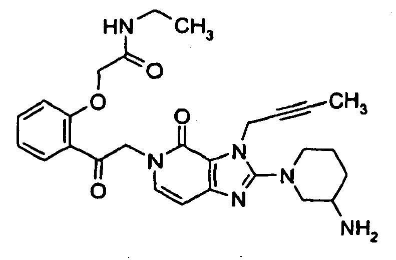 | (74) | 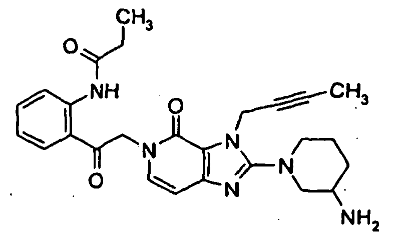 |
| (75) | 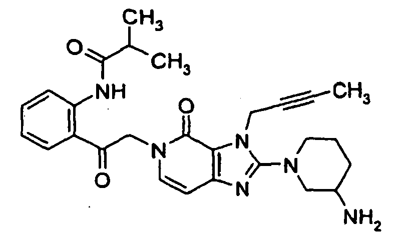 | (76) | 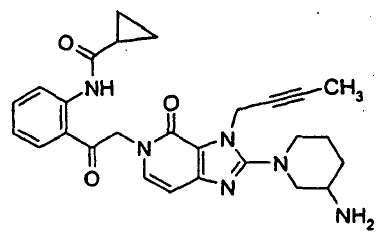 |
| (77) | 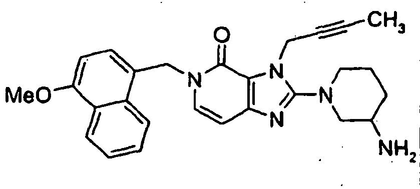 | (78) | 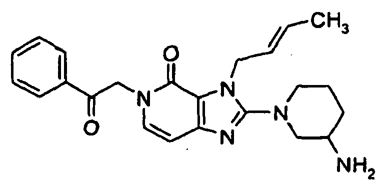 |
| (79) | 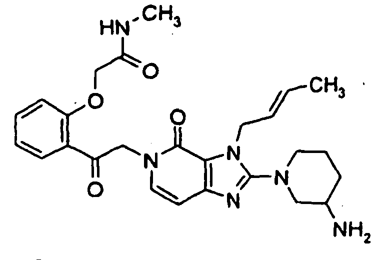 | (80) | 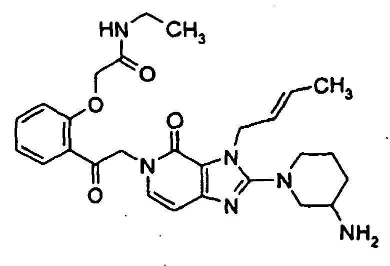 |
| (81) | 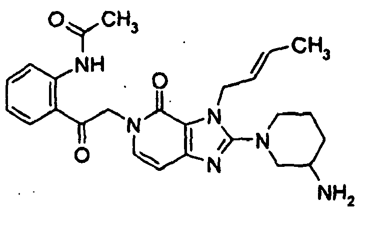 | (82) | 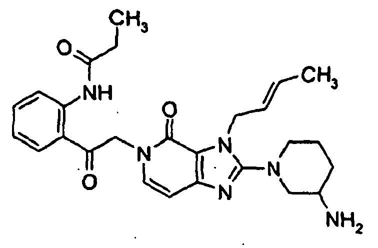 |
| (83) | 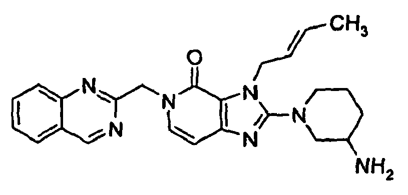 | (84) | 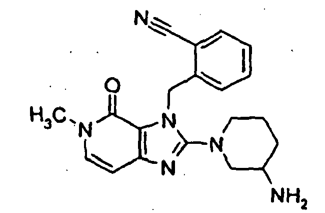 |
| (85) | 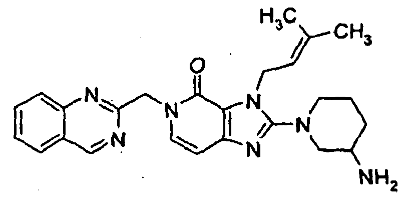 | (86) | 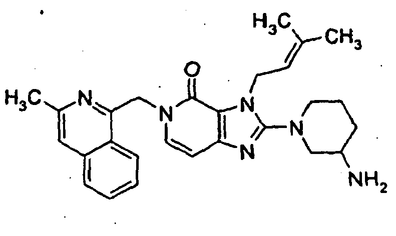 |
| (87) | 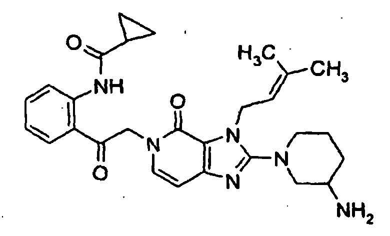 | (88) | 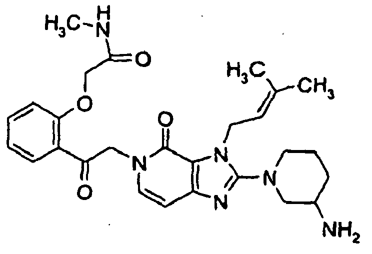 |
| (89) | 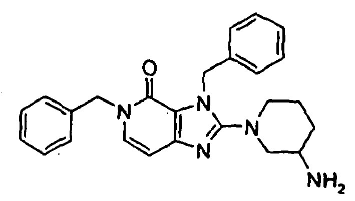 | (90) | 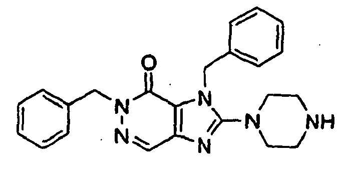 |
| (91) | 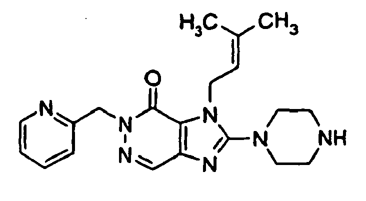 | (92) | 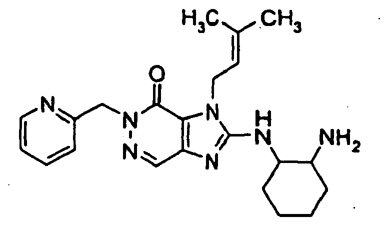 |
| (93) | 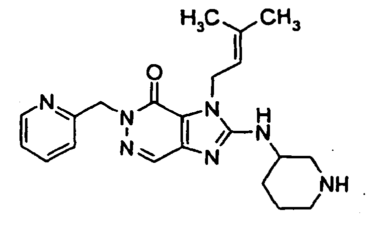 | (94) | 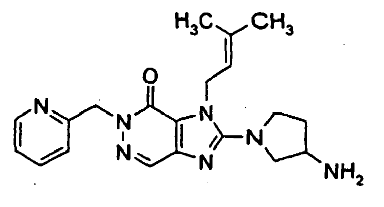 |
| (95) | 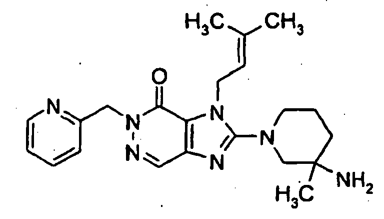 | (96) | 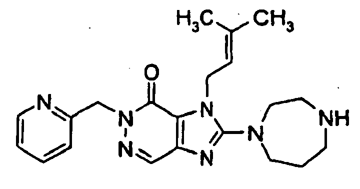 |
| (97) | 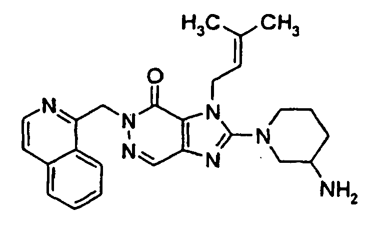 | (98) | 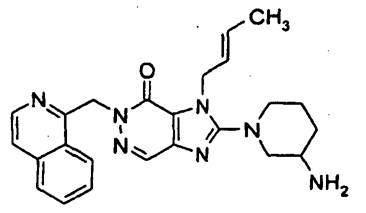 |
| (99) | 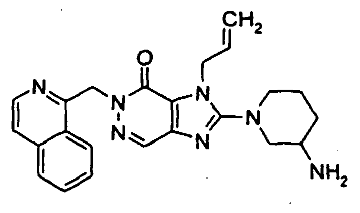 | (100) | 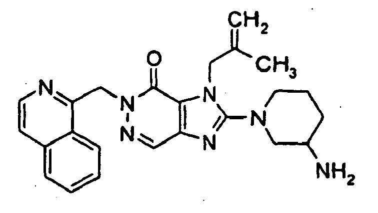 |
| (101) | 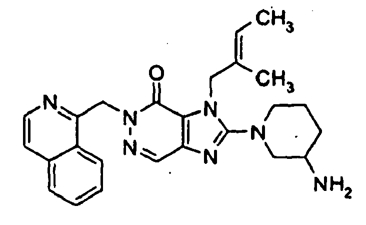 | (102) | 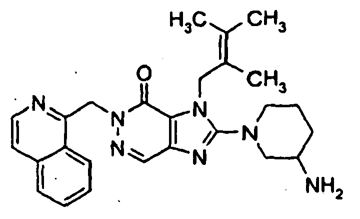 |
| (103) | 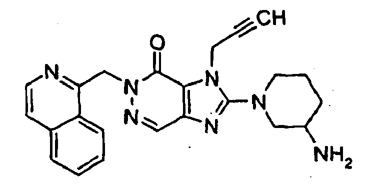 | (104) | 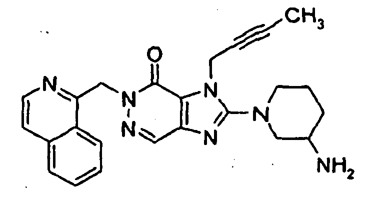 |
| (105) | 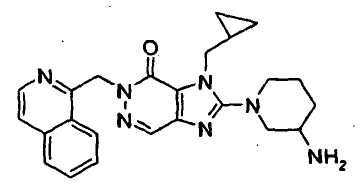 | (106) | 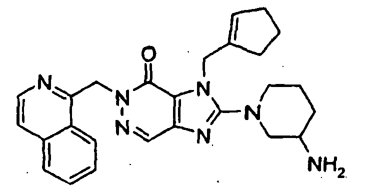 |
| (107) | 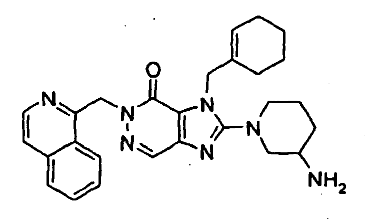 | (108) | 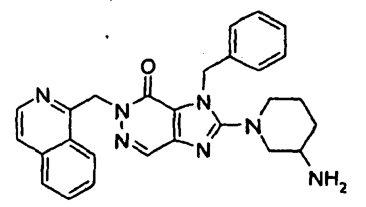 |
| (109) | 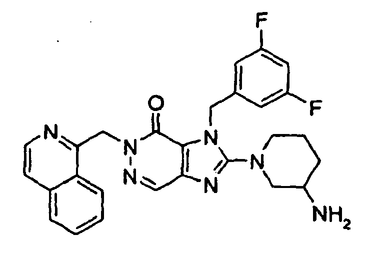 | (110) | 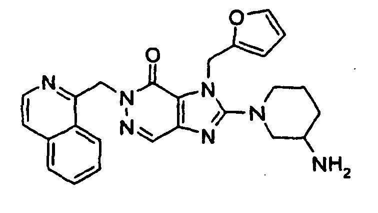 |
| (111) | 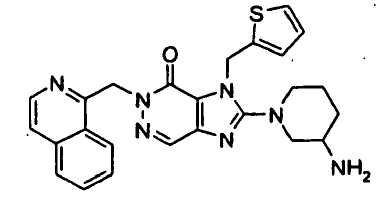 | (112) | 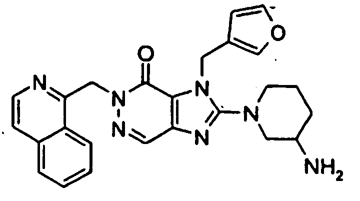 |
| (113) | 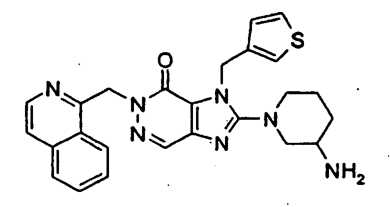 | (114) | 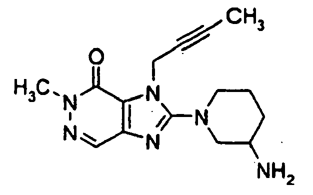 |
| (115) | 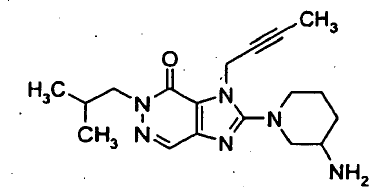 | (116) | 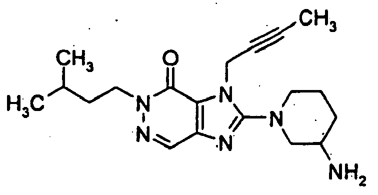 |
| (117) | 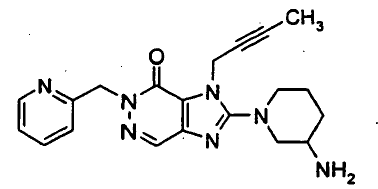 | (118) | 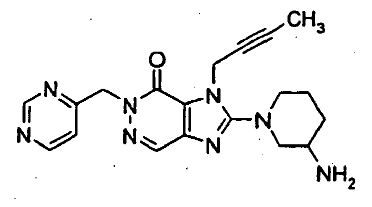 |
| (119) | 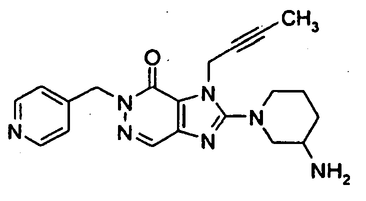 | (120) | 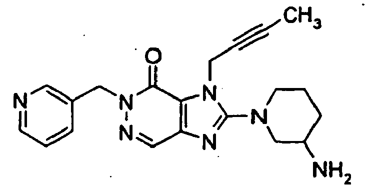 |
| (121) | 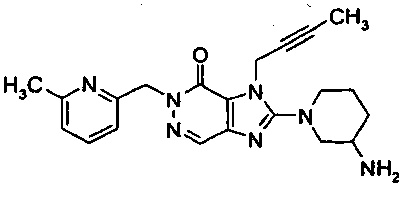 | (122) | 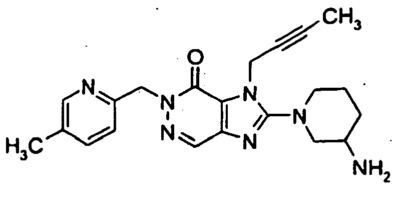 |
| (123) | 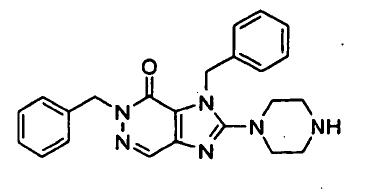 | (124) | 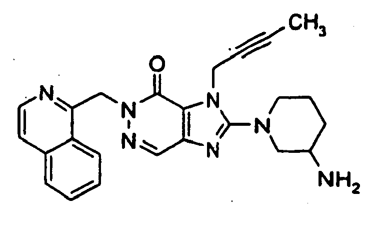 |
| (125) | 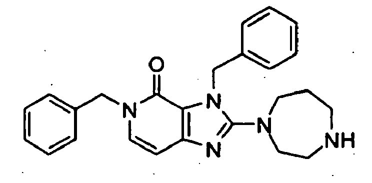 | (126) | 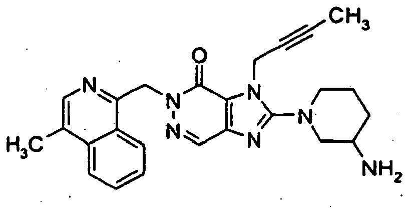 |
| (127) | 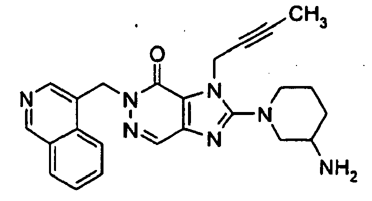 | (128) | 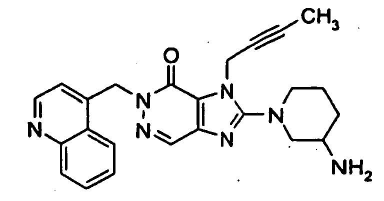 |
| (129) | 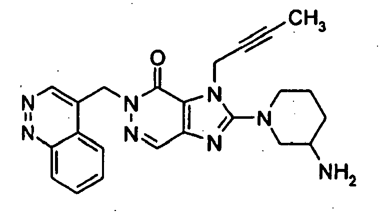 | (130) | 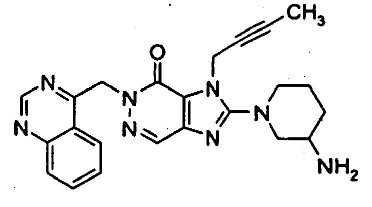 |
| (131) | 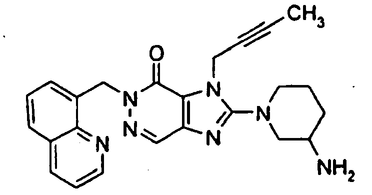 | (132) | 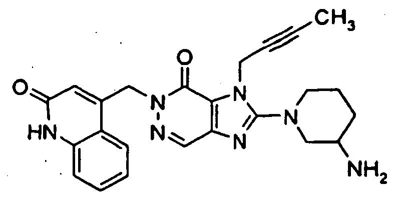 |
| (133) | 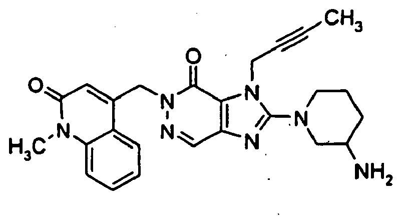 | (134) | 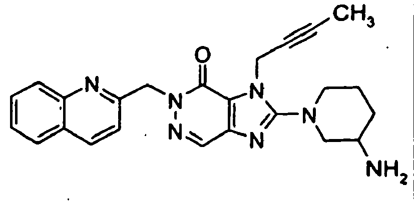 |
| (135) | 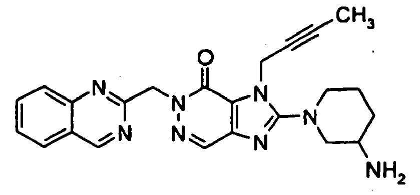 | (136) | 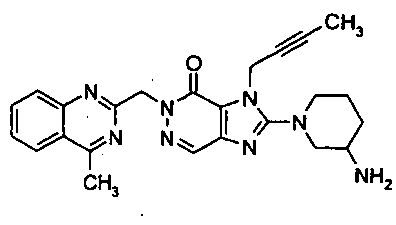 |
| (137) | 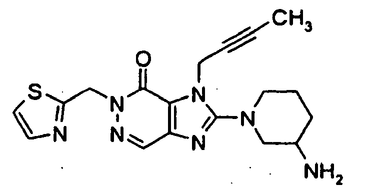 | (138) | 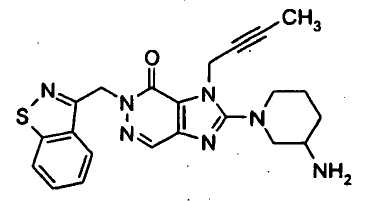 |
| (139) | 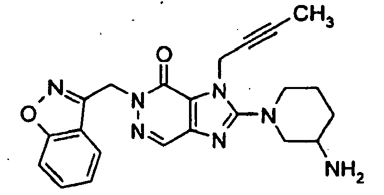 | (140) | 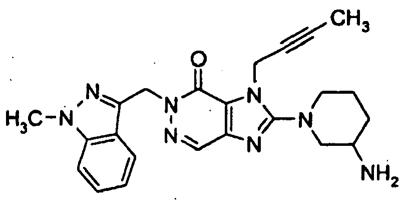 |
| (141) | 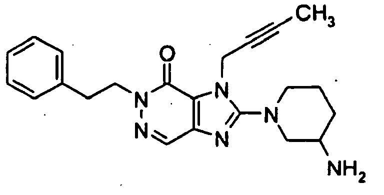 | (142) | 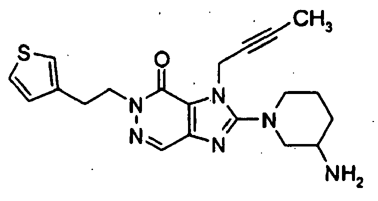 |
| (143) | 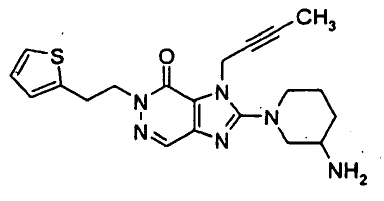 | (144) | 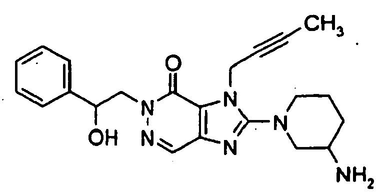 |
| (145) | 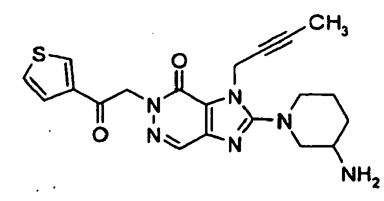 | (146) | 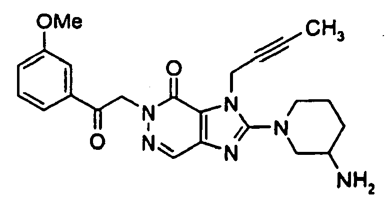 |
| (147) | 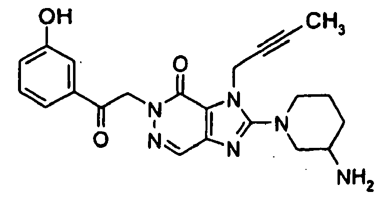 | (148) | 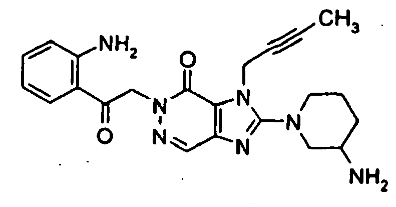 |
| (149) | 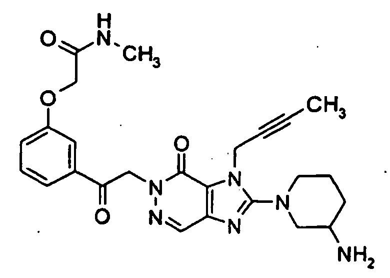 | (150) | 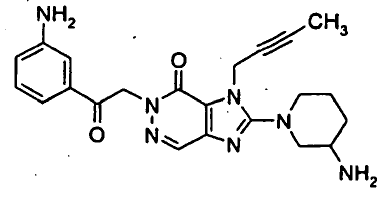 |
| (151) | 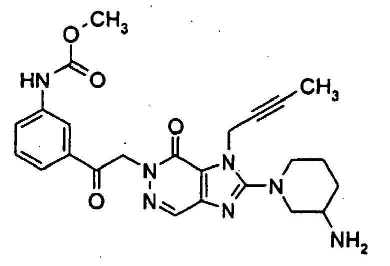 | (152) | 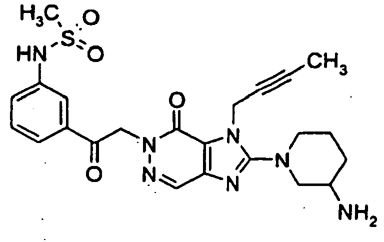 |
| (153) | 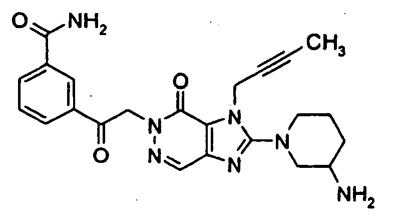 | (154) | 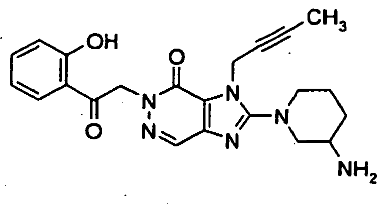 |
| (155) | 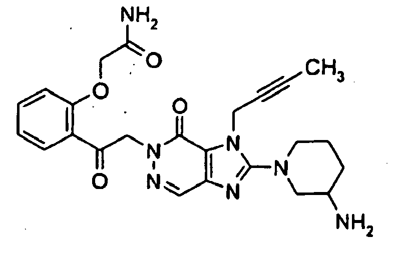 | (156) | 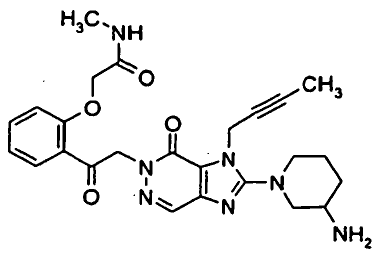 |
| (157) | 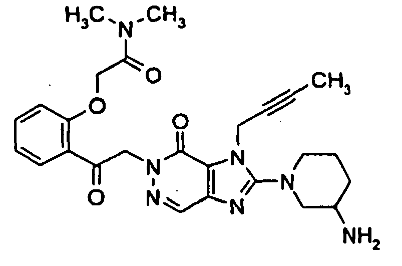 | (158) | 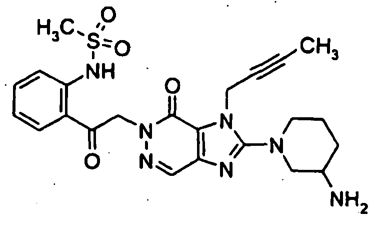 |
| (159) | 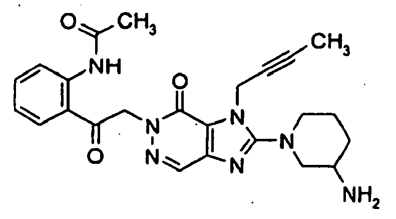 | (160) | 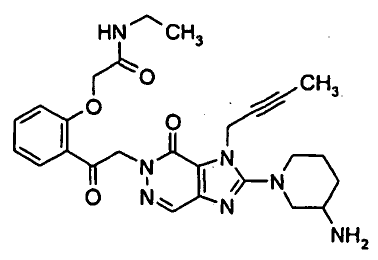 |
| (161) | 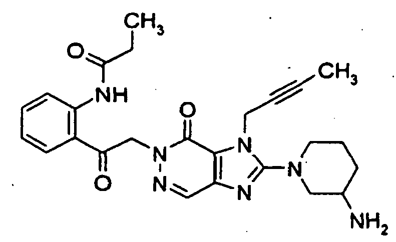 | (162) | 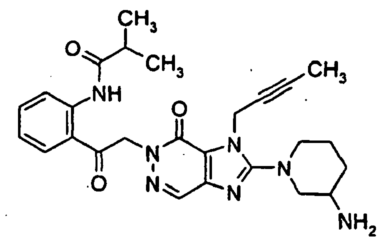 |
| (163) | 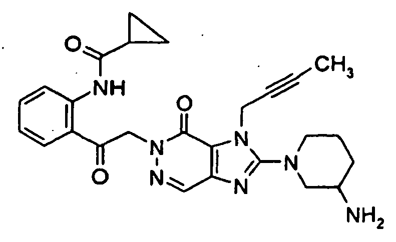 | (164) | 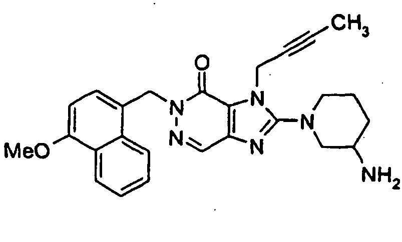 |
| (165) | 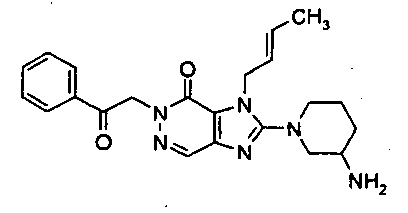 | (166) | 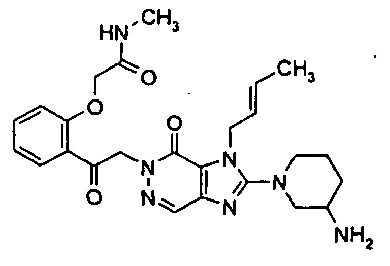 |
| (167) | 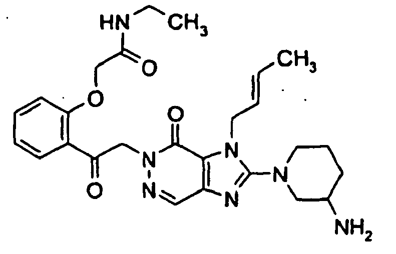 | (168) | 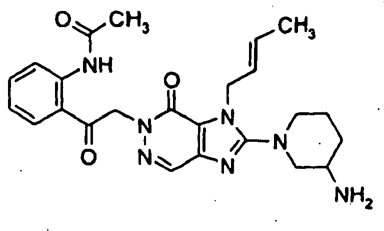 |
| (169) | 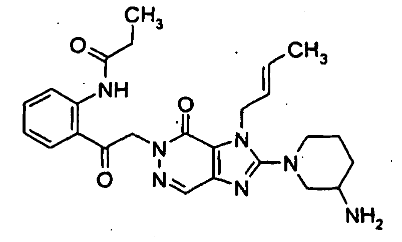 | (170) | 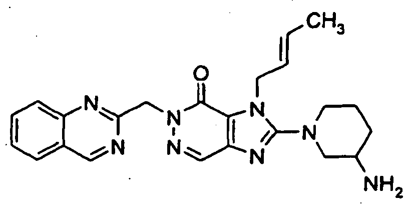 |
| (171) | 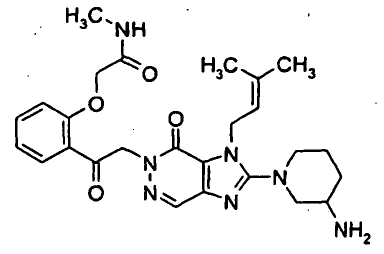 | (172) | 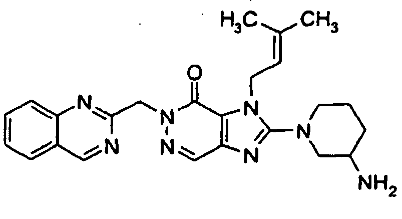 |
| (173) | 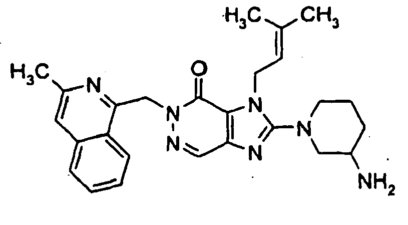 | (174) | 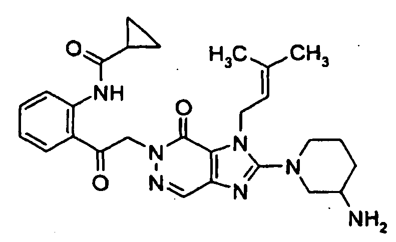 |
| (175) | 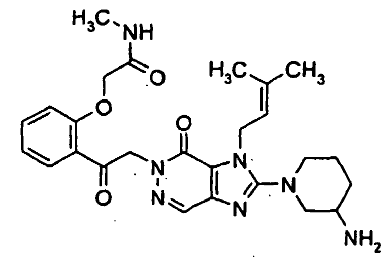 | (176) | 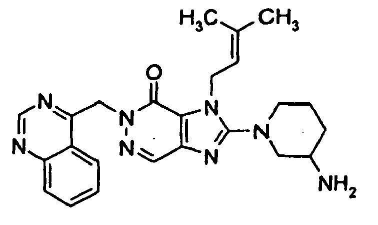 |
| (177) | 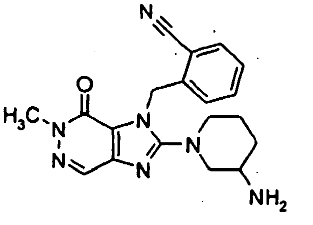 | (178) | 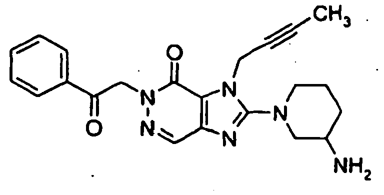 |
| (179) | 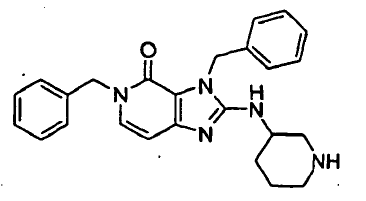 | (180) | 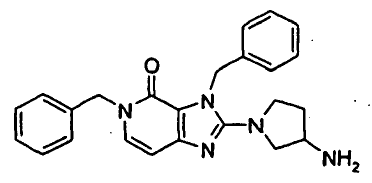 |
| (181) | 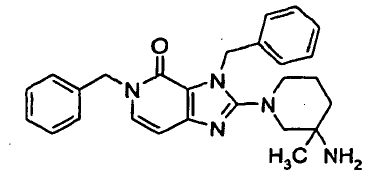 | | |

### Beispiel 182

### 2-(3-Amino-piperidin-1-yl)-3-(but-2-inyl)-5-(chinoxalin-6-ylmethyl)-3,5-dihydroimidazo[4,5-d]pyridazin-4-on

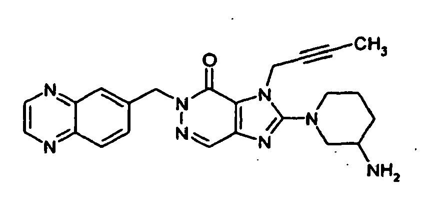

Hergestellt analog Beispiel 1j durch Abspaltung der Boc-Schutzgruppe mit Trifluoressigsäure in Dichlormethan.
Ausbeute: 69% der Theorie.
C₂₃H₂₄N₈O (428.50)
Massenspektrum: (M + H)⁺ = 429
¹H-NMR-Spektrum (d₆-DMSO): δ = 1.22 - 1.33 (m, 1H); 1.60 - 1.94 (m 5H); 2.75 - 3.08 (m, 3H); 3.53 - 3.70 (m, 2H); 5.10 (s, 2H); 5.60 (s, 2H); 7.79 (dd, 1H); 7.90 (s, 1H); 8.08 (d, 1H); 8.32 (s, 1H); 8.92 (s, 2H) ppm (austauschbare Protonen nicht sichtbar).

### Beispiel 183

### 2-(3-Amino-piperidin-1-yl)-3-(but-2-inyl)-5-(4-methyl-pyridin-2-ylmethyl)-3,5-dihydro-imidazo[4,5-d]pyridazin-4-on

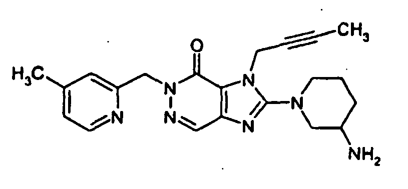

Hergestellt analog Beispiel 1j durch Abspaltung der Boc-Schutzgruppe mit Trifluoressigsäure in Dichlormethan.
Ausbeute: 90% der Theorie.
C₂₁H₂₅N₇O × 2 HCl (464.40)
Rf-Wert:: 0.43 (Kieselgel; Dichlormethan / Methanol 7 : 3)
Massenspektrum: (M + H)⁺ = 392
¹H-NMR-Spektrum (d₆-DMSO): δ = 1.65 - 1.80 (m, 2H); 1.80 (s, 3H); 1.90 - 2.10 (m, 2H); 3.15 - 3.43 (m, 3H); 3.45 - 3.55 (m, 1H); 3.72 - 3.82 (m, 1H); 5.18 (q, 2H); 5.70 (s, 2H); 7.58 (s, 1H); 7.79 (d, 1H); 8.39 (s, 1H); 8.60 (breites s; 2H); 8.73 - (d, 1H) ppm.

### Beispiel 184

### 2-((R)-3-Amino-piperidin-1-yl)-3-(but-2-inyl)-5-(4-phenyl-chinazolin-2-ylmethyl)-3,5-dihydro-imidazo[4,5-d]pyridazin-4-on

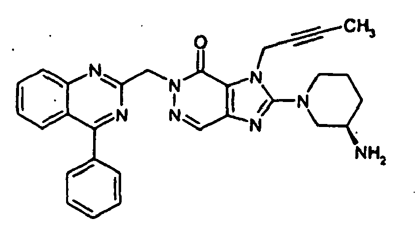

Hergestellt analog Beispiel 1j durch Abspaltung der Boc-Schutzgruppe mit Trifluoressigsäure in Dichlormethan.
Ausbeute: 63% der Theorie.
C₂₉H₂₈N₈O (504.60)
Massenspektrum: (M + H)⁺ = 505
¹H-NMR-Spektrum (d₆-DMSO): δ = 1.22 - 1.33 (m, 1H); 1.60 - 1.95 (m, 5H); 2.78 (dd, 1H); 2.85 - 3.06 (m, 2H); 3.56 - 3.72 (m, 2H); 5.11 (s, 2H); 5.72 (s, 2H); 7.56 - 7.75 (m, 6H); 7.90 - 8.03 (m, 2H); 8.08 (d, 1H); 8.30 (s, 1H) ppm.

### Beispiel 185

### 2-((R)-3-Amino-piperidin-1-yl)-3-(but-2-inyl)-5-(chinoxalin-2-ylmethyl)-3,5-dihydro imidazo[4,5-d]pyridazin-4-on

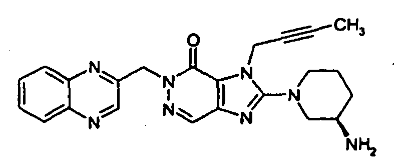

Hergestellt analog Beispiel 1j durch Abspaltung der Boc-Schutzgruppe mit Trifluoressigsäure in Dichlormethan.
Ausbeute: 88% der Theorie.
C₂₃H₂₄N₈O x HCl (464.96)
Massenspektrum: (M + H)⁺ = 429
¹H-NMR-Spektrum (d₆-DMSO): δ = 1.63 - 1.75 (m, 2H); 1.77 (s, 3H); 1.88 - 2.10 (m, 2H); 3.13 - 3.26 (m, 2H); 3.35 - 3.56 (m, 2H); 3.75 (dd, 1H); 5.15 (q, 2H); 5.71 (s, 2H); 7.80 - 7.86 (m, 2H); 7.99 (m, 1H); 8.09 .(m, 1H); 8.33 (s, 1H); 8.45 (breites s, 2H); 8.92 (s, 1H) ppm.

### Beispiel 186

### 2-((R)-3-Amino-piperidin-1-yl)-3-(but-2-inyl)-5-(2,3-dimethyl-chinoxalin-6-ylmethyl)-3,5-dihydro-imidazo[4,5-d]pyridazin-4-on

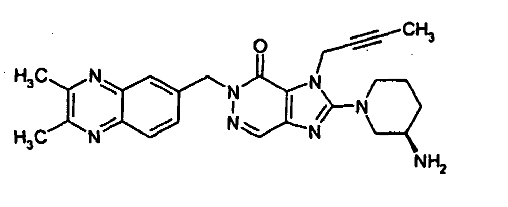

Hergestellt analog Beispiel 1j durch Abspaltung der Boc-Schutzgruppe mit Trifluoressigsäure in Dichlormethan.
Ausbeute: 52% der Theorie.
C₂₅H₂₈N₈O (456.56)
Massenspektrum: (M + H)⁺ = 457
¹H-NMR-Spektrum (d₆-DMSO): δ = 1.18 - 1.31(m, 1H); 1.58 -1.72 (m, 1H); 1.73 - 1.93 (m, 5H); 2.66 (s, 3H); 2.68 (s, 3H); 2.75 (dd, 1H); 2.83 - 3.05 (m, 1H); 3.55 - 3.68 (m, 2H); 5.10 (s, 2H); 5.53 (s, 2H); 7.62 (dd, 1H); 7.70 (s, 1 H); 7.92 (d, 1H); 8.31 (s, 1 H) ppm.

### Beispiel 187

### 2-((R)-3-Amino-piperidin-1-yl)-3-(but-2-inyl)-5-(4-cyano-naphthalin-1-ylmethyl)-3,5-dihydro-imidazo[4,5-d]pyridazin-4-on

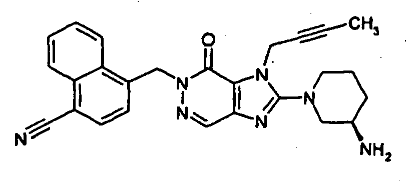

Hergestellt analog Beispiel 1j durch Abspaltung der Boc-Schutzgruppe mit Trifluoressigsäure in Dichlormethan.
Ausbeute: 62% der Theorie.
C₂₆H₂₅N₇O (451.53)
Massenspektrum: (M + H)⁺ = 452
¹H-NMR-Spektrum (d₆-DMSO): δ = 1.22 -1.32 (m, 1H); 1.58 -1.71 (m, 1H); 1.75 -1.93 (m, 5H); 2.77 (dd, 1 H); 2.83 - 3.05 (m, 2H); 3.55 - 3.70 (m, 2H); 5.60 (s, 2H), 5.88 (s, 2H); 7.28 (d, 1 H); 7.76 - 7.88 (m, 2H); 8.10 (d, 1H); 8.17 (d. 1 H); 8.30 (s, 1H); 8.47 (d, 1H) ppm.

### Beispiel 188

### 2-(3-Amino-Piperidin-1-yl)-3-(3-methyl-but-2-enyl)-5-(2-oxo-2-phenyl-ethyl)-3,5-dihydro-imidazo[4,5-c]pyridin-4-on

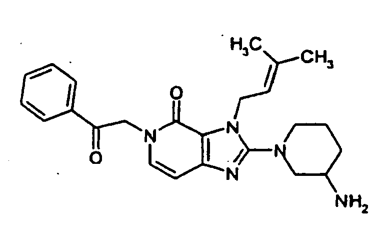

Hergestellt analog Beispiel 1j durch Abspaltung der Boc-Schutzgruppe mit Trifluoressigsäure in Dichlormethan.
Ausbeute: 69% der Theorie.
C₂₄H₂₉NO₂ (419.53)
Massenspektrum: (M + H)⁺ = 420
¹H-NMR-Spektrum (d₆-DMSO): δ = 1.15 - 1.27 (m, 1H); 1.57 - 1.92 (m, 9H); 2.55 - 2.70 (m, 2H); 2.76 - 2.92 (m, 2H); 3.41 (dt, 1 H); 4.80 (d, 2H); 5.32 (t, 1 H); 5.04 (s, 2H); 6.51 (d, 1H); 7.34 (d, 1H); 7.60 (t, 2H); 7.70 (t, 1H); 8.08 (d, 2H) ppm.

### Beispiel 189

### 2-(3-Amino-piperidin-1-yl)-3-(3-methyl-but-2-enyl)-5-(2-oxo-2-phenyl-ethyl)-3,5-dihydro-imidazo[4,5-d]pyridazin-4-on

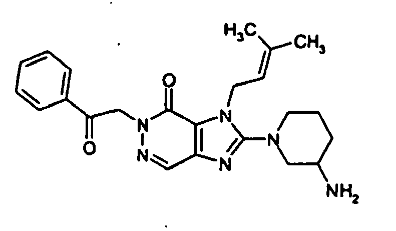

Hergestellt analog Beispiel 1j durch Abspaltung der Boc-Schutzgruppe mit Trifluoressigsäure in Dichlormethan.
Ausbeute: 88% der Theorie.
C₂₂H₂₄N₆O₂ (404.47)
Massenspektrum: (M + H)⁺ = 405
¹H-NMR-Spektrum (d₆-DMSO): δ = 1.55 (m, 1H); 1.70 (m, 1H); 1.88 (m, 1H); 1.99 (m, 1 H); 3.05 - 3.22 (m, 3H); 3.57 (m, 1 H); 3.71 (dt, 1 H); 5.11 (s, 2H); 5.70 (s, 2H); 7.60 (t, 2H); 7.72 (t, 1H); 8.08 (d, 2H); 8.32 (s, 1H) ppm.

### Beispiel 190

### 2-(3-Amino-piperidin-1-yl)-3-(3-methyl-but-2-enyl)-5-(naphthalin-1-ylmethyl)-3,5-dihydro-imidazo[4,5-c]pyridin-4-on

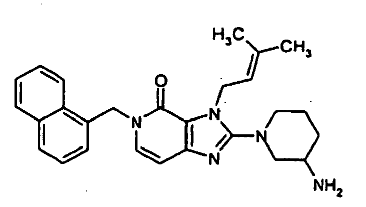

Hergestellt analog Beispiel 1j durch Abspaltung der Boc-Schutzgruppe mit Trifluoressigsäure in Dichlormethan.
Ausbeute: 86% der Theorie.
C₂₇H₃₁N₅O (441.58)
Massenspektrum: (M + H)⁺ = 442
¹H-NMR-Spektrum (d₆-DMSO): δ = 1.13 -1.75 (m,1H); 1.57 -1.95 (m, 9H); 2.57 (dd, 1 H); 2.83 (m, 2H); 3.30 - 3.47 (m, 2H); 4.87 (d, 2H); 5.39 (t, 1 H); 5.68 (s, 2H); 6.50 (d, 1 H); 7.09 (d, 1H); 7.32 (d,1 H); 7.42 (t, 1H); 7.58 (m, 2H); 7.89 (d, 1H); 7.98 (d, 1H); 8.21 (d, 1H) ppm.

### Beispiel 191

### 2-((R)-3-Amino-piperidin-1-yl)-3-(but-2-inyl)-5-(4-fluornaphthalin-1-ylmethyl)-3,5-dihydro-imidazo[4,5-d]pyridazin-4-on - Hydrochlorid

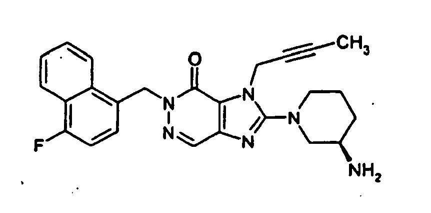

Hergestellt analog Beispiel 1j durch Abspaltung der Boc-Schutzgruppe mit Trifluoressigsäure in Dichlormethan.
Ausbeute: 88% der Theorie.
C₂₅H₂₅FN₆O (444.52)
Massenspektrum: (M + H)⁺ = 445
¹H-NMR-Spektrum (d₆-DMSO): δ = 1.70 (m, 2H); 1.81 (s, 3H); 1.94 (m, 1H); 2.03 (m, 1 H); 3.19 (m, 2H); 3.40 (m, 1H); 3.50 M, 1H); 3.74 (m, 1H); 5.19 (m, 2H); 5.77 (s, 2H); 7.28 (d, 2H); 7.76 (m, 2H); 8.10 (d, 1 H); 8.31 (s, 1 H); 8.38 (d, 1H); 8.47 (breites s, 3H) ppm.

### Beispiel 192

### 2-((R)-3-Amino-piperidin-1-yl)-3-(but-2-inyl)-5-(6-methylbenzoxazol-2-ylmethyl)-3,5-dihydro-imidazo[4,5-d]pyridazin-4-on- Hydrochlorid

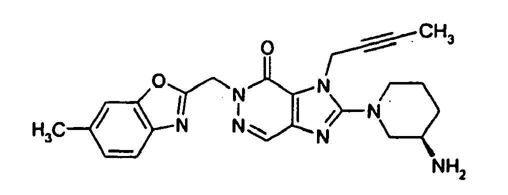

Hergestellt analog Beispiel 1j durch Abspaltung der Boc-Schutzgruppe mit Trifluoressigsäure in Dichlormethan.
Ausbeute: 81% der Theorie.
C₂₃H₂₅N₇O₂ (431.50)
Massenspektrum: (M + H)⁺ = 432
¹H-NMR-Spektrum (d₆-DMSO): δ = 1.70 (m, 2H); 1.80 (s, 3H); 1.94 (m, 1H); 2.04 (m, 1H); 2.43 (s, 3H); 3.21 (m, 2H); 3.40 (m, 1 H); 3.51 (m, 1 H); 3.75 (m, 1 H); 5.15 (dd, 2H); 5.63 (s, 2H); 7.17 (d, 1H); 7.50 (s, 1 H); 7.56 (d, 1H); 8.35 (s, 1 H); 8.46 (breites s, 3H) ppm.

### Beispiel 193

### 2-((R)-3-Amino-pioeridin-1-yl)-3-(but-2-inyl)-5-(1-phenylbenzimidazol-2-ylmethyl)-3,5-dihydro-imidazo[4,5-d]pyridazin-4-on-Hydrochlorid

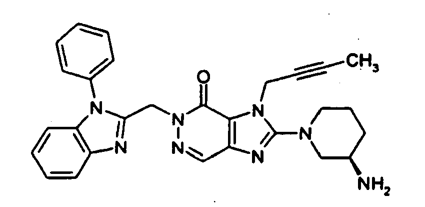

Hergestellt analog Beispiel 1j durch Abspaltung der Boc-Schutzgruppe mit Trifluoressigsäure in Dichlormethan.
Ausbeute: 62% der Theorie.
C₂₈H₂₈N₈O (492.59)
Massenspektrum: (M + H)⁺ = 493
¹H-NMR-Spektrum (d₆-DMSO): δ = 1.72 (m, 2H); 1.80 (s, 3H); 1.95 (m, 1 H); 2.03 (m, 1H); 3.21 (m, 2H); 3.40 (m, 1 H); 3.49 (m, 1H); 5.08 (dd, 2H); 5.75 (s, 2H); 7.31 (d, 1H); 7.45 - 7.66 (m, 7H), 7.86 (d, 1 H); 822 (s, 1 H); 8.54 (breites s, 3H) ppm.

### Beispiel 194

### 2-((R)-3-Amino-piperidin-1-yl)-3-(but-2-inyl)-5-(4-methylbenzoxazol-2-ylmethyl)-3,5-dihydro-imidazo[4,5-d]pyridazin-4-on-Hydrochlorid

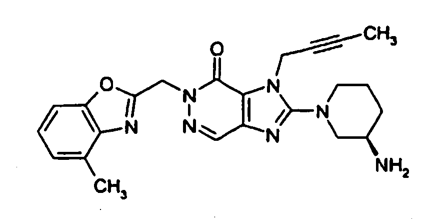

Hergestellt analog Beispiel 1j durch Abspaltung der Boc-Schutzgruppe mit Trifluoressigsäure in Dichlormethan.
Ausbeute: 83% der Theorie.
C₂₃H₂₅N₇O₂ (431.50)
Massenspektrum: (M + H)⁺ = 432
¹H-NMR-Spektrum (d₆-DMSO): δ = 1.71 (m, 2H); 1.80 (s, 3H); 1.93 (m, 1H); 2.05 (m, 1H); 3.22 (m, 2H); 3.41 (m, 1 H); 3.53 (m, 1H); 3.75 (m, 1H); 5.16 (dd, 2H); 5.66 (s, 2H); 7.20 (d, 1H); 7.27 (t, 1H); 7.48 (d, 1H); 8.35 (s, 1H); 8.44 (breites s, 3H) ppm.

### Beispiel 195

### 2-((R)-3-Amino-piperidin-1-yl)-3-(but-2-inyl)-5-(5-trifluormethylbenzthiazol-2-ylmethyl)-3,5-dihydro-imidazo[4,5-d]pyridazin-4-on - Hydrochlorid

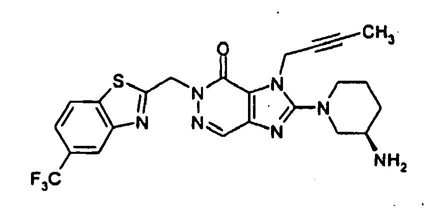

Hergestellt analog Beispiel 1j durch Abspaltung der Boc-Schutzgruppe mit Trifluoressigsäure in Dichlormethan.
Ausbeute: 98% der Theorie.
C₂₃H₂₂ F₃N₇OS (501.54)
Massenspektrum: (M + H)⁺ = 502
¹H-NMR-Spektrum (d₆-DMSO): δ = 1.70 (m, 2H); 1.80 (s, 3H); 1.95 (m, 1H); 2.05 (m, 1 H); 3.22 (m, 2H); 3.40 (m, 1H); 3.52 (m, 1H); 3.77 (m, 1H); 5.19 (dd, 2H); 5.82 (s, 2H); 7.77 (d, 1H): 8.31 (d, 1H); 8.35 (s. 1 H); 8.41 (s, 1H); 8.50 (breites s, 3H) ppm.

### Beispiel 196

### 2-((R)-3-Amino-piperidin-1-yl)-3-(but-2-inyl)-5-(5-chlorbenzoxazol-2-ylmethyl)-3,5-dihydro-imidazo[4,5-d]pyridazin-4-on - Hydrochlorid

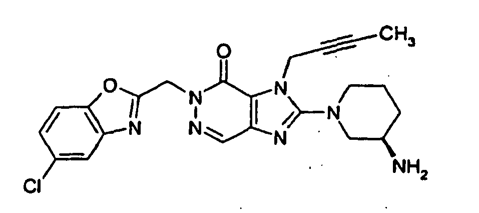

Hergestellt analog Beispiel 1j durch Abspaltung der Boc-Schutzgruppe mit Trifluoressigsäure in Dichlormethan,
Ausbeute: 77% der Theorie.
C₂₂H₂₂ClN₇O₂ (451.92)
Massenspektrum: (M + H)⁺ = 452
¹H-NMR-Spektrum (d₆-DMSO): δ = 1.71 (m, 2H); 1.80 (s, 3H); 1.94 (m, 1H) 2.05 (m, 1H); 3.22 (m, 2H); 3.41 (m, 1 H); 3.53 (m, 1 H); 3.75 (m, 1 H); 5.16 (dd, 2H); 5.69 (s, 2H); 7.43 (d, 1 H); 7.75 (d, 1H); 7.83 (s, 1 H); 8.39 (s, 1 H); 8.97 (breites s, 3H) ppm.

### Beispiel 197

### 2-((R)-3-Amino-piperidin-1-yl)-3-(but-2-inyl)-5-(5-methylbenzoxazol-2-ylmethyl)-3,5-dihydro-imidazo[4,5-d]pyridazin-4-on- Hydrochlorid

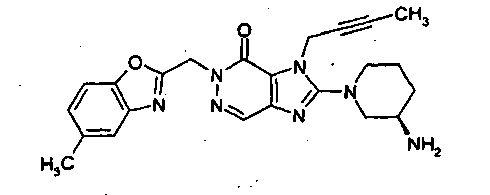

Hergestellt analog Beispiel 1j durch Abspaltung der Boc-Schutzgruppe mit Trifluoressigsäure in Dichlormethan.
Ausbeute: 57% der Theorie.
C₂₃H₂₅N₇O₂ (431.50)
Massenspektrum: (M + H)⁺ = 432
¹H-NMR-Spektrum (d₆-DMSO): δ = 1.71 (m, 2H); 1.80 (s, 3H); 1.93 (m, 1H); 2.04 (m, 1 H); 2.41 (s, 3H); 3.21 (m, 2H); 3.40 (m, 1H); 3.53 (m, 1 H); 3.75 (m, 1 H); 5.16 (dd, 2H); 5.64 (s, 2H); 7.20 (d, 1H); 7.49 (s, 1H); 7.57 (d, 1 H); 8.38 (s, 1 H); 8.92 (breites s, 3H) ppm.

### Beispiel 198

### 2-((R)-3-Amino-piperidin-1-yl)-3-(but-2-inyl)-5-[1-(pyridin-3-yl)-benzimidazol-2-ylmethyl]-3,5-dihydro-imidazo[4,5-d]pyridazin-4-on-Hydrochlorid

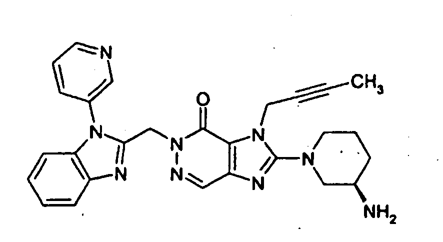

Hergestellt analog Beispiel 1j durch Abspaltung der Boc-Schutzgruppe mit Trifluoressigsäure in Dichlormethan.
Ausbeute: 37% der Theorie.
C₂₇H₂₇N₉O (493.58)
Massenspektrum: (M + H)⁺ = 494
¹H-NMR-Spektrum (d₆-DMSO): δ = 1.71 (m, 2H); 1.81 (s, 3H); 1.95 (m, 1H); 2.02 (m, 1H); 3.22 (m, 2H), 3.40 (m, 1H); 3.48 (m, 1H); 3.73 (m, 1H); 5.08 (dd, 2H); 5.67 (s, 2H), 7.28 (d, 1H); 7.41 (m, 2H); 7.70 (dd, 1H); 7.28 (d, 1H); 8.17 (d, 1H); 8.22 (s, 1H); 8.43 (breites s, 3H); 8.78 (d, 1H); 8.88 (s, 1H) ppm.

### Beispiel 199

### 2-((R)-3-Amino-piperidin-1-yl)-3-(but-2-inyl)-5-(5,7-dimethylbenzoxazol-2-ylmethyl)-3,5-dihydro-imidazo[4,5-d]pyridazin-4-on-Hydrochlorid

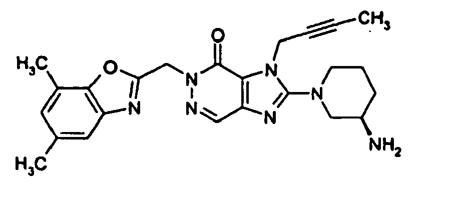

Hergestellt analog Beispiel 1j durch Abspaltung der Boc-Schutzgruppe mit Trifluoressigsäure in Dichlormethan.
Ausbeute: 86% der Theorie.
C₂₄H₂₇N₇O₂ (445.53)
Massenspektrum: (M+H)⁺ = 446
¹H-NMR-Spektrum (d₆-DMSO): δ = 1.71 (m, 2H); 1.80 (s, 3H); 1.94 (m, 1H); 2.05 (m, 1H); 2.36 (s, 3H); 2.42 (s, 3H); 3.22 (m, 2H); 3.41 (m, 1H); 3.54 (m, 1H); 3.75 (m, 1H); 5.16 (dd, 2H); 5.63 (s, 2H); 7.02 (s, 1H); 7.28 (s, 1H); 8.34 (s, 1H); 8.41 (breites s, 3H) ppm.

### Beispiel 200

### 2-((R)-3-Amino-piperidin-1-yl)-3-(but-2-inyl)-5-(4-chlomaphth-1-ylmethyl)-3,5-dihydro-imidazo[4,5-d]pyridazin-4-on

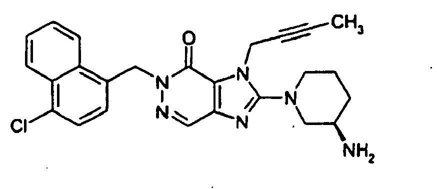

Hergestellt analog Beispiel 1j durch Abspaltung der Boc-Schutzgruppe mit Trifluoressigsäure in Dichlormethan.
Ausbeute: 54% der Theorie.
C₂₅H₂₅ClN₆O (460.97)
Massenspektrum: (M + H)⁺ = 461/3
¹H-NMR-Spektrum (d₆-DMSO): δ = 1.22 (m, 1H); 1.62 (m, 1H); 1.73 (m, 1H); 1.80 (s, 3H); 1.87 (m, 1H); 2.73 (m, 1H); 2.99 (m, 1H); 3.60 (m, 2H); 5.12 (s, 2H); 5.80 (s, 2H); 7.22 (d, 1H); 7.65 (d, 1H) 7.72 (m, 2H); 8.23 (d, 1H); 8.30 (s, 1H); 8.39 (d, 1H) ppm.

### Beispiel 201

### 2-((R)-3-Amino-piperidin-1-yl)-3-(but-2-inyl)-5-(4-bromnaphth-1-ylmethyl)-3,5 dihydro-imidazo[4,5-d]pyridazin-4-on - Hydrochlorid

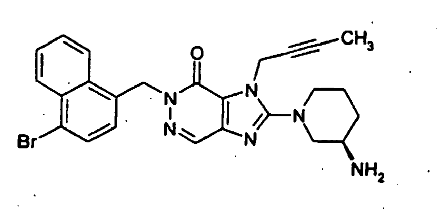

Hergestellt analog Beispiel 1j durch Abspaltung der Boc-Schutzgruppe mit Trifluoressigsäure in Dichlormethan.
Ausbeute: 67% der Theorie.
C₂₅H₂₅BrN₆O (505.42)
Massenspektrum: (M + H)⁺ = 505/7
¹H-NMR-Spektrum (d₆-DMSO): δ = 1.70 (m, 2H); 1.80 (s, 3H); 1.93 (m, 1H); 2.04 (m, 1H); 3.20 (m, 2H); 3.40 (m, 1 H); 3.51 (m, 1 H); 3.74 (m, 1 H); 5.117 (dd, 2H); 5.78 (s, 2H); 7.16 (d, 1H); 7.70 (m, 2H); 7.83 (d, 1 H); 8.20 (d, 1 H); 8.32 (s, 1 H); 8.37 (d, 1 H); 8.47 (breites s, 3H) ppm.

### Beispiel 202

### 2-((R)-3-Amino-piperidin-1-yl)-3-(but-2-inyl)-5-benzo[1,2,5]oxadiazol-5-ylmethyl) 3,5-dihydro-imidazo[4,5-d]pyridazin-4-on

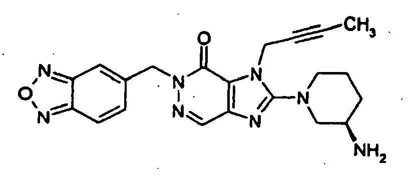

Hergestellt analog Beispiel 1j durch Abspaltung der Boc-Schutzgruppe mit Trifluoressigsäure in Dichlormethan.
Ausbeute: 83% der Theorie.
C₂₁H₂₂N₈O₂ (418.46)
Massenspektrum: (M + H)⁺ = 419
¹H-NMR-Spektrum (d₆-DMSO): δ = 1.41 (m, 1H); 1.67 (m, 1H); 1.80 (s, 3H); 1.82 (m, 1H); 1.93 (m, 1H); 2.42 (dd, 1H); 3.10 (m, 2H); 3.57 (m, 1H); 3.68 (dd, 1H); 5.11 (s, 2H); 5.47 (s, 2H); 7.53 (d, 1H); 7.76 (s, 1H); 8.04 (d, 1H); 8.3 (s, 1H); ppm.

### Beispiel 203

### 2-((R)-3-Amino-piperidin-1-yl)-3-(but-2-inyl)-5-(benzo[1,2,5]thiadiazol-4-ylmethyl)-3,5-dihydro-imidazo[4,5-d]pyridazin-4-on

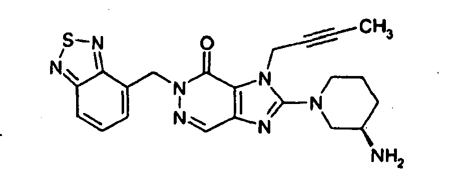

Hergestellt analog Beispiel 1j durch Abspaltung der Boc-Schutzgruppe mit Trifluoressigsäure in Dichlormethan.
Ausbeute: 73% der Theorie.
C₂₁H₂₂N₈OS (434.53)
Massenspektrum: (M + H)⁺ = 435
¹H-NMR-Spektrum (d₆-DMSO): δ = 1.30 (m, 1H); 1.65 (m, 1 H); 1.79 (s, 3H); 1.82 (m, 1 H); 1.90 (m, 1H) 2.82 (dd, 1H); 2.96 (m, 1H); 3.04 (m, 1H), 3.61 (m, 1H); 3.66 (dd, 1H); 5.12 (s, 2H); 5.84 (s, 2H); 7.22 (d, 1H); 7.64 (t, 1H); 8.01 (d, 1H); 8.30 (s, 1H); ppm.

### Beispiel 204

### 2-((R)-3-Amino-piperidin-1-yl)-3-(but-2-inyl)-5-(benzo[1,2,5]thiadiazol-5-ylmethyl)-3,5-dihydro-imidazo[4,5-d]pyridazin-4-on

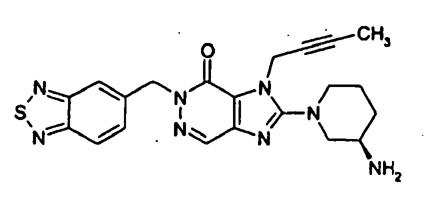

Hergestellt analog Beispiel 1j durch Abspaltung der Boc-Schutzgruppe mit Trifluoressigsäure in Dichlormethan.
Ausbeute: 81% der Theorie.
C₂₁H₂₂N₈OS (434.53)
Massenspektrum: (M + H)⁺ = 435
¹H-NMR-Spektrum (d₆-DMSO): δ = 1.30 (m, 1H); 1.65 (m, 1H); 1.79 (s, 3H); 1.81 (m, 1 H); 1.90 (m, 1 H); 2.32 (dd, 1H); 2.95 (m, 1 H); 3.03 (m, 1 H); 3.57 (m, 1 H); 3.65 (dd, 1 H); 5.11 (s, 2H); 5.54 (s, 2H); 7.66 (d, 1 H); 7.85 (s, 1 H); 8.07 (d, 1 H); 8.31 (s, 1H) ppm.

### Beispiel 205

### 2-((R)-3-Amino-piperidin-1-yl)-3-(2-chlorbenzyl)-5-(3-methyl-isochinolin-1-ylmethyl)-3,5-dihydro-imidazo[4,5-d]pyridazin-4-on

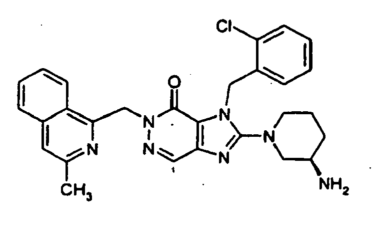

Hergestellt analog Beispiel 1j durch Abspaltung der Boc-Schutzgruppe mit Trifluoressigsäure in Dichlormethan.
Ausbeute: 70% der Theorie.
C₂₈H₂₈ClN₇O (514.03)
Massenspektrum: (M + H)⁺ = 514/6
¹H-NMR-Spektrum (d₆-DMSO): δ = 1.14 (m, 1H), 1.46 (m, 1H); 1.64 (m, 1H); 1.79 (m, 1H); 2.44 (s, 3H); 2.62 (dd, 1H); 2.70)m, 1H); 2.85 (m, 1H); 3.20 - 3.44 (m, 2H); 5.63 (s, 2H); 5.88 (s, 2H); 6.70 (d, 1 H); 7.25 (t, 1H); 7.32 (t, 1H); 7.52 (m, 3H); 7.71 (t, 1H); 7.85 (d, 1H); 8.18 (d, 1 H); 8.30 (s, 1 H) ppm.

### Beispiel 206

### 2-((R)-3-Amino-piperidin-1-yl)-3-(but-2-inyl)-5-(2-methyl-naphthalin-1-ylmethyl-3,5-dihydro-imidazo[4,5-d]pyridazin-4-on

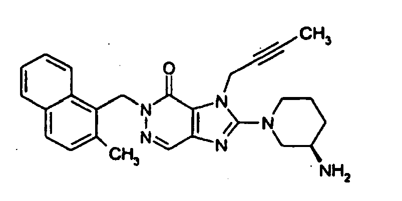

Hergestellt analog Beispiel 1j durch Abspaltung der Boc-Schutzgruppe mit Trifluoressigsäure in Dichlormethan.
Ausbeute: 56% der Theorie.
C₂₆H₂₈N₆O (440.55)
Massenspektrum: (M + H)⁺ = 441
Rf-Wert: 0.29 (Kieselgel; Dichlormethan/Methanol 9 : 1)

### Beispiel 207

### 2-((R)-3-Amino-piperidin-1-yl)-3-(but-2-inyl)-5-(5-methyl-imidazo[1,2-a]pyridin-2-ylmethyl)-3,5-dihydro-imidazo[4,5-d]pyridazin-4-on

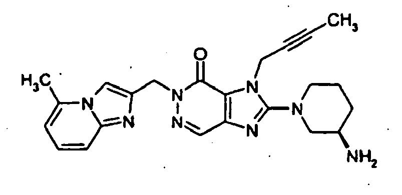

Hergestellt analog Beispiel 1j durch Abspaltung der Boc-Schutzgruppe mit Trifluoressigsäure in Dichlormethan.
Ausbeute: 86% der Theorie.
C₂₃H₂₆N₈O (430.52)
Massenspektrum: (M + H)⁺ = 431
Rf-Wert: 0.37 (Kieselgel; Dichlormethan / Methanol / konz. Ammoniak 9 : 1 : 0.1)

### Beispiel 208

### 2-((R)-3-Amino-piperidin-1-yl)-3-(but-2-inyl)-5-(isochinolin-3-ylmethyl)-3,5-dihydro-imidazo[4,5-d]pyridazin-4-on

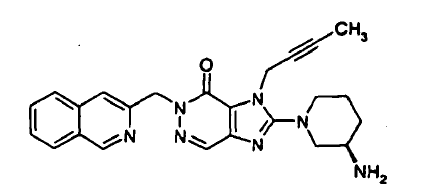

Hergestellt analog Beispiel 1j durch Abspaltung der Boc-Schutzgruppe mit Trifluoressigsäure in Dichlormethan.
Ausbeute: 97% der Theorie.
C₂₄H₂₅N₇O (427.51)
Massenspektrum: (M + H)⁺ = 428
Rf-Wert: 0.15 (Kieselgel; Dichlormethan / Methanol 9 : 1)

### Beispiel 209

### 2-((R)-3-Amino-piperidin-1-yl)-3-(but-2-inyl)-5-(1-methyl-1H-chinolin-2-on-6-ylmethyl)-3,5-dihydro-imidazo[4,5-d]pyridazin-4-on

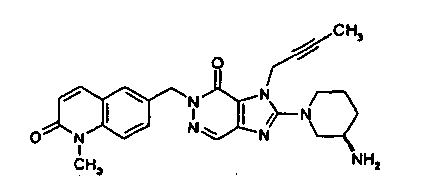

Hergestellt analog Beispiel 1j durch Abspaltung der Boc-Schutzgruppe mit Trifluoressigsäure in Dichlormethan.
Ausbeute: 32% der Theorie.
C₂₅H₂₇N₇O₂ (457.54)
Massenspektrum: (M + H)⁺ = 458
Rf-Wert: 0.11 (Kieselgel; Dichlormethan / Methanol 9 : 1)

### Beispiel 210

### 2-((R)-3-Amino-piperidin-1-yl)-3-(but-2-inyl)-5-(2-methyl-2H-indazol-3-ylmethyl)-3,5-dihydro-imidazo[4,5-d]pyridazin-4-on

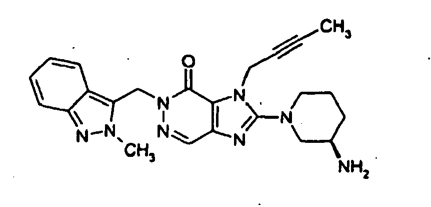

Hergestellt analog Beispiel 1j durch Abspaltung der Boc-Schutzgruppe mit Trifluoressigsäure in Dichlormethan.
Ausbeute: 84% der Theorie.
C₂₃H₂₆N₈O (430.52)
Massenspektrum: (M + H)⁺ = 431
Rf-Wert: 0.18 (Kieselgel; Dichlormethan / Methanol 9 : 1)

### Beispiel 211

### 2-((R)-3-Amino-piperidin-1-yl-3-(but-2-inyl)-7-methyl-5-(4-phenyl-chinazolin-2-ylmethyl)-3,5-dihydro-imidazo[4,5-d]pyridazin-4-on

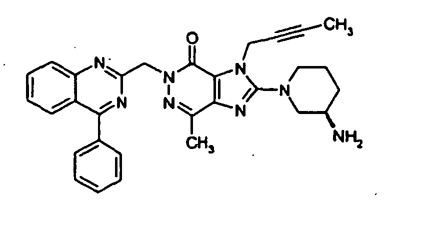

Hergestellt analog Beispiel 1j durch Abspaltung der Boc-Schutzgruppe mit Trifluoressigsäure in Dichlormethan.
Ausbeute: 29% der Theorie.
C₃₀H₃₀N₈O (518.63)
Massenspektrum: (M + H)⁺ = 519
Rf-Wert: 0.26 (Kieselgel; Dichlormethan / Methanol 8 : 2)

### Beispiel 212

### 2-((R)-3-Amino-piperidin-1-yl)-3-(but-2-inyl)-7-methyl-5-(4-methyl-chinazolin-2-ylmethyl)-3,5-dihydro-imidazo[4,5-d]pyridazin-4-on

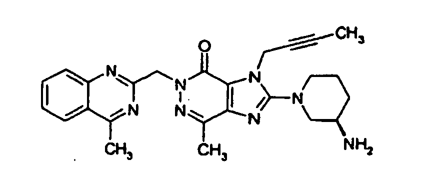

Hergestellt analog Beispiel 1j durch Abspaltung der Boc-Schutzgruppe mit Trifluöressigsäure in Dichlormethan.
Ausbeute: 61% der Theorie.
C₂₅H₂₈N₈O (456.55)
Massenspektrum: (M + H)⁺ = 457
Rf-Wert: 0.27 (Kieselgel; Dichlormethan / Methanol 9 : 1)

### Beispiel 213

### 2-((R)-3-Amino-piperidin-1-yl)-3-(but-2-inyl)-7-methyl-5-(3-methyl-isochinolin-1-ylmethyl)-3,5-dihydro-imidazo[4,5-d]pyridazin-4-on

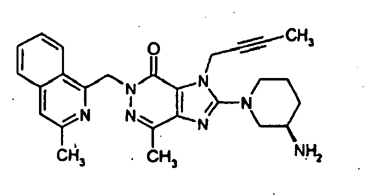

Hergestellt analog Beispiel 1j durch Abspaltung der Boc-Schutzgruppe mit Trifluoressigsäure in Dichlormethan.
Ausbeute: 56% der Theorie.
C₂₆H₂₉N₇O (455.57)
Massenspektrum: (M + H)⁺ = 456
Rf-Wert: 0.53
(Kieselgel RP-8; Wasser / Acetonitril / Trifluoressigsäure 50 : 50 : 0.1)

### Beispiel 214

### 2-((R)-3-Amino-piperidin-1-yl)-3-(but-2-inyl)-5-(1-methyl)-1H-indazo)-4-ylmethyl)-3,5-dihydro-imidazo[4,5-d]pyridazin-4-on

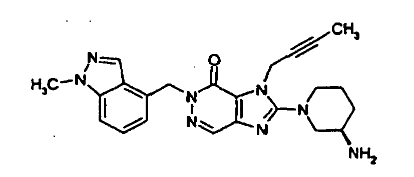

Hergestellt analog Beispiel 1j durch Abspaltung der Boc-Schutzgruppe mit Trifluoressigsäure in Dichlormethan.
Ausbeute: 45% der Theorie.
C₂₃H₂₆N₈O (430.52)
Massenspektrum: (M + H)⁺ = 431
HPLC-Analytik:
Die HPLC-Analytik erfolgte unter folgenden experimentellen Bedingungen:
   Säule: Xterra MS18; 3.5µm; 4.6 x 50 mm
   Fluß: 1 mUmin
   Fließmittel A: Wasser / 0.1 % Trifluoressigsäure
   Fließmittel B: Acetonitril / 0.1 % Trifluoressigsäure
   Gradient: 5% B → 98% B innerhalb 5 min.; 98% B halten bis 7.5 min.
Für diese Substanz wurde eine Retentionszeit von 3.13 min. beobachtet.

### Beispiel 215

### 2-((R)-3-Amino-piperidin-1-yl)-3-(but-2-inyl)-5-(4-methyl-phthalazin-1-ylmethyl)-3,5-dihydro-imidazo[4,5-d]pyridazin-4-on

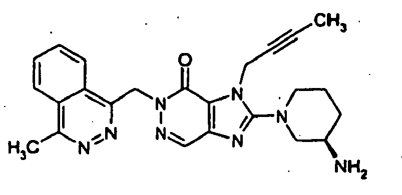

Hergestellt analog Beispiel 1j durch Abspaltung der Boc-Schutzgruppe mit Trifluoressigsäure in Dichlormethan.
Ausbeute: 48% der Theorie.
C₂₄H₂₆N₈O (442.53)
Massenspektrum: (M + H)⁺ = 433
HPLC-Analytik (siehe Beispiel 214):
Retentionszeit: 2.53 min.

### Beispiel 216

### 2-((R)-3-Amino-piperidin-1-yl)-3-(but-2-inyl)-5-[2-(3-methyl-2-oxo-2,3-dihydrobenzoxazol-4-yl)-2-oxo-ethyl]-3,5-dihydro-imidazo[4,5-d]pyridazin-4-on

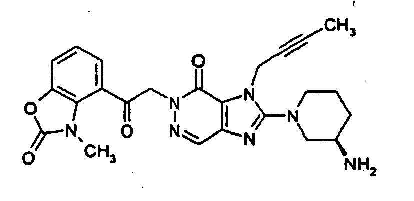

Hergestellt analog Beispiel 1j durch Abspaltung der Boc-Schutzgruppe mit Trifluoressigsäure in Dichlormethan.
Ausbeute: 36% der Theorie.
C₂₄H₂₅N₇O₄ (475.51)
Massenspektrum: (M + H)⁺ = 476
HPLC-Analytik (siehe Beispiel 214):
Retentionszeit: 3.30 min.

### Beispiel 217

### 2-((R)-3-Amino-piperidin-1-yl)-3-(but-2-inyl)-5-(2-methyl-2H-isochinolin-1-on-4-ylmethyl)-3,5-dihydro-imidazo[4,5-d]pyridazin-4-on

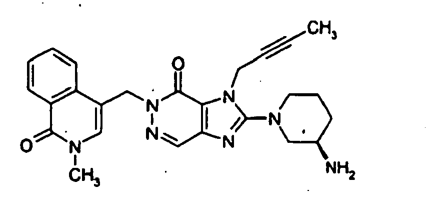

Hergestellt analog Beispiel 1j durch Abspaltung der Boc-Schutzgruppe mit Trifluoressigsäure in Dichlormethan.
Ausbeute: 18% der Theorie.
C₂₅H₂₇N₇O₂ (457.54)
Massenspektrum: (M + H)⁺ = 458
HPLC-Analytik (siehe Beispiel 214):
Retentionszeit: 2.67 min.

### Beispiel 218

### 2-((R)-3-Amino-piperidin-1-yl)-3-(but-2-inyl)-5-(8-methoxy-chinolin-5-ylmethyl)-3,5-dihydro-imidazo[4,5-d]pyridazin-4-on

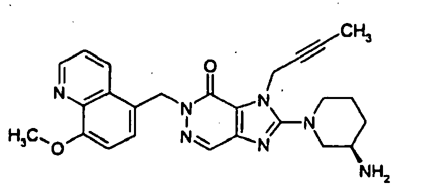

Hergestellt analog Beispiel 1j durch Abspaltung der Boc-Schutzgruppe mit Trifluoressigsäure in Dichlormethan.
Ausbeute: 33% der Theorie.
C₂₅H₂₇N₇O₂ (457.54)
Massenspektrum: (M + H)⁺ = 458
HPLC-Analytik (siehe Beispiel 214):
Retentionszeit: 3.18 min.

### Beispiel 219

### 2((R)-3-Amino-piperidin-1-yl)-3-(but-2-inyl)5-([1,5]naphthyridin-2-ylmethyl)-3,5-dihydro-imidazo[4,5-d]pyridazin-4-on

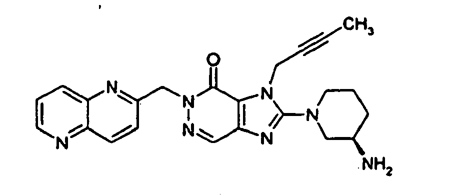

Hergestellt analog Beispiel 1j durch Abspaltung der Boc-Schutzgruppe mit Trifluoressigsäure in Dichlormethan.
Ausbeute: 25% der Theorie.
C₂₃H₂₄N₈O (428.50)
Massenspektrum: (M + H)⁺ = 429
HPLC-Analytik (siehe Beispiel 214):
Retentionszeit: 2.06 min.

### Beispiel 220

### 2-((R)-3-Amino-piperidin-1-yl)-3-(but-2-inyl)-5-(2,3,8-trimethyl-chinoxalin-6-ylmethyl)-3,5-dihydro-imidazo[4,5-d]pyridazin-4-on

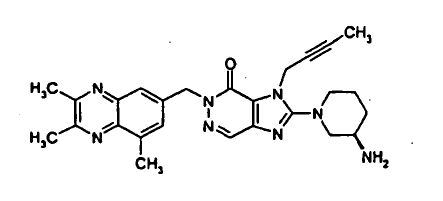

Hergestellt analog Beispiel 1j durch Abspaltung der Boc-Schutzgruppe mit Trifluoressigsäure in Dichlormethan.
Ausbeute: 76% der Theorie.
C₂₆H₃₀N₈O (470.58)
Massenspektrum: (M + H)⁺ = 471
HPLC-Analytik (siehe Beispiel 214):
Retentionszeit: 3.44 min.

### Beispiel 221

### 2-((R)-3-Amino-piperidin-1-yl)-3-(but-2-inyl)-5-(4-morpholin-4-yl-chinazolin-2-methyl)-3,5-dihydro-imidazo[4,5-d]pyridazin-4-on

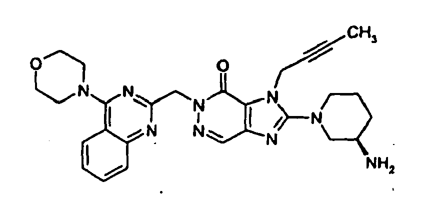

Hergestellt analog Beispiel 1j durch Abspaltung der Boc-Schutzgruppe mit Trifluoressigsäure in Dichlormethan.
Ausbeute: 71 % der Theorie.
C₂₇H₃₁N₉O₂ (513.61)
Massenspektrum: (M + H)⁺ = 514
HPLC-Analytik (siehe Beispiel 214):
Retentionszeit: 1.76 min.

### Beispiel 222

### 2-((R)-3-Amino-piperidin-1-yl)-3-(but-2-inyl)-5-(3-pentafluorphenyl-allyl)-3,5-dihydro-imidazo[4,5-d]pyridazin-4-on

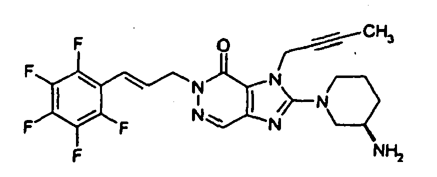

Hergestellt analog Beispiel 1j durch Abspaltung der Boc-Schutzgruppe mit Trifluoressigsäure in Dichlormethan.
Ausbeute: 89% der Theorie.
C₂₃H₂₁F₅N₆O (492.46)
Massenspektrum: (M + H)⁺ = 493
Rf-Wert: 0.18 (Kieselgel; Dichlormethan / Ethanol 9 : 1)

### Beispiel 223

### 2-((R)-3-Amino-piperidin-1-yl)-3-(but-2-inyl)-7-methyl-5-(4-cyano-naphthalin-1-ylmethyl)-3,5-dihydro-imidazo[4,5-d]pyridazin-4-on

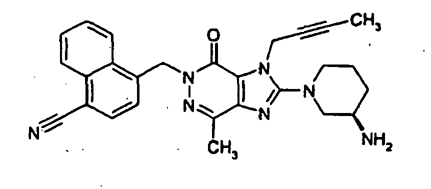

Hergestellt analog Beispiel 1j durch Abspaltung der Boc-Schutzgruppe mit Trifluoressigsäure in Dichlormethan.
Ausbeute: 66% der Theorie.
C₂₇H₂₇N₇O (465.56)
Massenspektrum: (M + H)⁺ = 466
Rf-Wert: 0.62

### (Kieselgel RP-8; Wasser / Acetonitril / Trifluoressigsäure 50 : 50 : 0.1)

### Beispiel 224

### Dragées mit 75 mg Wirksubstanz

### 1 Drageekem enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung_{:}

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragees werden mit Bienenwachs geglänzt.
Dragéegewicht: 245 mg.

### Beispiel 225

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet,

| | |
|---|---|
| Tablettengewicht: | 220 mg |
| Durchmesser: | 10 mm, biplan mit beidseitiger Facette und einseitiger Teilkerbe. |

### Beispiel 226

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1.0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 227

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hiffsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 228

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 229

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 230

### Ampullen mit 10 mg Wirksubstanz

Zusammensetzung:

| | |
|---|---|
| Wirkstoff 0,01 N Salzsäure s.q. | 10,0 mg |
| Aqua bidest | ad 2,0 ml |

### Herstellung:

Die Wirksubstanz wird in der erforderlichen Menge 0,01 N HCl gelöst, mit Kochsalz isotonisch gestellt, sterilfiltriert und in 2 ml Ampullen abgefüllt.

### Beispiel 231

### Ampullen mit 50 mg Wirksubstanz

Zusammensetzung:

| | |
|---|---|
| Wirkstoff 0,01 N Salzsäure s.q. | 50,0 mg |
| Aqua bidest | ad 10,0 ml |

### Herstellung:

Die Wirksubstanz wird in der erforderlichen Menge 0,01 N HCl gelöst, mit Kochsalz isotonisch gestellt, sterilfiltriert und in 10 ml Ampullen abgefüllt.

## Patentansprüche

1. Verbindungen der allgemeinen Formel 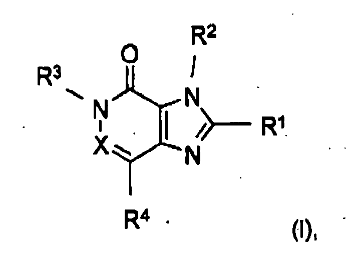 in der
X ein Stickstoffatom oder eine Gruppe der Formel C-R⁵,
wobei R⁵ ein Wasserstoffatom oder eine Methylgruppe bedeutet,
R¹ ein 5- bis 7-gliedrige Cycloalkyleniminogruppe, die im Kohlenstoffgerüst durch eine Aminogruppe substituiert ist und durch eine C₁₋₃-Alkylgruppe substituiert sein kann,
oder eine durch eine C₅₋₇-Cycloalkylgruppe substituierte Aminogruppe,
wobei die C₅₋₇-Cycloalkylgruppe durch eine Aminogruppe substituiert ist oder ein Kohlenstoffatom in 3-Position der C₅₋₇-Cycloalkylgruppe durch eine -NH- Gruppe ersetzt ist,
R² eine Benzylgruppe, in der der Phenylrest durch ein oder zwei Fluor-, Chlor-oder Bromatome oder durch eine Cyanogruppe substituiert sein kann,
eine lineare oder verzweigte C₃₋₈-Alkenylgruppe,
eine C₃₋₅-Alkinylgruppe,
eine C₃₋₇-Cycloalkylmethylgruppe,
eine C₅₋₇-Cycloalkenylmethytgruppe,
oder eine Furylmethyl-, Thienylmethyl-, Pyrrolylmethyl-, Thiazolylmethyl-, Imidazolylmethyl-, Pyridinylmethyl-, Pyrimidinylmethyl-, Pyridazinylmethyl- oder Pyrazinylmethylgruppe,
R³ eine lineare oder verzweigte C₁₋₆-Alkylgruppe.,
eine gegebenenfalls im Arylteil durch ein Halogenatom, eine Cyano-, eine C₁₋₃-Alkyl- oder eine Methoxygruppe substituierte Phenyl-C₁₋₃-alkyl- oder Naphthyl-C₁₋₃-alkylgruppe,
eine 2-Phenyl-2-hydroxy-ethylgruppe,
eine Phenylcarbonylmethylgruppe,
in der die Phenylgruppe durch eine Hydroxy-, C₁₋₃-Alkyloxy-, Aminocarbonyl-C₁₋₃-alkoxy-, (C₁-₃-Alkylamino)-carbonyl-C₁₋₃alkoxy-, [Di-(C₁₋₃-alkyl)-amino]-carbonyl-C₁₋₃-alkoxy-, Amino-, C₁₋₃-Alkyl-carbonylamino-, C₃₋₆-Cycloalkyl-carbonylamino-, C₁₋₃-Alkoxy-carbonylamino-, C₁₋₃Alkylsulfonyl-amino- oder Aminocarbonylgruppe substituiert sein kann,
eine (3-Methyl-2-oxo-2,3-dihydro-benzoxazolyl)-carbonylmethylgruppe,
eine Thienylcarbonylmethylgruppe,
eine Heteroaryl-C₁₋₃-alkylgruppe,
wobei unter dem Ausdruck "Heteroarylgruppe" eine gegebenenfalls durch eine oder zwei C₁₋₃-Alkylgruppen oder durch eine Morpholin-4-yl-, Pyridyl-oder Phenylgruppe substituierte monocyclische 5- oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-oder Phenyl-C₁₋₃-alkylgruppe substituierte Iminogruppe, ein Sauerstoff- oder Schwefelatom oder
eine gegebenenfalls durch eine C₁₋₃-Alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe oder ein Sauerstoff- oder Schwefelatom und zusätzlich zwei oder drei Stickstoffatome enthält,
und wobei zusätzlich an die vorstehend erwähnten monocyclischen Heteroacrylgruppen über zwei benachbarte Kohlenstoffatome ein Phenylring, der gegebenenfalls durch ein Halogenatom, durch eine oder zwei C₁₋₃-Alkylgruppen oder durch eine Trifluormethyl- oder Methoxygruppe substituiert sein kann, ankondensiert sein kann
und die Bindung über ein Atom des heterocyclischen Teils oder des ankondensierten Phenylringes erfolgen kann,
eine bicyclische Heteroarylmethylgruppe gemäß einer der Formeln 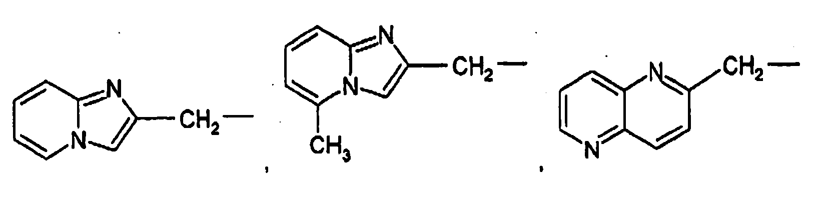 oder 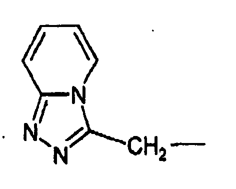 eine Gruppe der Formel 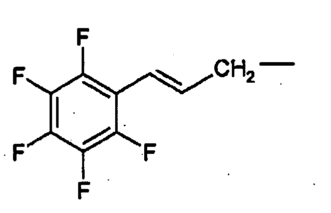 oder eine Gruppe der Formeln 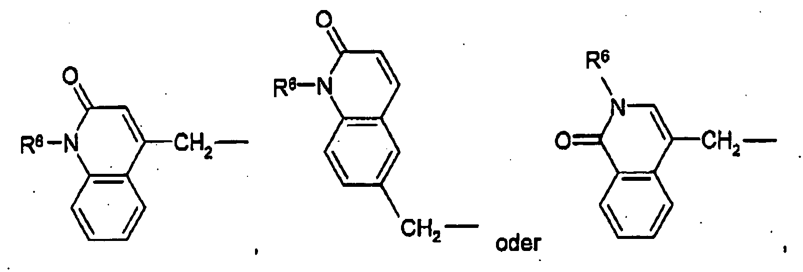 in der R⁶ jeweils ein Wasserstoffatom oder eine Methylgruppe bedeutet,
und R⁴ ein Wasserstoffatom oder eine C₁₋₃-Alkylgruppe bedeuten,
wobei die in den Definitionen enthaltenen Alkyl- und Alkoxygruppen, die mehr als zwei Kohlenstoffatome aufweisen, soweit nichts anderes erwähnt wurde, geradkettig oder verzweigt sein können,
und wobei die Wasserstoffatome der in den Definitionen enthaltenen Methyl- oder Ethylgruppen ganz oder teilweise durch Fluoratome ersetzt sein können,
mit der Maßgabe, dass die folgenden beiden Verbindungen ausgeschlossen sind:
2-(2-Amino-cyclohexylamino)-3-(but-2-inyl)-5-methyl-3,5-dihydro-imidazo[4,5-d]pyridazin-4- und
2-(3-Amino-piperidin-1-yl)-3-(but-2-inyl)-5-methyl-3,5-dihydro-imidazo[4,5-d]pyridazin-4-,
deren Tautomere, deren Enantiomere, deren Diastereomere, deren Gemische, und deren Salze.

2. Verbindungen der allgemeinen Formel I gemäß Anspruch 1, in der
X ein Stickstoffatom oder eine Methingruppe,
R¹ eine 3-Amino-piperidin-1-yl-, 3-Amino-3-methyl-piperidin-1-yl-, 3-Amino-pyrrolidin-1-yl-, 1,4-Diazepan-1-yl-, (2-Amino-cyclohexyl)-amino-oder Piperidin-3-yl-aminogruppe,
R² eine Benzylgruppe, in der der Phenylrest durch ein oder zwei Fluoratome, durch ein Chlor- oder Bromatom oder durch eine Cyanogruppe substituiert sein kann,
eine lineare oder verzweigte C₃₋₈-Alkenylgruppe,
eine Propin-3-yl- oder But-2-in-4-ylgruppe,
eine Cyclopropylmethylgruppe,
eine C₅₋₇-Cycloalkenylmethylgruppe,
oder eine Furylmethyl- oder Thienylmethylgruppe,
R³ eine lineare oder verzweigte C₁₋₈-Alkylgruppe,
eine gegebenenfalls im Arylteil durch ein Fluor-, Chlor- oder Bromatom oder durch eine Cyano-, C₁₋₃-Alkyl- oder Methoxygruppe substituierte Phenyl-C₁₋₂-alkyl- oder Naphthyl-C₁₋₂-alkylgruppe,
eine 2-Phenyl-2-hydroxy-ethylgruppe,
eine Phenylcarbonylmethylgruppe,
in der die Phenylgruppe durch eine Hydroxy-, C₁₋₃-Alkyloxy-, Aminocarbonyl-C₁₋₃-alkoxy-, (C₁₋₃-Alkylamino)-carbonyl-C₁₋₃alkoxy-, [Di-(C₁₋₃-alkyl)-amino]-carbonyl-C₁₋₃-alkoxy-, Amino-, C₁₋₃-Alkyl-carbonylamino-,C₃₋₆-Cycloalkyl-carbonylamino-, C₁₋₃-Alkoxy-carbonylamino-, C₁₋₃-Alkylsulfonylamino- oder Aminocarbonylgruppe substituiert sein kann,
eine (3-Methyl-2-oxo-2,3-dihydro-benzoxazolyl)-carbonylmethylgruppe,
eine Thienylcarbonylmethylgruppe,
eine Heteroaryl-methylgruppe,
wobei unter dem Ausdruck "Heteroarylgruppe" eine gegebenenfalls durch eine oder zwei Methylgruppen oder durch eine Pyridyl- oder Phenylgruppe substituierte Pyridinyl-, Pyrimidinyl-, Pyridazinyl-, Pyrazinyl-, Thiazolyl-, Oxazolyl-, Isothiazolyl-, Isoxazolyl-, Pyrazolyl-, Imidazolyl-, Oxadiazolyl-, Thiadiazolyl- oder Thienylgruppe zu verstehen ist,
und wobei zusätzlich an die vorstehend erwähnten monocyclischen Heteroarylgruppen über zwei benachbarte Kohlenstoffatome ein Phenylring, der gegebenenfalls durch ein Chloratom, durch eine oder zwei Methylgruppen oder durch eine Trifluormethyl- oder Methoxygruppe substituiert sein kann, ankondensiert sein kann
und die Bindung über ein Atom des heterocyclischen Teils oder des ankondensierten Phenylrings erfolgen kann,
eine Imidazo[1,2-a]pyridin-2-yl-methyl-gruppe der Formeln 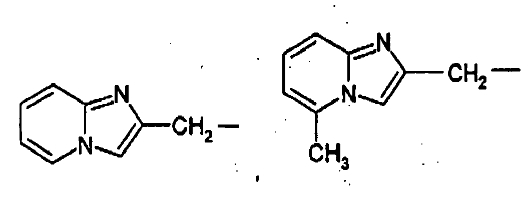 oder 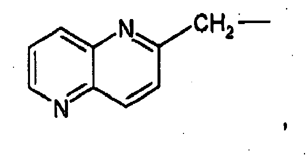 eine 1,2,4-Triazolo[4,3-a]pyridin-3-yl-gruppe der Formel 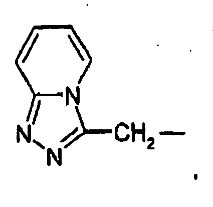 oder eine Gruppe der Formeln 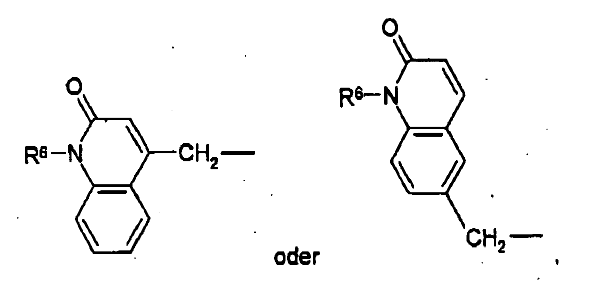 in der R⁶ jeweils ein Wasserstoffatom oder eine Methylgruppe bedeutet,
und R⁴ ein Wasserstoffatom oder eine Methylgruppe bedeuten,
wobei die in den Definitionen enthaltenen Alkyl- und Alkoxygruppen, die mehr als zwei Kohlenstoffatome aufweisen, soweit nichts anderes erwähnt wurde, geradkettig oder verzweigt sein können,
und wobei die Wasserstoffatome der in den Definitionen enthaltenen Methyl- oder Ethylgruppen ganz oder teilweise durch Fluoratome ersetzt sein können,
mit der Maßgabe, dass die folgenden beiden Verbindungen ausgeschlossen sind:
2-(2-Amino-cyclohexylamino)-3-(but-2-inyl)-5-methyl-3,5-dihydro-imidazo[4,5-d]pyridazin-4- und
2-(3-Amino-piperidin-1-yl)-3-(but-2-inyl)-5-methyl-3,5-dihydro-imidazo[4,5-d]pyridazin-4-.
deren Tautomere, deren Enantiomere, deren Diastereomere, deren Gemische und deren Salze.

3. Verbindungen der allgemeinen Formel I gemäß Anspruch 1, in der
X, R², R³ und R⁴ wie in Anspruch 2 erwähnt definiert sind und
R¹ eine 3-Amino-piperidin-1-yl-gruppe bedeutet,
mit der Maßgabe, dass die folgende Verbindung ausgeschlossen ist:
2-(3-Amino-piperidin-1-yl)-3-(but-2-inyl)-5-methyl-3,5-dihydro-imidazo[4,5-d]pyridazin-4-,
deren Tautomere, deren Enantiomere, deren Diastereomere, deren Gemische und deren Salze.

4. Verbindungen der allgemeinen Formel I gemäß Anspruch 1, in der
X, R¹, R³ und R⁴ wie in Anspruch 2 oder 3 erwähnt definiert sind und
R² eine 3-Methylallyl-, eine 3,3-Dimethylallyl- oder eine But-2-in-4-yigruppe bedeutet,
mit der Maßgabe, dass die folgenden beiden Verbindungen ausgeschlossen sind:
2-(2-Amino-cyclohexylamino)-3-(but-2-inyl)-5-methyl-3,5-dihydro-imidazo[4,5-d]pyridazin-4- und
2-(3-Amino-piperidin-1-yl)-3-(but-2-inyl)-5-methyl-3,5-dihydro-imidazo[4,5-d]pyridazin-4-.
deren Tautomere, deren Enantiomere, deren Diastereomere, deren Gemische und deren Salze.

5. Folgende Verbindungen der allgemeinen Formel I gemäß Anspruch 1:
(1) 2-(3-Amino-piperidin-1-yl)-3-(but-2-inyl)-5-(naphthalin-1-ylmethyl)-3,5-dihydro-imidazo[4,5-d]pyridazin-4- 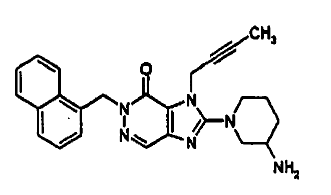
(2) 2-(3-Amino-piperidin-1-yl)-3-(but2-inyl)-5-(3-methyl-isochinolin-1-ylmethyl)-3,5-dihydro-imidazo[4,5-d]pyridazin-4- 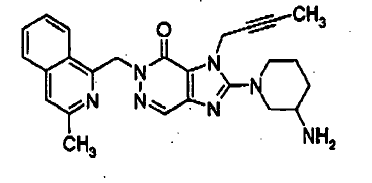
(3) 2-(3-Amino-piperidin-1-yl)-3-(but-2-inyl)-5-(chinazolin-2-ylmethyl)-3,5-dihydro-imidazo[4,5-d]pyridazin-4- 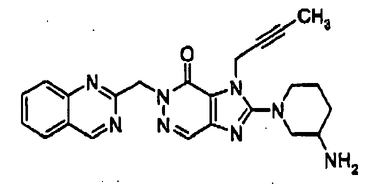
(4) 2-((*R*)-3-Amino-pipend)n-1-yl)-3-(but-2-inyl)-5-(4-methyl-chinazolin-2-ylmethyl)-3,5-dihydro-imidazo[4,5-d]pyridazin-4- 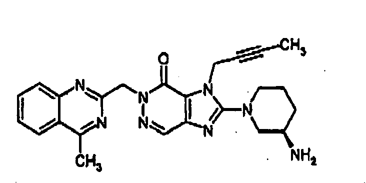
(5) 2-((*R*)-3-Amino-piperidin-1-yl)-3-(but-2-inyl)-5-(4-cyano-naphthalin-1-ylmethyl)-3,5-dihydro-imidazo[4,5-d]pyridazin-4- 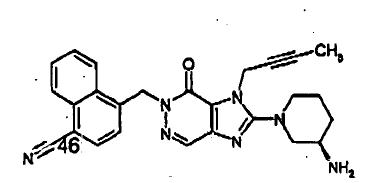
(6) 2-((*R*)-3-Amino-piperidin-1-yl)-3-(but-2-inyl)-5-(4-bromnaphth-1-ylmethyl)-3,5-dihydro-imidazo[4,5-d]pyridazin-4- 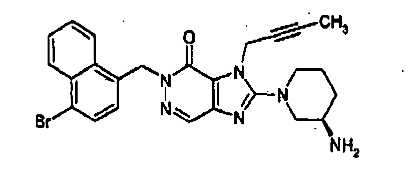
(7) 2-((*R*)-3-Amino-piperidin-1-yl)-3-(but-2-inyl)-5-(benzo[1,2,5]thiadiazol-5-ylmethyl)-3,5-dihydro-imidazo[4,5-d]pyridazin-4- 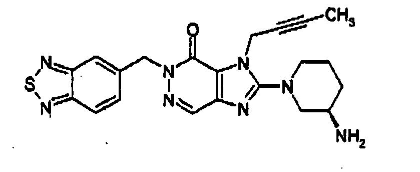
(8) 2-((*R*)-3-Amino-piperidin-1-yl)-3-(2-chlorbenzyl)-5-(3-methyl-isochinolin-1-ylmethyl)-3,5-dihydro-imidazo[4,5-d]pyridazin-4- 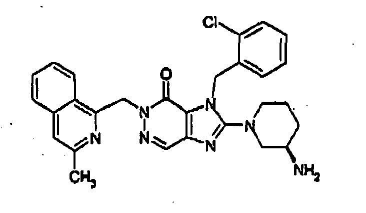
(9) 2-(3-Amino-piperidin-1-yl)-3-(but-2-inyl)-5-(chinozalin-6-ylmethyl)3,5-dihydro-imidazo[4,5-d]pyridazin-4- 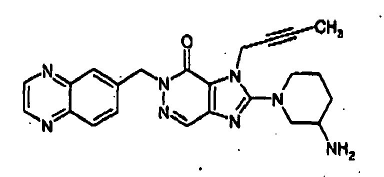
(10) 2-((*R*)-3-Amino-piperidin-1-yl)-3-(but-2-inyl)-5-(2,3-dimethyl-chinozalin-6-ylmethyl)-3,5-dihydro-imidazo[4,5-d]pyridazin4- 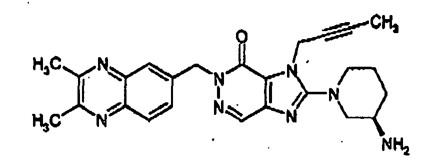
(11) 2-((*R*)-3-Amino-piperidin-1-yl)-3-(but-2-inyl)-5-(5-methyl-imidazo[1,2-a]pyridin-2-ylmethyl)-3,5-dihydro-imidazo[4,5-d]pyridazin-4- 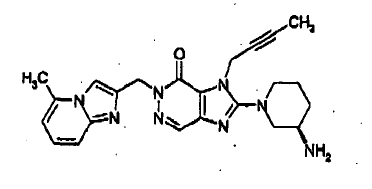
(12) 2-((*R*)-3-Amino-piperidin-1-yl)-3-(but-2-inyl)-5-(1-methyl-1*H*-chinolin-2-on-6 ylmethyl)-3,5-dihydro-imidazo[4,5-d]pyridazin-4- 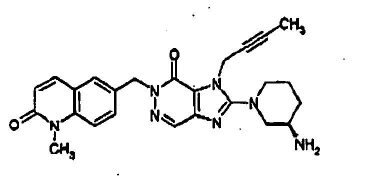
(13) 2-((*R*)-3-Amino-piperidin-1-yl)-3-(but-2-inyl)-5-(4-methyl-phthalazin-1-ylmethyl)-3,5-dihydro-imidazo[4,5-d]pyridazin-4- 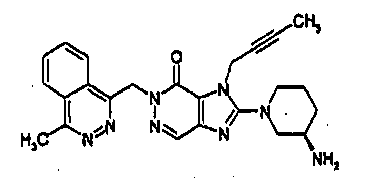
(14) 2-((*R*)-3-Amino-piperidin-1-yl)-3-(but-2-inyl)-5-([1,5]naphthyridin-2-ylmethyl)-3,5-dihydro-imidazo[4,5-d]pyridazin-4- 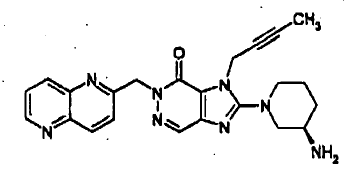
(15) 2-((*R*)-3-Amino-piperidin-1-yl)-3-(but-2-inyl)-5-(2,3,8-trimethyl-chinoxalin-6-ylmethyl)-3,5-dihydro-imidazo[4,5-d]pyridazin-4- 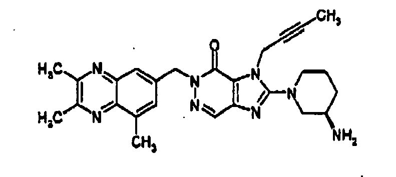
sowie deren Enantiomere und deren Salze.

6. Physiologisch verträgliche Salze der Verbindungen gemäß den Ansprüchen 1 bis 5 mit anorganischen oder organischen Säuren.

7. Arzneimittel, enthaltend eine Verbindung nach mindestens einem der Ansprüche 1 bis 5 oder ein Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 5 oder eines Salzes gemäß Anspruch 6 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

9. Eine Verbindung nach mindestens einem der Ansprüche 1 bis 5 oder eines Salzes gemäß Anspruch 6 für die Behandlung von Diabetes mellitus Typ I und Typ II, Arthritis, Adipositas, Allograft Transplantation und durch Calcitonin verursachte Osteoporose.

10. Verfahren zur Herstellung eines Arzneimittels gemäß Anspruch 7, **dadurch gekennzeichnet, daß** auf nichtchemischem Wege eine Verbindung nach mindestens einem der Ansprüche 1 bis 5 oder ein Salz gemäß Anspruch 6 in einen oder mehrere inerte Trägerstoffe und/oder verdünnungsmittel eingearbeitet wird.

11. Verfahren zur Herstellung der Verbindungen der Formel I gemäß den Ansprüchen 1 bis 6, **dadurch gekennzeichnet, daß**
a) eine Verbindung der allgemeinen Formel 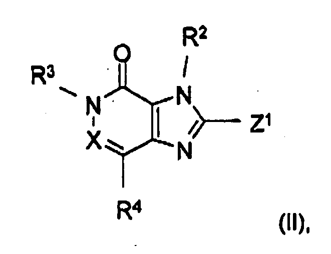 in der X, R², R³ und R⁴ wie in den Ansprüchen 1 bis 5 erwähnt definiert sind und Z¹ eine nukleofuge Austrittsgruppe wie beispielsweise ein Chlor- oder Bromatom oder eine C₁₋₃-Alkylsulfanyl-, C₁₋₃-Alkylsulfinyl- oder C₁₋₃-Alkylsulfonylgruppe bedeutet,
mit einem Amin der allgemeinen. Formel
H-R¹ (III),
in der R¹ wie in den Ansprüchen 1 bis 5 erwähnt definiert ist, umgesetzt wird oder
b) eine Verbindung der allgemeinen Formel 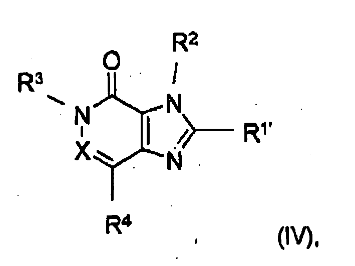 in der R², R³ und R⁴ wie in den Ansprüchen 1 bis 5 erwähnt definiert sind und R^{1'} eine der eingangs für R¹ erwähnten Gruppen bedeutet, in der die Imino-, Amino- bzw. Alkylaminogruppe durch eine Schutzgruppe substituiert ist, entschütz wird und
gewünschtenfalls anschließend ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure, übergeführt wird.

## Claims

1. Compounds of general formula 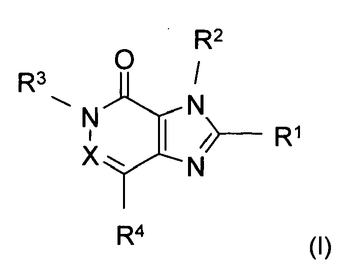 wherein
X denotes a nitrogen atom or a group of formula C-R⁵,
while R⁵ denotes a hydrogen atom or a methyl group,
R¹ denotes a 5- to 7-membered cycloalkyleneimino group which is substituted by an amino group in the carbon skeleton and may be substituted by a C₁₋₃-alkyl group,
or an amino group substituted by a C₅₋₇-cycloalkyl group,
while the C₅₋₇-cycloalkyl group is substituted by an amino group or a carbon atom in the 3 position of the C₅₋₇-cycloalkyl group is replaced by an -NH- group,
R² denotes a benzyl group wherein the phenyl group may be substituted by one or two fluorine, chlorine or bromine atoms or by a cyano group,
a straight-chain or branched C₃₋₈-alkenyl group,
a C₃₋₅-alkynyl group,
a C₃₋₇-cycloalkylmethyl group,
a C₅₋₇-cycloalkenylmethyl group,
or a furylmethyl, thienylmethyl, pyrrolylmethyl, thiazolylmethyl, imidazolylmethyl, pyridinylmethyl, pyrimidinylmethyl, pyridazinylmethyl or pyrazinylmethyl group,
R³ denotes a straight-chain or branched C₁₋₆-alkyl group,
a phenyl-C₁₋₃-alkyl or naphthyl-C₁₋₃-alkyl group optionally substituted in the aryl moiety by a halogen atom, a cyano, a C₁₋₃-alkyl or a methoxy group,
a 2-phenyl-2-hydroxy-ethyl group,
a phenylcarbonylmethyl group,
wherein the phenyl group may be substituted by a hydroxy, C₁₋₃-alkyloxy, aminocarbonyl-C₁₋₃-alkoxy, (C₁₋₃-alkylamino)-carbonyl-C₁₋₃-alkoxy, [di-(C₁₋₃-alkyl)-amino]-carbonyl-C₁₋₃-alkoxy, amino, C₁₋₃-alkylcarbonylamino, C₃₋₆-cycloalkyl-carbonylamino, C₁₋₃-alkoxycarbonylamino, C₁₋₃-alkylsulphonylamino or aminocarbonyl group,
a (3-methyl-2-oxo-2,3-dihydro-benzoxazolyl)-carbonylmethyl group,
a thienylcarbonylmethyl group,
a heteroaryl-C₁₋₃-alkyl group,
while by the phrase "heteroaryl group" is meant a monocyclic 5- or 6-membered heteroaryl group optionally substituted by one or two C₁₋₃-alkyl groups or by a morpholin-4-yl, pyridyl or phenyl group, while
the 6-membered heteroaryl group contains one, two or three nitrogen atoms and
the 5-membered heteroaryl group contains an imino group optionally substituted by a C₁₋₃-alkyl or phenyl-C₁₋₃-alkyl group, or an oxygen or sulphur atom or
an imino group optionally substituted by a C₁₋₃-alkyl or phenyl-C₁₋₃-alkyl group or an oxygen or sulphur atom and additionally contains a nitrogen atom or
an imino group optionally substituted by a C₁₋₃-alkyl or phenyl-C₁₋₃-alkyl group or an oxygen or sulphur atom and additionally contains two or three nitrogen atoms,
and additionally a phenyl ring, which may optionally be substituted by a halogen atom, by one or two C₁₋₃-alkyl groups or by a trifluoromethyl or methoxy group, may be fused to the above-mentioned monocyclic heteroaryl groups via two adjacent carbon atoms
and the bond may be formed via an atom of the heterocyclic moiety or of the fused-on phenyl ring,
a bicyclic heteroarylmethyl group according to one of the formulae 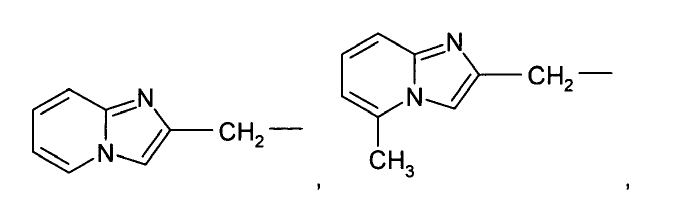 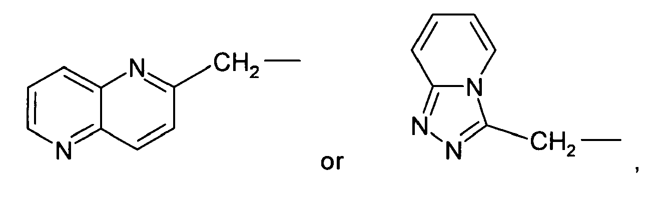 a group of formula 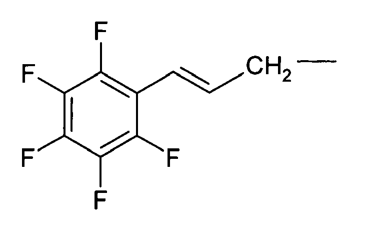 or a group of formulae 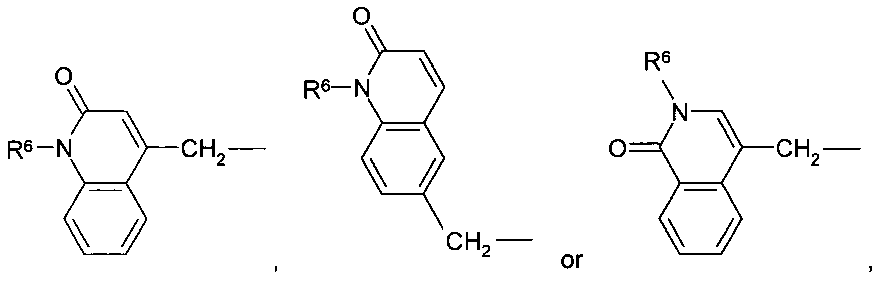 wherein R⁶ in each case denotes a hydrogen atom or a methyl group,
and R⁴ denotes a hydrogen atom or a C₁₋₃-alkyl group,
while unless otherwise stated the alkyl and alkoxy groups listed in the definitions which have more than two carbon atoms may be straight-chain or branched,
and the hydrogen atoms of the methyl or ethyl groups listed in the definitions may be wholly or partly replaced by fluorine atoms,
with the proviso that the following two compounds are excluded:
2-(2-amino-cyclohexylamino)-3-(but-2-ynyl)-5-methyl-3,5-dihydro-imidazo[4,5-d]pyridazin-4-one and
2-(3-amino-piperidin-1-yl)-3-(but-2-ynyl)-5-methyl-3,5-dihydro-imidazo[4,5-d]pyridazin-4-one,
the tautomers, the enantiomers, the diastereomers, the mixtures thereof and the salts thereof.

2. Compounds of general formula I according to claim 1, wherein
X denotes a nitrogen atom or a methyne group,
R¹ denotes a 3-amino-piperidin-1-yl, 3-amino-3-methyl-piperidin-1-yl, 3-amino-pyrrolidin-1-yl, 1,4-diazepan-1-yl, (2-amino-cyclohexyl)-amino or piperidin-3-yl-amino group,
R² denotes a benzyl group wherein the phenyl group may be substituted by one or two fluorine atoms, by a chlorine or bromine atom or by a cyano group,
a straight-chain or branched C₃₋₈-alkenyl group,
a propyn-3-yl or but-2-yn-4-yl group,
a cyclopropylmethyl group,
a C₅₋₇-cycloalkenylmethyl group,
or a furylmethyl or thienylmethyl group,
R³ denotes a straight-chain or branched C₁₋₆-alkyl group,
a phenyl-C₁₋₂-alkyl or naphthyl-C₁₋₂-alkyl group optionally substituted in the aryl moiety by a fluorine, chlorine or bromine atom or by a cyano, C₁₋₃-alkyl or methoxy group,
a 2-phenyl-2-hydroxy-ethyl group,
a phenylcarbonylmethyl group,
wherein the phenyl group may be substituted by a hydroxy, C₁₋₃-alkyloxy, aminocarbonyl-C₁₋₃-alkoxy, (C₁₋₃-alkylamino)-carbonyl-C₁₋₃-alkoxy, [di-(C₁₋₃-alkyl)-amino]-carbonyl-C₁₋₃-alkoxy, amino, C₁₋₃-alkylcarbonylamino, C₃₋₆-cycloalkyl-carbonylamino, C₁₋₃-alkoxycarbonylamino, C₁₋₃-alkylsulphonylamino or aminocarbonyl group,
a (3-methyl-2-oxo-2,3-dihydro-benzoxazolyl)-carbonylmethyl group,
a thienylcarbonylmethyl group,
a heteroaryl-methyl group,
while by the phrase a "heteroaryl group" is meant a pyridinyl, pyrimidinyl, pyridazinyl, pyrazinyl, thiazolyl, oxazolyl, isothiazolyl, isoxazolyl, pyrazolyl, imidazolyl, oxadiazolyl, thiadiazolyl or thienyl group optionally substituted by one or two methyl groups or by a pyridyl or phenyl group,
and additionally a phenyl ring, which may optionally be substituted by a chlorine atom, by one or two methyl groups or by a trifluoromethyl or methoxy group, may be fused to the above-mentioned monocyclic heteroaryl groups via two adjacent carbon atoms
and the bond may be formed via an atom of the heterocyclic moiety or
of the fused-on phenyl ring,
an imidazo[1,2-a]pyridin-2-yl-methyl group of formulae 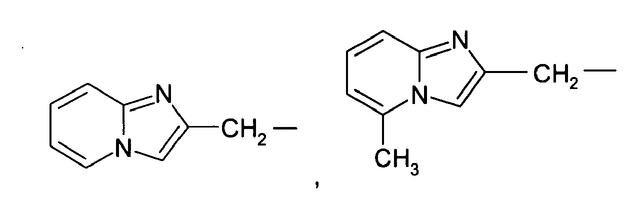 or 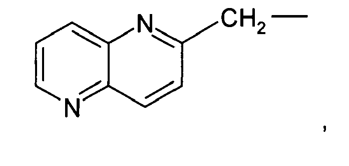 a 1,2,4-triazolo[4,3-a]pyridin-3-yl group of formula 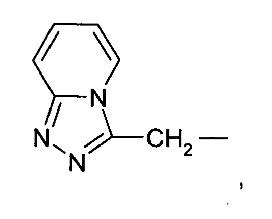 or a group of formulae 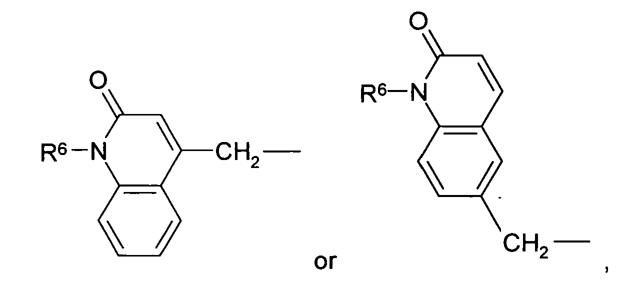 wherein R⁶ in each case denotes a hydrogen atom or a methyl group,
and R⁴ denotes a hydrogen atom or a methyl group,
while unless otherwise stated the alkyl and alkoxy groups listed in the definitions which have more than two carbon atoms may be straight-chain or branched,
and the hydrogen atoms of the methyl or ethyl groups listed in the definitions may be wholly or partly replaced by fluorine atoms,
with the proviso that the following two compounds are excluded:
2-(2-amino-cyclohexylamino)-3-(but-2-ynyl)-5-methyl-3,5-dihydro-imidazo[4,5-d]pyridazin-4-one and
2-(3-amino-piperidin-1-yl)-3-(but-2-ynyl)-5-methyl-3,5-dihydro-imidazo[4,5-d]pyridazin-4-one,
the tautomers, the enantiomers, the diastereomers, the mixtures thereof and the salts thereof.

3. Compounds of general formula I according to claim 1, wherein
X, R², R³ and R⁴ are defined as in claim 2 and
R¹ denotes a 3-amino-piperidin-1-yl group,
with the proviso that the following compound is excluded:
2-(3-amino-piperidin-1-yl)-3-(but-2-ynyl)-5-methyl-3,5-dihydro-imidazo[4,5-d]pyridazin-4-one,
the tautomers, the enantiomers, the diastereomers, the mixtures thereof and the salts thereof.

4. Compounds of general formula I according to claim 1, wherein
X, R¹, R³ and R⁴ are defined as in claim 2 or 3 and
R² denotes a 3-methylallyl, a 3,3-dimethylallyl or a but-2-yn-4-yl group,
with the proviso that the following two compounds are excluded:
2-(2-amino-cyclohexylamino)-3-(but-2-ynyl)-5-methyl-3,5-dihydro-imidazo[4,5-d]pyridazin-4-one and
2-(3-amino-piperidin-1-yl)-3-(but-2-ynyl)-5-methyl-3,5-dihydro-imidazo[4,5-d]pyridazin-4-one,
the tautomers, the enantiomers, the diastereomers, the mixtures thereof and the salts thereof.

5. The following compounds of general formula I according to claim 1:
(1) 2-(3-amino-piperidin-1-yl)-3-(but-2-ynyl)-5-(naphthalen-1-ylmethyl)-3,5-dihydro-imidazo[4,5-d]pyridazin-4-one 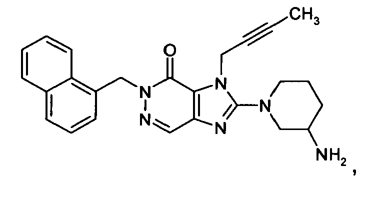
(2) 2-(3-amino-piperidin-1-yl)-3-but-2-ynyl-5-(3-methyl-isoquinolin-1-ylmethyl)-3,5-dihydro-imidazo[4,5-d]pyridazin-4-one, 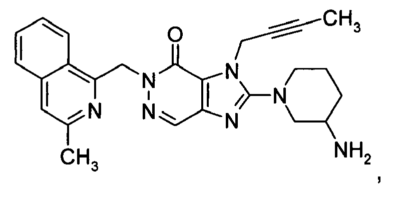
(3) 2-(3-amino-piperidin-1-yl)-3-(but-2-ynyl)-5-(quinazolin-2-ylmethyl)-3,5-dihydro-imidazo[4,5-d]pyridazin-4-one 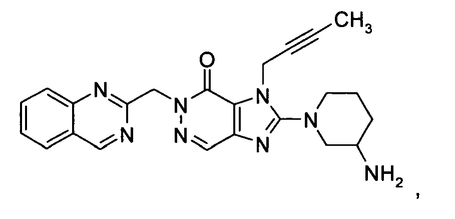
(4) 2-((*R*)-3-amino-piperidin-1-yl)-3-(but-2-ynyl)-5-(4-methyl-quinazolin-2-ylmethyl)-3,5-dihydro-imidazo[4,5-d]pyridazin-4-one 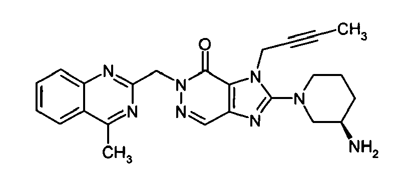
(5) 2-((R)-3-amino-piperidin-1-yl)-3-(but-2-ynyl)-5-(4-cyano-naphthalen-1-ylmethyl)-3,5-dihydro-imidazo[4,5-d]pyridazin-4-one 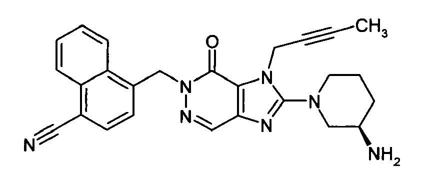
(6) 2-((R)-3-amino-piperidin-1-yl)-3-(but-2-ynyl)-5-(4-bromonaphth-1-ylmethyl)-3,5-dihydro-imidazo[4,5-d]pyridazin-4-one 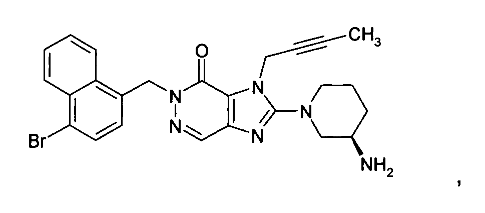
(7) 2-((R)-3-amino-piperidin-1-yl)-3-(but-2-ynyl)-5-(benzo[1,2,5]thiadiazol-5-ylmethyl)-3,5-dihydro-imidazo[4,5-d]pyridazin-4-one 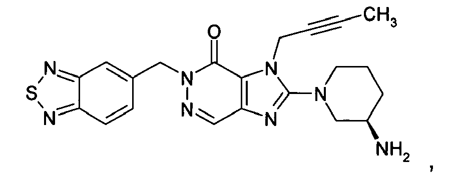
(8) 2-((*R*)-3-amino-piperidin-1-yl)-3-(2-chlorobenzyl)-5-(3-methylisoquinolin-1-ylmethyl)-3,5-dihydro-imidazo[4,5-d]pyridazin-4-one 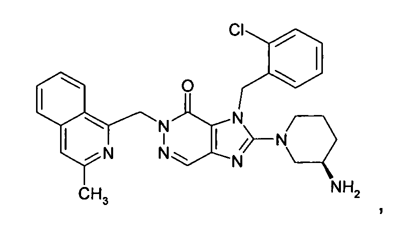
(9) 2-(3-amino-piperidin-1-yl)-3-(but-2-ynyl)-5-(quinoxalin-6-ylmethyl)-3,5-dihydro-imidazo[4,5-d]pyridazin-4-one 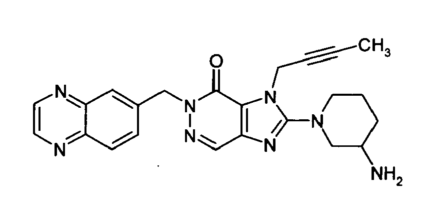
(10) 2-((R)-3-amino-piperidin-1-yl)-3-(but-2-ynyl)-5-(2,3-dimethyl-quinoxalin-6-ylmethyl)-3,5-dihydro-imidazo[4,5-d]pyridazin-4-one 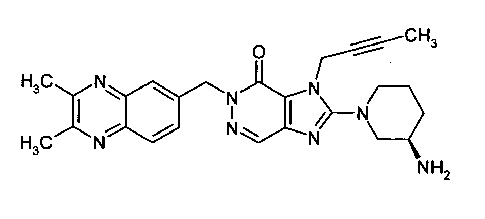
(11) 2-((*R*)-3-amino-piperidin-1-yl)-3-(but-2-ynyl)-5-(5-methyl-imidazo[1,2-a]pyridin-2-ylmethyl)-3,5-dihydro-imidazo[4,5-d]pyridazin-4-one 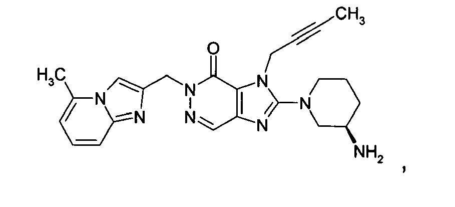
(12) 2-((R)-3-amino-piperidin-1-yl)-3-(but-2-ynyl)-5-(1-methyl-1H-quinolin-2-on-6-ylmethyl)-3,5-dihydro-imidazo[4,5-d]pyridazin-4-one 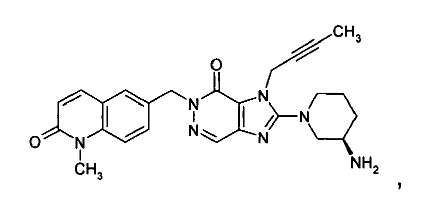
(13) 2-((R)-3-amino-piperidin-1-yl)-3-(but-2-ynyl)-5-(4-methyl-phthalazin-1-ylmethyl)-3,5-dihydro-imidazo[4,5-d]pyridazin-4-one 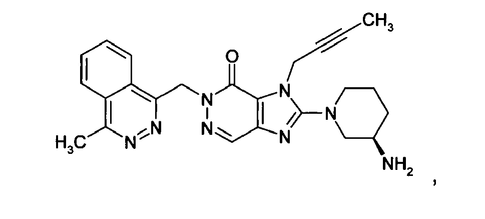
(14) 2-((R)-3-amino-piperidin-1-yl)-3-(but-2-ynyl)-5-([1,5]naphthyridin-2-ylmethyl)-3,5-dihydro-imidazo[4,5-d]pyridazin-4-one 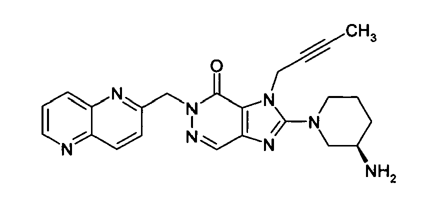
(15) 2-((*R*)-3-amino-piperidin-1-yl)-3-(but-2-ynyl)-5-(2,3,8-trimethylquinoxalin-6-ylmethyl)-3,5-dihydro-imidazo[4,5-d]pyridazin-4-one 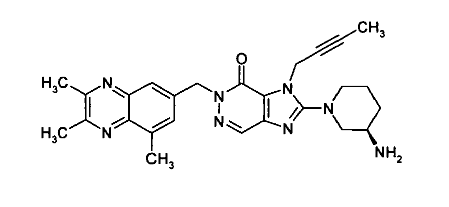
and the enantiomers and the salts thereof.

6. Physiologically acceptable salts of the compounds according to at least one of claims 1 to 5 with inorganic or organic acids.

7. Pharmaceutical compositions containing a compound according to at least one of claims 1 to 5 or a salt according to claim 6 optionally together with one or more inert carriers and/or diluents.

8. Use of a compound according to at least one of claims 1 to 5 or a salt according to claim 6 for preparing a pharmaceutical composition which is suitable for the treatment of type I and type II diabetes mellitus, arthritis, obesity, allograft transplantation and osteoporosis caused by calcitonin.

9. A compound according to at least one of claims 1 to 5 or a salt according to claim 6 for the treatment of type I and type II diabetes mellitus, arthritis, obesity, allograft transplantation and osteoporosis caused by calcitonin.

10. Process for preparing a pharmaceutical composition according to claim 7, **characterised in that** a compound according to at least one of claims 1 to 5 or a salt according to claim 6 is incorporated in one or more inert carriers and/or diluents by a non-chemical method.

11. Process for preparing the compounds of general formula I according to claims 1 to 6, **characterised in that**
a) a compound of general formula 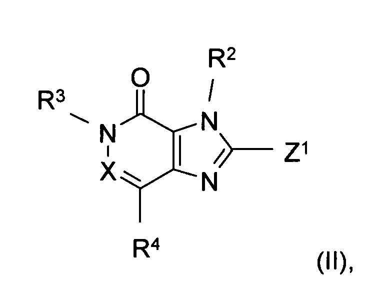 wherein X, R², R³ and R⁴ are defined as in claims 1 to 5 and Z¹ denotes a nucleofugic leaving group such as for example a chlorine or bromine atom or a C₁₋₃-alkylsulphanyl, C₁₋₃-alkylsulphinyl or C₁₋₃-alkylsulphonyl group, is reacted with an amine of general formula
H-R¹ (III),
wherein R¹ is defined as in claims 1 to 5, or
b) a compound of general formula 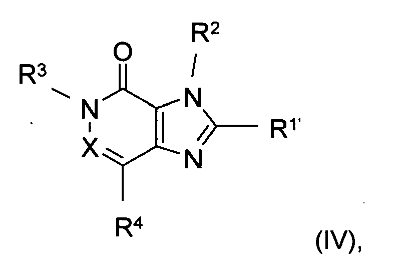 wherein R², R³ and R⁴ are defined as in claims 1 to 5 and
R^{1'} denotes one of the groups mentioned for R¹ hereinbefore, wherein the imino, amino or alkylamino group is substituted by a protective group, is deprotected and
subsequently if desired any protecting group used to protect reactive groups during the reactions is cleaved and/or
a compound of general formula I thus obtained is resolved into its stereoisomers and/or
a compound of general formula I thus obtained is converted into the salts thereof, particularly for pharmaceutical use into the physiologically acceptable salts thereof with an inorganic or organic acid.

## Revendications

1. Composés de formule générale 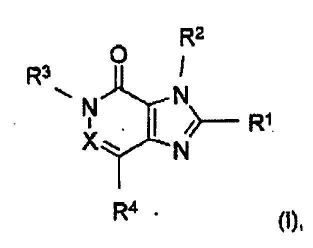 où
X représente un atome d'azote ou un groupe de formule C-R⁵,
où R⁵ représente un atome d'hydrogène ou un groupe méthyle,
R¹ représente un groupe cycloalkylèneimino à 5 à 7 chaînons qui est substitué dans le squelette carboné par un groupe amino et peut être substitué par un groupe C₁₋₃-alkyle,
ou un groupe amino substitué par un groupe C₅₋₇-cycloalkyle,
où le groupe C₅₋₇-cycloalkyle est substitué par un groupe amino ou un atome de carbone en position 3 du groupe C₅₋₇-cycloalkyle est remplacé par un groupe -NH-,
R² représente un groupe benzyle où le groupement phényle peut être substitué par un ou deux atomes de fluor, chlore ou brome ou par un groupe cyano,
un groupe C₃-₈-alcényle linéaire ou ramifié,
un groupe C₃₋₅-alcynyle,
un groupe C₃₋₇-cycloalkylméthyle,
un groupe C₅₋₇-cycloalcénylméthyle,
ou un groupe furylméthyle, thiénylméthyle, pyrrolylméthyle, thiazolylméthyle, imidazolylméthyle, pyridinylméthyle, pyrimidinylméthyle, pyridazinylméthyle ou pyrazinylméthyle,
R³ représente un groupe C₁₋₆-alkyle linéaire ou ramifié,
un groupe phényl-C₁₋₃-alkyle ou naphtyl-C₁₋₃-alkyle éventuellement substitué dans la partie aryle par un atome d'halogène, un groupe cyano, un groupe C₁₋₃-alkyle ou un groupe méthoxy,
un groupe 2-phényl-2-hydroxy-éthyle,
un groupe phénylcarbonylméthyle,
où le groupe phényle peut être substitué par un groupe hydroxy, C₁₋₃-alkyloxy, aminocarbonyl-C₁₋₃-alcoxy, (C₁₋₃-alkylamino)-carbonyl-C₁₋₃-alcoxy, [di-(C₁₋₃-alkyl)-amino]-carbonyl-C₁₋₃-alcoxy, amino, C₁₋₃-alkyl-carbonylamino, C₃₋₆-cycloalkyl-carbonylamino, C₁-₃-alcoxy-carbonylamino, C₁₋₃-alkylsulfonylamino ou aminocarbonyle, un groupe (3-méthyl-2-oxo-2,3-dihydro-benzoxazolyl)-carbonylméthyle,
un groupe thiénylcarbonylméthyle,
un groupe hétéroaryl-C₁₋₃-alkyle,
où par l'expression « groupe hétéroaryle » on doit entendre un groupe hétéroaryle monocyclique à 5 ou 6 chaînons éventuellement substitué par un
ou deux groupes C₁₋₃-alkyle ou par un groupe morpholin-4-yle, pyridyle ou phényle, où
le groupe hétéroaryle à 6 chaînons contient un, deux ou trois atomes d'azote et
le groupe hétéroaryle à 5 chaînons contient un groupe imino éventuellement substitué par un groupe C₁₋₃-alkyle ou phényl-C₁₋₃-alkyle, un atome d'oxygène ou de soufre ou
un groupe imino éventuellement substitué par un groupe C₁₋₃-alkyle ou phényl-C₁₋₃-alkyle ou un atome d'oxygène ou de soufre et en outre un atome d'azote ou
un groupe imino éventuellement substitué par un groupe C₁₋₃-alkyle ou phényl-C₁₋₃-alkyle ou un atome d'oxygène ou de soufre et en outre deux ou trois atomes d'azote,
et où en outre un cycle phényle qui peut éventuellement être substitué par un atome d'halogène, par un ou deux groupes C₁₋₃-alkyle ou par un groupe trifluorométhyle ou méthoxy peut être condensé aux groupes hétéroaryle monocycliques cités précédemment via deux atomes de carbone voisins,
et la liaison peut se faire via un atome de la partie hétérocyclique ou du cycle phényle condensé,
un groupe hétéroarylméthyle bicyclique selon l'une des formules 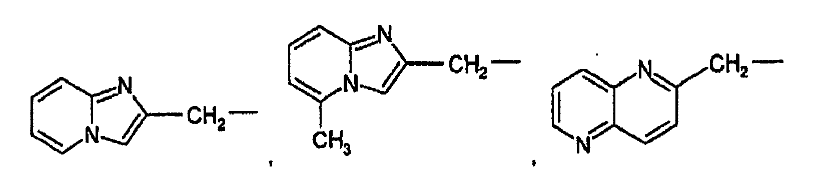 ou 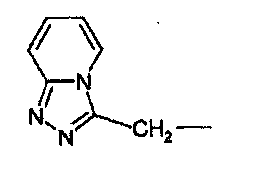 un groupe de formule 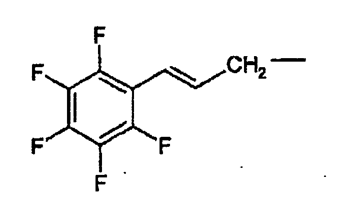 ou un groupe de formule 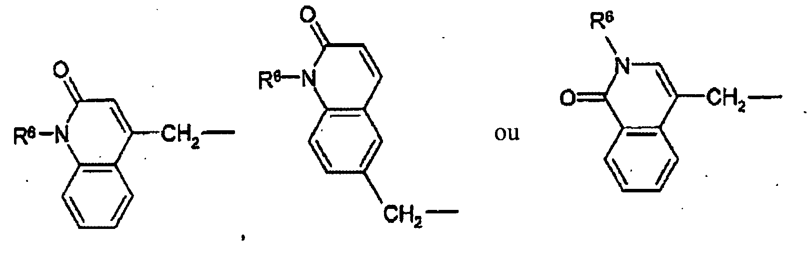 où R⁶ représente dans chaque cas un atome d'hydrogène ou un groupe méthyle, et R⁴ représente un atome d'hydrogène ou un groupe C₁₋₃-alkyle,
où les groupes alkyle et alcoxy contenus dans les définitions, qui comportent plus de deux atomes de carbone, peuvent être linéaire ou ramifiés, sauf indication contraire,
et où les atomes d'hydrogène des groupes méthyle ou éthyle contenus dans les définitions peuvent être remplacés totalement ou partiellement par des atomes de fluor,
avec la condition que les deux composés suivants sont exclus :
2-(2-amino-cyclohexylamino)-3-(but-2-ynyl)-5-méthyl-3, 5-dihydro-imidazo [4, 5-d]pyridazin-4-one et
2-(3-amino-pipéridin-1-yl)-3-(but-2-ynyl)-5-méthyl-3,5-dihydro-imidazo [4,5-d]pyridazin-4-one,
leurs tautomères, leurs énantiomères, leurs diastéréoisomères, leurs mélanges et leurs sels.

2. Composés de formule générale I selon la revendication 1 où
X représente un atome d'azote ou un groupe méthyne,
R¹ représente un groupe 3-amino-pipéridin-1-yle, 3-amino-3-méthyl-pipéridin-1-yle, 3-amino-pyrrolidin-1-yle, 1,4-diazépan-1-yle, (2-amino-cyclohexyl)-amino ou pipéridin-3-yl-amino,
R² représente un groupe benzyle où le groupement phényle peut être substitué par un ou deux atomes de fluor, par un atome de chlore ou de brome ou par un groupe cyano,
un groupe C₃₋₈-alcényle linéaire ou ramifié,
un groupe propyn-3-yle ou but-2-yn-4-yle,
un groupe cyclopropylméthyle,
un groupe C₅₋₇-cycloalcénylméthyle,
ou un groupe furylméthyle ou thiénylméthyle,
R³ représente un groupe C₁₋₆-alkyle linéaire ou ramifié,
un groupe phényl-C₁₋₂-alkyle ou naphtyl-C₁₋₂-alkyle éventuellement substitué dans la partie aryle par un atome de fluor, de chlore ou de brome ou par un groupe cyano, C₁₋₃-alkyle ou méthoxy,
un groupe 2-phényl-2-hydroxy-éthyle,
un groupe phénylcarbonylméthyle,
où le groupe phényle peut être substitué par un groupe hydroxy, C₁₋₃-alkyloxy, aminocarbonyl-C₁₋₃-alcoxy, (C₁₋₃-alkylamino)-carbonyl-C₁₋₃-alcoxy, [di-(C₁₋₃-alkyl)-amino]-carbonyl-C₁₋₃-alcoxy, amino, C₁₋₃-alkyl-carbonylamino, C₃₋₆-cycloalkyl-carbonylamino, C₁₋₃-alcoxy-carbonylamino, C₁₋₃-alkylsulfonylamino ou aminocarbonyle, un groupe (3-méthyl-2-oxo-2,3-dihydro-benzoxazolyl)-carbonylméthyle,
un groupe thiénylcarbonylméthyle,
un groupe hétéroarylméthyle,
où par l'expression « groupe hétéroaryle » on doit entendre un groupe pyridinyle, pyrimidinyle, pyridazinyle, pyrazinyle, thiazolyle, oxazolyle, isothiazolyle, isoxazolyle, pyrazolyle, imidazolyle, oxadiazolyle, thiadiazolyle ou thiényle éventuellement substitué par un ou deux groupes méthyle ou par un groupe pyridyle ou phényle,
et où en outre un cycle phényle qui peut éventuellement être substitué par un atome de chlore, par un ou deux groupes méthyle ou par un groupe trifluorométhyle ou méthoxy peut être condensé aux groupes hétéroaryle monocycliques cités précédemment via deux atomes de carbone voisins,
et la liaison peut se faire via un atome de la partie hétérocyclique ou du cycle phényle condensé,
un groupe imidazo[1,2-a]pyridin-2-yl-méthyle de formule 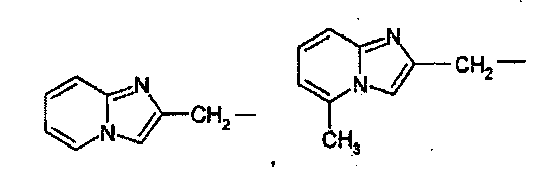 ou 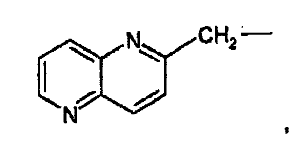 un groupe 1,2,4-triazolo[4,3-a]pyridin-3-yle de formule 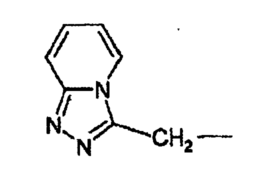 ou un groupe de formule 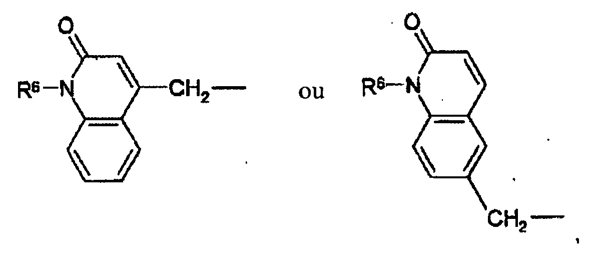 où R⁶ représente dans chaque cas un atome d'hydrogène ou un groupe méthyle, et R⁴ représente un atome d'hydrogène ou un groupe méthyle,
où les groupes alkyle et alcoxy contenus dans les définitions, qui comportent plus de deux atomes de carbone, peuvent être linéaire ou ramifiés, sauf indication contraire,
et où les atomes d'hydrogène des groupes méthyle ou éthyle contenus dans les définitions peuvent être remplacés totalement ou partiellement par des atomes de fluor,
avec la condition que les deux composés suivants sont exclus :
2-(2-amino-cyclohexylamino)-3-(but-2-ynyl)-5-méthyl-3, 5-dihydro-imidazo [4, 5-d]pyridazin-4-one et
2-(3-amino-pipéridin-1-yl)-3-(but-2-ynyl)-5-méthyl-3,5-dihydro-imidazo [4,5-d]pyridazin-4-one,
leurs tautomères, leurs énantiomères, leurs diastéréoisomères, leurs mélanges et leurs sels.

3. Composés de formule générale 1 selon la revendication 1 où
X, R², R³ et R⁴ sont définis comme indiqué dans la revendication 2 et
R¹ représente un groupe 3-amino-pipéridin-1-yle,
avec la condition que le composé suivant est exclu :
2-(3-amino-pipéridin-I-yl)-3-(but-2-ynyl)-5-méthyl-3,5-dihydro-imidazo[4,5-d]pyridazin-4-one,
leurs tautomères, leurs énantiomères, leurs diastéréoisomères, leurs mélanges et leurs sels.

4. Composés de formule générale I selon la revendication 1 où
X, R¹, R³ et R⁴ sont définis comme indiqué dans la revendication 2 ou 3 et
R² représente un groupe 3-méthylallyle, un groupe 3,3-diméthylallyle ou un groupe but-2-yn-4-yle,
avec la condition que les deux composés suivants sont exclus :
2-(2-amino-cyclohexylamino)-3-(but-2-ynyl)-5-méthyl-3, 5-dihydro-imidazo [4, 5 - d]pyridazin-4-one et
2-(3-amino-pipéridin-l-yl)-3-(but-2-ynyl)-5-méthyl-3,5-dihydro-imidazo[4,5-d]pyridazin-4-one,
leurs tautomères, leurs énantiomères, leurs diastéréoisomères, leurs mélanges et leurs sels.

5. Composés de formule générale 1 selon la revendication 1 suivants :
(1) 2-(3-amino-pipéridin-1-yl)-3-(but-2-ynyl)-5-(naphtalén-1-ylméthyl)-3,5-dihydro-imidazo[4,5-d]pyridazin-4-one, 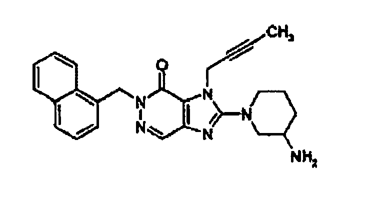
(2) 2-(3-amino-pipéridin-1-yl)-3-(but-2-ynyl)-5-(3-méthyl-isoquinolin-1-ylméthyl)-3,5-dihydro-imidazo[4,5-d]pyridazin-4-one, 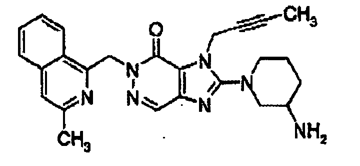
(3) 2-(3-amino-pipéridin-1-yl)-3-(but-2-ynyl)-5-(quinazolin-2-ylméthyl)-3,5-dihydro-imidazo[4,5-d]pyridazin-4-one, 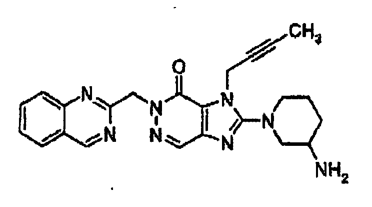
(4) 2-((*R*)-3-amino-pipéridin-1-yl)-3-(but-2-ynyl)-5-(4-méthyl-quinazolin-2-ylméthyl)-3,5-dihydro-imidazo[4,5-d]pyridazin-4-one, 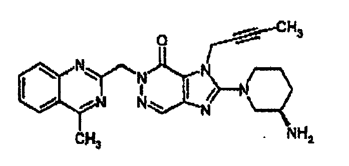
(5) 2-((*R*)-3-amino-pipéridin-1-yl)-3-(but-2-ynyl)-5-(4-cyano-naphtalén-1-ylméthyl)-3,5-dihydro-imidazo[4,5-d]pyridazin-4-one, 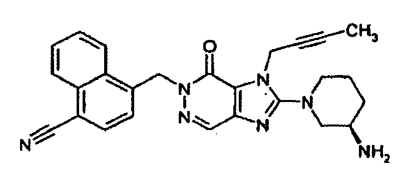
(6) 2-((*R*)-3-amino-pipéridin-1-yl)-3-(but-2-ynyl)-5-(4-bromonapht-1-ylméthyl)-3,5-dihydro-imidazo[4,5-d]pyridazin-4-one, 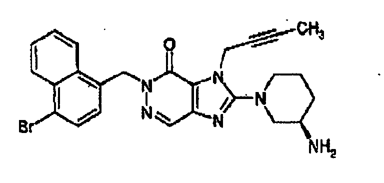
(7) 2-((*R*)-3-amino-pipéridin-1-yl)-3-(but-2-ynyl)-5-(benzo[1,2,5]thiadiazol-5-ylméthyl)-3,5-dihydro-imidazo[4,5-d]pyridazin-4-one, 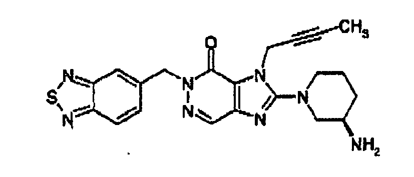
(8) 2-((*R*)-3-amino-pipéridin-1-yl)-3-(2-chlorobenzyl)-5-(3-méthyl-isoquinolin-1-ylméthyl)-3,5-dihydro-imidazo[4,5-d]pyridazin-4-one, 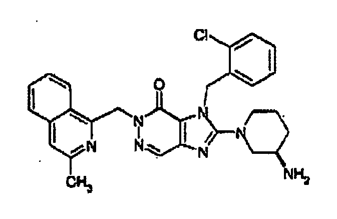
(9) 2-(3-amino-pipéridin-1-yl)-3-(but-2-ynyl)-5-(quinoxalin-6-ylméthyl)-3,5-dihydro-imidazo[4,5-d]pyridazin-4-one, 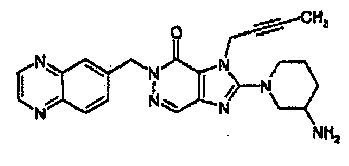
(10) 2-((*R*)-3-amino-pipéridin-1-yl)-3-(but-2-ynyl)-5-(2,3-diméthyl-quinoxalin-6-ylméthyl)-3,5-dihydro-imidazo[4,5-d]pyridazin-4-one, 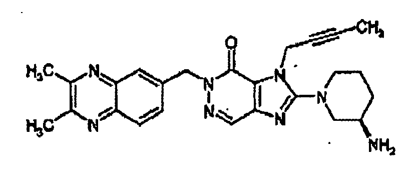
(11) 2-((*R*)-3-amino-pipéridin-1-yl)-3-(but-2-ynyl)-5-(5-méthyl-imidazo[1,2-a]pyridin-2-ylméthyl)-3,5-dihydro-imidazo[4,5-d]pyridazin-4-one, 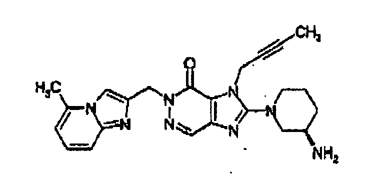
(12) 2-((*R*)-3-amino-pipéridin-1-yl)-3-(but-2-ynyl)-5-(1-méthyl-1*H*-quinolin-2-on-6-ylméthyl)-3,5-dihydro-imidazo[4,5-d]pyridazin-4-one, 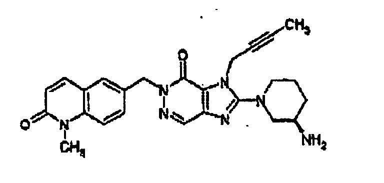
(13) 2-((*R*)-3-amino-pipéridin-1-yl)-3-(but-2-ynyl)-5-(4-méthyl-phtalazin-1-ylméthyl)-3,5-dihydro-imidazo[4,5-d]pyridazin-4-one, 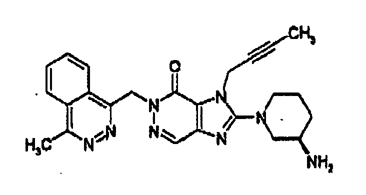
(14) 2-((*R*)-3-amino-pipéridin-1-yl)-3-(but-2-ynyl)-5-([1,5]naphtyridin-2-ylméthyl)-3,5-dihydro-imidazo[4,5-d]pyridazin-4-one, 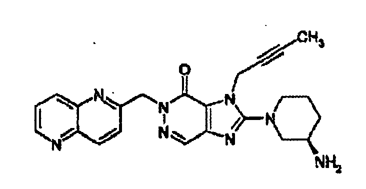
(15) 2-((*R*)-3-amino-pipéridin-1-yl)-3-(but-2-ynyl)-5-(2,3,8-triméthyl-quinoxalin-6-ylméthyl)-3,5-dihydro-imidazo[4,5-d]pyridazin-4-one, 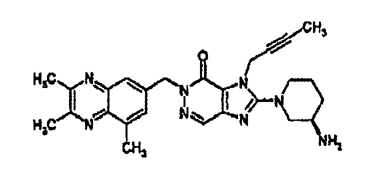
ainsi que leurs énantiomères et leurs sels.

6. Sels physiologiquement acceptables des composés selon les revendications 1 à 5 avec des acides inorganiques ou organiques.

7. Médicament contenant un composé selon au moins l'une des revendications 1 à 5 ou un sel selon la revendication 6 outre éventuellement un ou plusieurs vecteurs et/ou diluants inertes.

8. Utilisation d'un composé selon au moins l'une des revendications 1 à 5 ou d'un sel selon la revendication 6 pour la production d'un médicament qui convient pour le traitement du diabète sucré de type 1 et de type II, de l'arthrite, de l'adiposité, de la transplantation d'allogreffes et de l'ostéoporose provoquée par la calcitonine.

9. Composé selon au moins l'une des revendications 1 à 5 ou sel selon la revendication 6 pour le traitement du diabète sucré de type 1 et de type II, de l'arthrite, de l'adiposité, de la transplantation d'allogreffes et de l'ostéoporose provoquée par la calcitonine.

10. Procédé de production d'un médicament selon la revendication 7 **caractérisé en ce que**, par voie non chimique, un composé selon au moins l'une des revendications 1 à 5 ou un sel selon la revendication 6 est incorporé dans un
ou plusieurs vecteurs et/ou diluants inertes.

11. Procédé de production des composés de formule 1 selon les revendications 1 à 6 **caractérisé en ce que**
a) un composé de formule générale 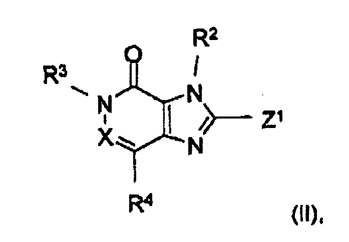 où X, R², R³ et R⁴ sont définis comme indiqué dans les revendications 1 à 5 et
Z¹ représente un groupe partant nucléofuge comme par exemple un atome de chlore ou de brome ou un groupe C₁₋₃-alkylsulfanyle, C₁₋₃-alkylsulfinyle ou C₁₋₃-alkylsulfonyle,
est mis à réagir avec une amine de formule générale
H-R¹ (III)
où R¹ est défini comme indiqué dans les revendications 1 à 5, ou
b) un composé de formule générale 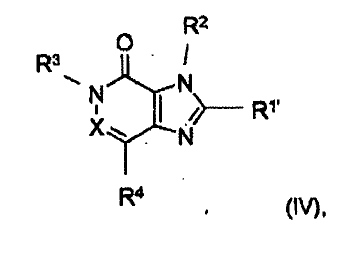
où R² R³ et R⁴ sont définis comme indiqué dans les revendications 1 à 5 et
R^{1'} représente l'un des groupes cités au début pour R¹ où le groupe imino, amino
ou alkylamino est substitué par un groupe protecteur, est déprotégé puis
si on le souhaite un groupe protecteur éventuellement utilisé pendant les réactions pour la protection de groupes réactifs est clivé et/ou
un composé de formule générale I ainsi obtenu est séparé en ses stéréoisomères et/ou
un composé de formule générale I ainsi obtenu est converti en ses sels, en particulier pour l'utilisation pharmaceutique en ses sels physiologiquement acceptables avec un acide inorganique ou organique.
